# EUROPÄISCHE PATENTSCHRIFT

(11) **EP 3 103 882 B1**
(45) Veröffentlichungstag und Bekanntmachung des Hinweises auf die Patenterteilung: **16.01.2019**
(21) Anmeldenummer: 15171247.8
(22) Anmeldetag: 09.06.2015
(51) Int. Cl.: C12Q 1/6883

(54) **VERFAHREN ZUR GENBASIERTEN DIAGNOSE EINES LEGASTHENIERISIKOS**
METHOD FOR GENE-BASED DIAGNOSIS OF A LEGASTHENIE RISK
PROCÉDÉ DE DIAGNOSTIC GÉNÉTIQUE D'UN RISQUE DE DYSLEXIE

(43) Veröffentlichungstag der Anmeldung: 14.12.2016
(73) Patentinhaber: Universität Leipzig, 04109 Leipzig (DE); Fraunhofer-Gesellschaft zur Förderung der angewandten Forschung e.V., 80686 München (DE); Universitätsklinikum Jena, 07743 Jena (DE)
(72) Erfinder: Wilcke, Arndt, 08412 Werdau (DE); Boltze, Johannes, 04105 Leipzig (DE); Ahnert, Peter, 04109 Leipzig (DE); Ligges, Carolin, 07743 Jena (DE); Kirsten, Holger, 04318 Leipzig (DE)
(74) Vertreter: Vossius & Partner Patentanwälte Rechtsanwälte mbB

(56) Entgegenhaltungen:
- WO-A2-2005/049796
- WO-A2-2010/007063
- SUN YIMIN ET AL: "Association Study of Developmental Dyslexia Candidate Genes DCDC2 and KIAA0319 in Chinese Population", AMERICAN JOURNAL OF MEDICAL GENETICS, Bd. 165, Nr. 8, Dezember 2014 (2014-12), Seiten 627-634, XP002746074,
- MUELLER B ET AL: "Genetic risk variants for dyslexia on chromosome 18 in a German cohort", GENES BRAIN AND BEHAVIOR, Bd. 13, Nr. 3, März 2014 (2014-03), Seiten 350-356, XP002746075,
- SILVIA PARACCHINI ET AL: "The Genetic Lexicon of Dyslexia", ANNUAL REVIEW OF GENOMICS AND HUMAN GENETICS, Bd. 8, Nr. 1, 1. September 2007 (2007-09-01), Seiten 57-79, XP055220367, US ISSN: 1527-8204, DOI: 10.1146/annurev.genom.8.080706.092312
- GIBSON C J ET AL: "The human lexinome: Genes of language and reading", JOURNAL OF COMMUNICATION DISORDERS, ELSEVIER SCIENCE, NEW YORK, NY, US, Bd. 41, Nr. 5, 1. September 2008 (2008-09-01), Seiten 409-420, XP022765439, ISSN: 0021-9924, DOI: 10.1016/J.JCOMDIS.2008.03.003 [gefunden am 2008-03-25]

### SEQUENCE LISTING

<110> Universitaet Leipzig et al.
<120> Verfahren zur genbasierten Diagnose eines Legasthenierisikos
<130> W2004 EP BLN
<160> 24
<170> BiSSAP 1.3
<210> 1
   <211> 1001
   <212> DNA
   <213> Homo sapiens
<220>
   <223> Chromosomale Region von Nukleotid 12091000 bis 12200000 von Chromosom 1 mit A an der Position von rs496888
<220>
   <221> variation
   <222> 501
   <223> /allele="rs496888"
<400> 1
   ccgacacttc cccaatattc taggaaagca ttggaaccag ggttgttagg tttcatagat      60
   agggatgctt aggcccagag aggttaagta acccaagcaa ggtagcacag caagccagtg     120
   acggtgctca gatgggaact gtgatctccc aacacccaag tcagtgctcc ttcctcagag     180
   ccatgctgtc ttagaagtca cttggaggca ggaaaatgga cagcatgatc tcagaagagc     240
   ccccataggg catccccttc tcagggtcac aagtaaacag aaccttcagc agcacagagg     300
   ttggagatgg aagaggtttt ggagatggcc tggaatcttc ccgtgcccat cttgcaaact     360
   gaagccttgt gcactctctc tgtgcttcac caaatgtacc cttcactcat ccccaaaaca     420
   cacagcacct gttactcact aaaaggggtg gcaaagggag gggaggggat atcctgaccc     480
   cacagccttt gccacaatac agtgtcctaa gaccaggctg tcaagcagtg ggtttgagag     540
   cccagccagg gggcactggg gcagaagcac agacaggaaa aggcagggag gaaagaagtt     600
   gctttctgag atgatgggca acaaagccac ttagacagga actctgggca actgcagggc     660
   tgccacccaa gcttcagggc cctggggctc agagcagaca gtctgactcc acaggggtcc     720
   ccacctaggc ccgagcacca ccccatggcc cagagagtcg ggtcagacct gcaaagccac     780
   caatggctca aatagctgcc aatgccagga ttagaaagca cttttaatcc tgacagccag     840
   cccgtgaggc taggattact cccattttac agataagaac acagaggctc cgagggatag     900
   aaagacttgc ccaggtcaca cagccagtaa aacagcagag cagcgacttg aggtttatct     960
   gattcagagg ccggggcctt tccccagagt catcagtgct g                        1001
<210> 2
   <211> 1001
   <212> DNA
   <213> Homo sapiens
<220>
   <223> Chromosomale Region von Nukleotid 12091000 bis 12200000 von Chromosom 1 mit G an der Position von rs496888
<220>
   <221> variation
   <222> 501
   <223> /allele="rs496888"
<400> 2
   ccgacacttc cccaatattc taggaaagca ttggaaccag ggttgttagg tttcatagat      60
   agggatgctt aggcccagag aggttaagta acccaagcaa ggtagcacag caagccagtg     120
   acggtgctca gatgggaact gtgatctccc aacacccaag tcagtgctcc ttcctcagag     180
   ccatgctgtc ttagaagtca cttggaggca ggaaaatgga cagcatgatc tcagaagagc     240
   ccccataggg catccccttc tcagggtcac aagtaaacag aaccttcagc agcacagagg     300
   ttggagatgg aagaggtttt ggagatggcc tggaatcttc ccgtgcccat cttgcaaact     360
   gaagccttgt gcactctctc tgtgcttcac caaatgtacc cttcactcat ccccaaaaca     420
   cacagcacct gttactcact aaaaggggtg gcaaagggag gggaggggat atcctgaccc     480
   cacagccttt gccacaatac ggtgtcctaa gaccaggctg tcaagcagtg ggtttgagag     540
   cccagccagg gggcactggg gcagaagcac agacaggaaa aggcagggag gaaagaagtt     600
   gctttctgag atgatgggca acaaagccac ttagacagga actctgggca actgcagggc     660
   tgccacccaa gcttcagggc cctggggctc agagcagaca gtctgactcc acaggggtcc     720
   ccacctaggc ccgagcacca ccccatggcc cagagagtcg ggtcagacct gcaaagccac     780
   caatggctca aatagctgcc aatgccagga ttagaaagca cttttaatcc tgacagccag     840
   cccgtgaggc taggattact cccattttac agataagaac acagaggctc cgagggatag     900
   aaagacttgc ccaggtcaca cagccagtaa aacagcagag cagcgacttg aggtttatct     960
   gattcagagg ccggggcctt tccccagagt catcagtgct g                        1001
<210> 3
   <211> 1081
   <212> DNA
   <213> Homo sapiens
<220>
   <223> Chromosomale Region von Nukleotid 89066000 bis 89088000 von Chromosom 15 mit A an der Position von rs7162960
<220>
   <221> variation
   <222> 501
   <223> /allele="rs7162960"
<400> 3
   aggcatgttg gtgaacaatt ttctttttgt gtccaacaat tgtatcgata ttttgatgga      60
   gtatgtacag aagagacacc cttatggttc ttagtactca ccaacttcct gaaagcattc     120
   ccactgagga ttttgagtaa gaaaattatg tactcattac catacttcag aatatgattc     180
   tggctggagg gtgctacctc gtgaggaata accaaagagc agtgcatttt aaaagcagga     240
   agaggtcccc tggagttaag gttgactgca tgctagctgt gaatccatag tgtaatcact     300
   acagttataa aatagttaat caaacaattg tactcctggg atggctaagt agacatataa     360
   aaccatgctg tggagtaatg taggaaatct cttattaaaa tatcatggct agttttgatc     420
   caaaaataag taagttatgg aaggaatgtg ggaaaagggt aagtaatgat gccatagacc     480
   actttgttct gcttagtgtc attaagactg aagatctgtg tacctctgcc tccttgtcag     540
   tagtctactt tgttgatgtt gaaattctta gagggcaata gtctgacttc actctttatc     600
   acttcgtatt tattcatgtg ggaaagactc ccacattgta caggaccttg aagaggggac     660
   gcctcaacaa aagatcaagg cttttatttt ttatttattt atttattttt tgagacagag     720
   tctagctctg tcaccaggct ggagtgcagt ggcacaatct cggttcactg caacctctgc     780
   ctcccgggtt caagcaatta tcctgcctca gcttcccaag taactgggat tacaggcaca     840
   tgccaccatg cccagctaat tttgtatttt tagtagagac agggattcac catattggcc     900
   aggttggtct tgatctcctg acctcatgat ccgcccacct cggccttcca aagtgctggg     960
   attacaggcg tgagccaccg tgcccagcca agatcaatac tcctcatgat ccgcccacct    1020
   cggccttcca aagtgctggg attacaggcg tgagccaccg tgcccagcca agatcaatac    1080
   t                                                                    1081
<210> 4
   <211> 1081
   <212> DNA
   <213> Homo sapiens
<220>
   <223> Chromosomale Region von Nukleotid 89066000 bis 89088000 von Chromosom 15 mit G an der Position von rs7162960
<220>
   <221> variation
   <222> 501
   <223> /allele="rs7162960"
<400> 4
   aggcatgttg gtgaacaatt ttctttttgt gtccaacaat tgtatcgata ttttgatgga      60
   gtatgtacag aagagacacc cttatggttc ttagtactca ccaacttcct gaaagcattc     120
   ccactgagga ttttgagtaa gaaaattatg tactcattac catacttcag aatatgattc     180
   tggctggagg gtgctacctc gtgaggaata accaaagagc agtgcatttt aaaagcagga     240
   agaggtcccc tggagttaag gttgactgca tgctagctgt gaatccatag tgtaatcact     300
   acagttataa aatagttaat caaacaattg tactcctggg atggctaagt agacatataa     360
   aaccatgctg tggagtaatg taggaaatct cttattaaaa tatcatggct agttttgatc     420
   caaaaataag taagttatgg aaggaatgtg ggaaaagggt aagtaatgat gccatagacc     480
   actttgttct gcttagtgtc gttaagactg aagatctgtg tacctctgcc tccttgtcag     540
   tagtctactt tgttgatgtt gaaattctta gagggcaata gtctgacttc actctttatc     600
   acttcgtatt tattcatgtg ggaaagactc ccacattgta caggaccttg aagaggggac     660
   gcctcaacaa aagatcaagg cttttatttt ttatttattt atttattttt tgagacagag     720
   tctagctctg tcaccaggct ggagtgcagt ggcacaatct cggttcactg caacctctgc     780
   ctcccgggtt caagcaatta tcctgcctca gcttcccaag taactgggat tacaggcaca     840
   tgccaccatg cccagctaat tttgtatttt tagtagagac agggattcac catattggcc     900
   aggttggtct tgatctcctg acctcatgat ccgcccacct cggccttcca aagtgctggg     960
   attacaggcg tgagccaccg tgcccagcca agatcaatac tcctcatgat ccgcccacct    1020
   cggccttcca aagtgctggg attacaggcg tgagccaccg tgcccagcca agatcaatac    1080
   t                                                                    1081
<210> 5
   <211> 1001
   <212> DNA
   <213> Homo sapiens
<220>
   <223> Chromosomale Region von Nukleotid 41930000-42032000 von Chromosom 2 mit C an der Position von rs2715079
<220>
   <221> variation
   <222> 501
   <223> /allele="rs2715079"
<400> 5
   cacatgggct ttttaatttc cattaaaatg catattcata tcacgcttag tctcactttt      60
   gtctctgatc cttcaccatg atgatcccgg caacctgatg agtttgctgg ccatccaata     120
   catctttgtg cgttgcttaa aacacctgct gtagtaagta tacttttgcc attctacata     180
   tatctgatat tgagattaac cgtgttctat attttagact aatatttatt gtccactagc     240
   ctataaattt ctgagaagta tgcagcctaa gaataacttt ttgtaacttt agaagaacta     300
   tgcaactaca aactaaactt ctaccaatgg ttcattagtg atactttgac atatagagtg     360
   tctttcaact ggaagtcatc ttcaacctgc tcagactact cacatgtgac actaagaagg     420
   atcaaggaaa atgattttct tttttatttt attaagtttt ttcagaggat aggggaacat     480
   tataacaact acacaacata ccaagaaaag gaacttgagg acttgttaag gaatattaaa     540
   agactatcta tgcaaatcat gaaacgcttc agtgttcact aatccatcat tgcagaaata     600
   ctaaatgaag gttggattaa atgctatgca agaaaattaa gttatccttg tcccaataca     660
   aaaaaaaatt aaaaacagat gggaaaatct tagaattcca tttaactgga aagaatttga     720
   aagcacagtg gacttccaag gaaaggcaaa taaatctgat gaacatgtgg caattacata     780
   actctgaatc agactcattc ttcagtggac tgaaaagact aaaaatgatg gagccaggat     840
   tttggaaaga tgattggata gaaacagaaa tattatttcc aataataccg atattttgct     900
   aaaacaatca ttgaaaggaa acataatttt tagcaaaaac caaccaacaa aatataatcc     960
   tctaaagttt attttgaaca attctaggga gattgtttct g                        1001
<210> 6
   <211> 1001
   <212> DNA
   <213> Homo sapiens
<220>
   <223> Chromosomale Region von Nukleotid 41930000-42032000 von Chromosom 2 mit T an der Position von rs2715079
<220>
   <221> variation
   <222> 501
   <223> /allele="rs2715079"
<400> 6
   cacatgggct ttttaatttc cattaaaatg catattcata tcacgcttag tctcactttt      60
   gtctctgatc cttcaccatg atgatcccgg caacctgatg agtttgctgg ccatccaata     120
   catctttgtg cgttgcttaa aacacctgct gtagtaagta tacttttgcc attctacata     180
   tatctgatat tgagattaac cgtgttctat attttagact aatatttatt gtccactagc     240
   ctataaattt ctgagaagta tgcagcctaa gaataacttt ttgtaacttt agaagaacta     300
   tgcaactaca aactaaactt ctaccaatgg ttcattagtg atactttgac atatagagtg     360
   tctttcaact ggaagtcatc ttcaacctgc tcagactact cacatgtgac actaagaagg     420
   atcaaggaaa atgattttct tttttatttt attaagtttt ttcagaggat aggggaacat     480
   tataacaact acacaacata tcaagaaaag gaacttgagg acttgttaag gaatattaaa     540
   agactatcta tgcaaatcat gaaacgcttc agtgttcact aatccatcat tgcagaaata     600
   ctaaatgaag gttggattaa atgctatgca agaaaattaa gttatccttg tcccaataca     660
   aaaaaaaatt aaaaacagat gggaaaatct tagaattcca tttaactgga aagaatttga     720
   aagcacagtg gacttccaag gaaaggcaaa taaatctgat gaacatgtgg caattacata     780
   actctgaatc agactcattc ttcagtggac tgaaaagact aaaaatgatg gagccaggat     840
   tttggaaaga tgattggata gaaacagaaa tattatttcc aataataccg atattttgct     900
   aaaacaatca ttgaaaggaa acataatttt tagcaaaaac caaccaacaa aatataatcc     960
   tctaaagttt attttgaaca attctaggga gattgtttct g                        1001
<210> 7
   <211> 1001
   <212> DNA
   <213> Homo sapiens
<220>
   <223> Chromosomale Region von Nukleotid 51508000 bis 51885000 von Chromosom 2 mit C an der Position von rs2374341
<220>
   <221> variation
   <222> 501
   <223> /allele="rs2374341"
<400> 7
   ccgttgctcc aggtgacata gagcaaacca ccagacgtga gtgtgaggcc actctagact 60
   gtccatcccc agccaggaca gtctgtacca gacaatctac ctagcctatc cccagaatag 120
   taagaccata ttctgttaag tcagtacatt ttgggagtag tttattatac agcaaaagct 180
   agctgataca agaatgcaga aaagtccatt catttctccc aaaaaacgcc tctcaaatca 240
   catatttcag gcaaaggact ggctcctctt tgtcaatgac ttctgaaaac tgtccatttt 300
   ttttctaaca tttcataaca tctattgatt cttcctttgg acactcacct accttaggag 360
   aaaaaaaaaa ttcacagctc tggccaagat aggagctggt cctatcagag ctatgtacaa 420
   aggaatcgga acaggtacag tggaacccaa gataagcatg tgatggcaaa gtcagagggg 480
   ctgcactgcc aatgttgaac cgggatagac aagagcataa caaagaagac accagggcag 540
   aaaacaccct aagcagcttt gctctgaact ctcccctctg caagaaccct tcacccagcc 600
   cagggatcgg aaactcaatg gagagccggt aagcacagta agaaagagtt tacagcttcc 660
   ttcctcaaaa gcttccaaga taaacaaaat aaacaccagt tggtctcagc tggtcgcaca 720
   ttggcctgcc tggaggaggg ggatgccaga gatagcttct tgaggttgct tccagcattg 780
   cattctagga aatagacaat tatgaacaac atggatttta ttatcgtgac atctctaagg 840
   tctctgcata aagcctagat ttaaaaatat aaacatgtaa gtgaaaaaat gtcaggggaa 900
   taaatatttg tcttaaaaga gaccatctgt ttcaaatttt tatttaaaaa aaaaaaaccc 960
   aacatgtgtt ttattttaaa cttgtatgtg tgttgttatc c 1001
<210> 8
   <211> 1001
   <212> DNA
   <213> Homo sapiens
<220>
   <223> Chromosomale Region von Nukleotid 51508000 bis 51885000 von Chromosom 2 mit T an der Position von rs2374341
<220>
   <221> variation
   <222> 501
   <223> /allele="rs2374341"
<400> 8
   ccgttgctcc aggtgacata gagcaaacca ccagacgtga gtgtgaggcc actctagact      60
   gtccatcccc agccaggaca gtctgtacca gacaatctac ctagcctatc cccagaatag     120
   taagaccata ttctgttaag tcagtacatt ttgggagtag tttattatac agcaaaagct     180
   agctgataca agaatgcaga aaagtccatt catttctccc aaaaaacgcc tctcaaatca     240
   catatttcag gcaaaggact ggctcctctt tgtcaatgac ttctgaaaac tgtccatttt     300
   ttttctaaca tttcataaca tctattgatt cttcctttgg acactcacct accttaggag     360
   aaaaaaaaaa ttcacagctc tggccaagat aggagctggt cctatcagag ctatgtacaa     420
   aggaatcgga acaggtacag tggaacccaa gataagcatg tgatggcaaa gtcagagggg     480
   ctgcactgcc aatgttgaac tgggatagac aagagcataa caaagaagac accagggcag     540
   aaaacaccct aagcagcttt gctctgaact ctcccctctg caagaaccct tcacccagcc     600
   cagggatcgg aaactcaatg gagagccggt aagcacagta agaaagagtt tacagcttcc     660
   ttcctcaaaa gcttccaaga taaacaaaat aaacaccagt tggtctcagc tggtcgcaca     720
   ttggcctgcc tggaggaggg ggatgccaga gatagcttct tgaggttgct tccagcattg     780
   cattctagga aatagacaat tatgaacaac atggatttta ttatcgtgac atctctaagg     840
   tctctgcata aagcctagat ttaaaaatat aaacatgtaa gtgaaaaaat gtcaggggaa     900
   taaatatttg tcttaaaaga gaccatctgt ttcaaatttt tatttaaaaa aaaaaaaccc     960
   aacatgtgtt ttattttaaa cttgtatgtg tgttgttatc c                        1001
<210> 9
   <211> 31
   <212> DNA
   <213> Artificial Sequence
<220>
   <223> Vorwaertsprimer zur Amplifikation einer bevorzugten chromosomalen Region umfassend rs496888
<400> 9
   acgttggatg caccaaatgt acccttcact c 31
<210> 10
   <211> 28
   <212> DNA
   <213> Artificial Sequence
<220>
   <223> Rueckwaertsprimer zur Amplifikation einer bevorzugten chromosomalen Region umfassend rs496888
<400> 10
   acgttggatg ctgcttgaca gcctggtc 28
<210> 11
   <211> 20
   <212> DNA
   <213> Artificial Sequence
<220>
   <223> Sequenz einer bevorzugten Polynukleotidsonde zur Bestimmung des Genotyps an rs496888
<400> 11
   cacagccttt gccacaatac 20
<210> 12
   <211> 19
   <212> DNA
   <213> Artificial Sequence
<220>
   <223> Polynukleotidsonde umfassend die Sequenz der SEQ ID NO. 11
<400> 12
   cacgcctttg ccacaatac 19
<210> 13
   <211> 31
   <212> DNA
   <213> Artificial Sequence
<220>
   <223> Vorwaertsprimer zur Amplifikation einer bevorzugten chromosomalen Region umfassend rs7162960
<400> 13
   acgttggatg taatgatgcc atagaccact t 31
<210> 14
   <211> 32
   <212> DNA
   <213> Artificial Sequence
<220>
   <223> Rueckwaertsprimer zur Amplifikation einer bevorzugten chromosomalen Region umfassend rs7162960
<400> 14
   acgttggatg tcagactatt gccctctaag aa 32
<210> 15
   <211> 28
   <212> DNA
   <213> Artificial Sequence
<220>
   <223> Sequenz einer bevorzugten Polynukleotidsonde zur Bestimmung des Genotyps an rs7162960
<400> 15
   gcagaggtac acagatcttc agtcttaa 28
<210> 16
   <211> 28
   <212> DNA
   <213> Artificial Sequence
<220>
   <223> Polynukleotidsonde mit der Sequenz der SEQ ID NO. 15, umfassend eine Biotinylierung und eine Photospaltstelle
<400> 16
   gcagaggta cacagacttc agtcttaa 28
<210> 17
   <211> 30
   <212> DNA
   <213> Artificial Sequence
<220>
   <223> Vorwaertsprimer zur Amplifikation einer bevorzugten chromosomalen Region umfassend rs2715079
<400> 17
   acgttggatg gttttttcag aggatagggg 30
<210> 18
   <211> 30
   <212> DNA
   <213> Artificial Sequence
<220>
   <223> Rueckwaertsprimer zur Amplifikation einer bevorzugten chromosomalen Region umfassend rs2715079
<400> 18
   acgttggatg cttaacaagt cctcaagttc 30
<210> 19
   <211> 28
   <212> DNA
   <213> Artificial Sequence
<220>
   <223> Sequenz einer bevorzugten Polynukleotidsonde zur Bestimmung des Genotyps an rs2715079
<400> 19
   gggaacatta taacaactac acaacata 28
<210> 20
   <211> 27
   <212> DNA
   <213> Artificial Sequence
<220>
   <223> Polynukleotidsonde mit der Sequenz der SEQ ID NO. 19, umfassend eine Biotinylierung und eine Photospaltstelle
<400> 20
   gggaacatta taacaactca caacata 27
<210> 21
   <211> 31
   <212> DNA
   <213> Artificial Sequence
<220>
   <223> Vorwaertsprimer zur Amplifikation einer bevorzugten chromosomalen Region umfassend
<400> 21
   acgttggatg ctggtgtctt ctttgttatg c 31
<210> 22
   <211> 30
   <212> DNA
   <213> Artificial Sequence
<220>
   <223> Rueckwaertsprimer zur Amplifikation einer bevorzugten chromosomalen Region umfassend rs2374341
<400> 22
   acgttggatg gcatgtgatg gcaaagtcag 30
<210> 23
   <211> 25
   <212> DNA
   <213> Artificial Sequence
<220>
   <223> Sequenz einer Polynukleotidsonde zur Bestimmung des Genotyps an rs2374341
<400> 23
   tctttgttat gctcttgtct atccc 25
<210> 24
   <211> 25
   <212> DNA
   <213> Artificial Sequence
<220>
   <223> Polynukleotidsonde mit der Sequenz der SEQ ID NO. 23, umfassend eine Biotinylierungund einer Photospaltstelle
<400> 24
   tctttgttat gctcttgtct atccc 25

## Beschreibung

### Technisches Gebiet der Erfindung

Die vorliegende Erfindung bezieht sich auf ein Verfahren zur *in vitro* Diagnose eines Legasthenierisikos bei einem Menschen, bei dem die DNS des besagten Menschen in regulatorischen und/oder kodierenden Regionen mindestens eines Gens oder einer spezifischen Region mindestens einer genetischen Variante analysiert wird, das aus einer Gruppe von spezifischen chromosomalen Regionen ausgewählt ist.

Die vorliegende Erfindung beschäftigt sich ferner mit der Anwendung eines solchen Verfahrens zur Diagnose eines Legasthenierisikos in einer frühen Phase in der Entwicklung eines Menschen. Außerdem kann ein Verfahren gemäß der Erfindung benutzt werden, um den Verdacht auf Legasthenie bei einem Menschen zu erhärten oder um Menschen zu identifizieren, die ein erhöhtes Risiko für eine Legasthenie haben.

### Hintergrund der Erfindung

Legasthenie kann allgemein als Krankheit beschrieben werden, die die Lese- und Schreibfähigkeiten eines Menschen beeinflusst. Ansonsten scheint ein Individuum, das an Legasthenie leidet, eine normale Entwicklung zu zeigen, einschließlich einer normalen intellektuellen Entwicklung, wie z.B. Gedächtnis- oder Lernfertigkeiten.

Legasthenie kann sich in verschiedenen Phänotypen widerspiegeln, so z.B. Lese- und Rechtschreibstörung, isolierter Rechtschreibstörung, Rechenstörung und eine kombinierten Störung schulischer Fertigkeiten (Beeinträchtigung des Lesens, Schreibens und Rechnens). Zu unterschiedlichen Stadien der schulischen Entwicklung können unterschiedliche Symptome auftreten, wie Probleme beim Aufsagen des Alphabets, der Benennung von Buchstaben oder dem Bilden von Reimen, Lese- und Schreibprobleme (Auslassen, Verdrehen oder Hinzufügen von Wörtern oder Wortteilen; niedrige Lesegeschwindigkeit; Ersetzen von Buchstaben, Silben und Wörtern; Startschwierigkeiten beim Vorlesen, langes Zögern oder Verlieren der Zeile im Text; Vertauschen von Wörtern im Satz oder von Buchstaben in den Wörtern; Schwierigkeiten bei Doppellauten; Unfähigkeit, Gelesenes wiederzugeben, aus Gelesenem Schlüsse zu ziehen oder Zusammenhänge zu sehen; Gebrauch allgemeinen Wissens anstelle der Textinformationen beim Beantworten von Fragen.

Aufgrund des spezifischen Phänotyps der Erkrankung beginnen sich die Symptome der Legasthenie üblicherweise erst in einer späten Phase der Entwicklung zu manifestieren, nämlich wenn Lese- und Schreibfähigkeiten benötigt und/oder gelehrt werden. Symptome der Legasthenie können typischerweise während der ersten Jahre bemerkt werden, die ein Kind im Alter von ungefähr 5 bis 8 Jahren in der Schule verbringt.

Klar ersichtlich leiden die betroffenen Kinder schon während dieser ersten Schuljahre unter Nachteilen, da sie nicht in der Lage sind, die Lese- und/oder Schreibaufgaben zu meistern, mit denen sie konfrontiert sind. Ferner kann dies von sozialem Ausschluss aufgrund besagten Scheiterns begleitet sein.

Auch in späteren Phasen ihres Lebens ist es sehr wahrscheinlich, dass legasthene Personen an den mit Legasthenie verknüpften Symptomen leiden und bei vielen Anforderungen ihres Alltagslebens beeinträchtigt sein können.

Die spezifischen molekularen Geschehnisse, die der Legasthenie zu Grunde liegen, sind noch nicht verstanden. Jedoch scheint es, dass eine genetische Prädisposition eine wichtige Rolle bei der Entstehung von Legasthenie spielt. Verschiedene Linkage-Studien führten zur Identifikation von Genen, die mit Legasthenie assoziiert zu sein scheinen. Besagte Gene liegen in chromosomalen Regionen, die in experimentellen Studien kartiert wurden. Für einen Überblick zu genetischen Komponenten der Legasthenie siehe Raskind, et al.; Front Psychol. 2012: "The genetics of reading disabilities: from phenotypes to candidate genes".

Es ist weithin als ein Grundprinzip anerkannt, dass spezifische medizinische und/oder pädagogische Behandlung mit dem Ziel der Linderung der Legastheniesymptome und der Krankheit selbst so früh wie möglich in der Entwicklung einer Person beginnen sollte.

Ein Verfahren zur Diagnose eines Legasthenierisikos sollte vorzugsweise so gestaltet sein, dass es so früh wie möglich durchgeführt werden kann, um frühzeitig den Folgen entgegenzuwirken bzw. den Betroffenen entsprechende Hilfe zu bieten.

Herkömmliche Diagnoseverfahren werden jedoch vor allem ausgeführt, nachdem sich die ersten Symptome manifestieren und somit erst nach dem ersten oder zweiten Schuljahr. Schon zu diesem Zeitpunkt kann, wie oben ausgeführt, ein Kind an den Nachteilen von Legasthenie leiden.

Ferner können einige Symptome der Legasthenie auch mit anderen Krankheiten wie dem Aufmerksamkeitsdefizitsyndrom (ADS) oder Hyperaktivität assoziiert sein.

Heutzutage werden im Fall eines unklaren Krankheitsbildes aufwändige Tests durchgeführt, um die entsprechende und zu Grunde liegende Krankheit eindeutig zuzuweisen. Jedoch führen viele Tests zu falsch-negativen und auch zu falsch-positiven Ergebnissen. Es ist daher erforderlich, ein Verfahren bereitzustellen, das frühzeitig ein Legasthenierisiko spezifisch und unabhängig von anderen verwandten Krankheiten erkennt.

Neben den individuellen Vorteilen sind auch die volkswirtschaftlichen Vorteile einer Frühdiagnostik zu betrachten. Bei einer Legasthenieprävalenz von ca. 4-5% aller deutschen Schüler (Schulte-Köme & Remschmidt, 2003) und den gegenwärtigen Einschulungen (>600.000) ist mit mehr als 25.000 neu hinzukommenden Betroffenen pro Jahr zu rechnen. Die volkswirtschaftlichen Kosten sind als immens anzusehen. So betragen die Beschulungskosten in speziellen Legasthenie-Klassen, d.h. Klassen für leserechtschreibschwache Kinder, 11.000 Euro pro Kind und Jahr, vergleichen mit 4.900 Euro für eine normale Beschulung. Addiert man sämtliche direkten und indirekten Kosten auf (Guggisberg et al., 2007), so kommt man für Deutschland auf mindestens 3.5 Milliarden Euro pro Jahr (siehe auch 4.3). Diese Kosten könnten durch eine frühzeitige Diagnose und die dadurch ermöglichte rechtzeitige Intervention nachhaltig gesenkt werden.

Sun et al. (American Journal of Medical Genetics 165(8):627-634 (2014)) beschreibt eine Assoziationsstudie von entwicklungsbedingter Dyslexie und den Kandidatengenen DCDC2 und KIAA319.
Mueller et al. (Genes Brain and Behavior 13(3):350-356 (2014)) beschreibt genetische Risikovarianten für Dyslexie auf dem Chromosom 18.
In WO 2010/007063 A2 wird ein Verfahren zu Diagnose von Dyslexie beschrieben, das auf der Analyse von Genen beruht, die an neuronaler Migration beteiligt sind.
In WO 2005/049796 A2 werden genetische Varianten auf dem Chromosom 5 beschrieben, die in Zusammenhang mit Dyslexie stehen.
Paracchini et al. (Annual Review of Genomics and human Genetics 8(1):57-79 (2007)) ist ein Übersichtsartikel, der Marker auf den Chromosomen 15, 3 und 6 zusammenfasst, die mit Legasthenie in Zusammenhang gebracht werden können.
Gibson et al. (Journal of Communication Disorders 41(5):409-420 (2008)) beschreibt Marker für ein Legasthenierisiko auf den Chromosomen 15, 6, 3 und 18.

Allgemein umfassen herkömmliche Tests unter anderem Lese- und Rechtschreibtests und können sich über mehrere Tage erstrecken. Die Auswertung und Durchführung erfordert vor Ort anwesendes hochgradig qualifiziertes Fachpersonal. Folglich können sie teuer und zeitaufwändig sein.

Es besteht also Bedarf für ein Verfahren, um ein Legasthenierisiko so früh möglich im Leben eines Individuums spezifisch zu diagnostizieren, welches leicht durchzuführen sowie zeit- und kosteneffizient ist.

### Beschreibung der Erfindung

Ein Ziel der vorliegenden Erfindung ist es, ein Verfahren bereitzustellen, das es erlaubt, Legasthenie unabhängig vom Alter des in Verdacht stehenden Individuums diagnostizieren zu können.

Insbesondere ist es ein Ziel der vorliegenden Erfindung ein Verfahren bereitzustellen, um ein erhöhtes Legasthenierisiko so früh wie möglich zu erkennen und das darüber hinaus leicht durchzuführen und zeit- und kosteneffektiv ist. Die Erfinder der vorliegenden Erfindung haben überraschenderweise festgestellt, dass es möglich ist, in einem frühen Stadium der Entwicklung eines Menschen festzustellen, dass dieser an einem erhöhtem Legasthenierisiko leidet, wenn dieser einen Genotyp in mindestens einem von drei genomischen Regionen aufweist, der von den einer Kontrollgruppe abweicht. Hierdurch stellen die Erfinder ein Verfahren bereit, das zur Lösung der genannten Aufgaben beiträgt und somit für eine frühe Behandlung und Förderung von Patienten mit Legasthenie von großer Bedeutung ist. Bei Vorliegen eines abweichenden Genotyps, gemäß des erfindungsgemäßen Verfahrens, lässt sich je nach der Zahl der individuell vorliegenden genetischen Risikovarianten eine Risikoerhöhung quantifiziert als Odds Ratio (OR) im Vergleich zu einer Kontrollgruppe von bis zu 3.07 (95% Konfidenzintervall 1.57 - 6.25) prädizieren. Dieses hohe Maß an vorhergesagtem Risiko lässt sich dadurch erreichen, dass die vorliegende Erfindung ein Set an chromosomalen Regionen bereitstellt, bei denen das Vorliegen von Abweichungen in einem entsprechenden Maße indikativ für das Vorliegen von Legasthenie in dem zu diagnostizierenden Menschen ist. Die vorliegende Erfindung trägt somit zur Lösung der beschriebenen Aufgaben bei. In der folgenden Beschreibung werden die Gegenstände der Erfindung näher dargelegt.

Die vorliegende Erfindung betrifft die in den Ansprüchen gekennzeichneten Ausführungsformen. Somit betrifft die vorliegende Erfindung die folgenden Gegenstände 1 bis 10:
(1). Verfahren zur Diagnose eines Legasthenierisikos, umfassend die Schritte:
   a) Bereitstellung einer Nukleinsäure beinhaltenden Probe von einem zu diagnostizierenden Menschen,
   b) Bestimmung des Genotyps der Nukleinsäure der Probe für die chromosomale Region von Nukleotid 12091000 bis 12200000 des Chromosoms 1;
   c) Vergleich des in Schritt b) bestimmten Genotyps mit dem Genotyp einer Kontrolle zur Feststellung von Abweichungen des bestimmten Genotyps vom Genotyp der Kontrolle;
   d) Zuschreiben einer Präsenz eines Legasthenierisikos in der zu diagnostizierenden Person bei Vorliegen mindestens einer festgestellten Abweichung des bestimmten Genotyps vom Genotyp besagter Kontrolle in der besagten Region, wobei besagte Abweichung SNP rs496888 ist, und wobei das Vorliegen eines A für mindestens ein Allel von rs496888 der Präsenz eines Legasthenierisikos in der zu diagnostizierenden Person zugeschrieben wird.
(2). Verfahren nach (1), wobei besagte Abweichung in mindestens zwei SNP ist, ausgewählt aus:
   (i) rs496888 und rs7162960;
   (ii) rs496888 und rs2715079; und/oder
   (iii) rs496888 und rs2374341 ist;
   wobei das Vorliegen eines C für mindestens ein Allel von rs7162960, das Vorliegen eines C für mindestens ein Allel von rs2715079 oder das Vorliegen eines T für mindestens ein Allel von rs2374341 der Präsenz eines Legasthenierisikos in der zu diagnostizierenden Person zugeschrieben wird.
(3). Verfahren nach (1) oder (2), wobei das Zuschreiben einer Präsenz eines Legasthenierisikos in der zu diagnostizierenden Person bei homozygotem Vorliegen besagter Abweichung(en) des bestimmten Genotyps vom Genotyp besagter Kontrolle erfolgt.
(4). Verfahren gemäß irgendeinem von (1) bis (3), wobei die DNS enthaltende Probe ausgewählt ist aus der Gruppe bestehend aus Gewebeprobe, Körperflüssigkeiten, Blut, Haut, Haar, Nagel und Speichel.
(5). Verfahren gemäß irgendeinem von (1) bis (4), wobei Schritt b) die Aufreinigung und/oder Amplifizierung der DNS in besagter Probe umfasst, bevorzugt die Amplifizierung besagter Regionen oder Teilen davon.

Zudem ist offenbart ein Verfahren zur Diagnose eines Legasthenierisikos, umfassend die Schritte: a) Bereitstellung einer Nukleinsäure beinhaltenden Probe von einem zu diagnostizierenden Menschen, b) Bestimmung des Genotyps der Nukleinsäure der Probe für mindestens eine Region, ausgewählt aus der Gruppe bestehend aus der Region von Nukleotid 12091000 bis 12200000 des Chromosoms 1, der Region von Nukleotid 89066000 bis 89088000 des Chromosoms 15, der Region von Nukleotid 41930000 bis 42032000 des Chromosom 2 und der Region von Nukleotid 51508000 bis 51885000 des Chromosom 2; c) Vergleich des in Schritt b) bestimmten Genotyps mit dem Genotyp einer Kontrolle zur Feststellung von Abweichungen des bestimmten Genotyps vom Genotyp der Kontrolle; d) Zuschreiben der Präsenz eines erhöhten Legasthenierisikos in der zu diagnostizierenden Person bei Vorliegen mindestens einer festgestellten Abweichung des bestimmten Genotyps vom Genotyp besagter Kontrolle in mindestens einer der besagten Regionen.

In einer bevorzugten Ausführungsform ist die Nukleinsäure, welche in der Probe beinhaltet ist, eine Desoxyribonukleinsäure (DNS).
Somit wird ein Verfahren zur Diagnose eines Legasthenierisikos offenbart,
umfassend die Schritte: a) Bereitstellung einer DNS beinhaltenden Probe von einem zu diagnostizierenden Menschen, b) Bestimmung des Genotyps der DNS der Probe für mindestens eine chromosomale Region, ausgewählt aus der Gruppe bestehend aus der Region von Nukleotid 12091000 bis 12200000 des Chromosoms 1, der Region von Nukleotid 89066000 bis 89088000 des Chromosoms 15, der Region von Nukleotid 41930000 bis 42032000 des Chromosom 2 und der Region von Nukleotid 51508000 bis 51885000 des Chromosom 2; c) Vergleich des in Schritt b) bestimmten Genotyps mit dem Genotyp einer Kontrolle zur Feststellung von Abweichungen des bestimmten Genotyps vom Genotyp der Kontrolle; und d) Zuschreiben einer Präsenz eines erhöhten Legasthenierisikos in dem zu diagnostizierenden Menschen bei Vorliegen mindestens einer festgestellten Abweichung des bestimmten Genotyps vom Genotyp besagter Kontrolle in mindestens einer der besagten Regionen.

Das offenbarte Diagnoseverfahren kann auch in eine Behandlung eingebettet sein. So ist auch ein Verfahren zur Behandlung von Legasthenie offenbart,
umfassend die Schritte a) Bereitstellung einer DNS beinhaltenden Probe von einem zu behandelnden Menschen, b) Bestimmung des Genotyps der DNS der Probe für mindestens eine chromosomale Region, ausgewählt aus der Gruppe bestehend aus der Region von Nukleotid 12091000 bis 12200000 des Chromosoms 1, der Region von Nukleotid 89066000 bis 89088000 des Chromosoms 15, der Region von Nukleotid 41930000 bis 42032000 des Chromosom 2 und der Region von Nukleotid 51508000 bis 51885000 des Chromosom 2; c) Vergleich des in Schritt b) bestimmten Genotyps mit dem Genotyp einer Kontrolle zur Feststellung von Abweichungen des bestimmten Genotyps vom Genotyp der Kontrolle; d) Zuschreiben einer Präsenz eines erhöhten Legasthenierisikos in dem zu diagnostizierenden Menschen bei Vorliegen mindestens einer festgestellten Abweichung des bestimmten Genotyps vom Genotyp besagter Kontrolle in mindestens einer der besagten Regionen; und e) Behandlung von besagtem Menschen mit Mitteln und/oder Verfahren zur Behandlung von Legasthenie nach Zuschreiben einer Präsenz eines erhöhten Legasthenierisikos in besagtem Menschen.

Mittel und Verfahren zur Behandlung von Legasthenie sind dem Fachmann bekannt. Mittel und Behandlungsmethoden umfassen im Frühförderbereich vor allem das Training der phonologischen Bewusstheit, die eine zentrale Vorläuferfertigkeit für das Erlernen von Lesen und Schreiben darstellt (z.B. das Würzburger Trainingsprogramm, Küspert & Schneider, 2006) und im schulischen Bereich das Erlernen der Zuordnung von Buchstaben zu Lauten (Graphem-Phonem-Korrespondenz) sowie das regelgeleitete Erlernen der Schrift. Weitere Maßnahmen zur Unterstützung legasthener Schüler sind: Unterstützung, Beratung, Hilfe und Annahme der Situation von Eltern, Schule und Fachleuten zum Erkennen und zur Bestimmung des eigenen (anderen) Lernstils des Schüler; Lernstrategien, um Schwächen durch Stärken auszugleichen; multisensorische Therapie, in dem alle Sinnesorgane wie Hören, Sehen, haptische Erfahrungen (Fühlen, Greifen), und daneben Gedächtnis, Konzentration, sprachliche Fähigkeiten im Zuhören, Antworten und Gespräch gefördert werden (geeignete Hilfsmittel hierzu sind der Umgang mit entsprechenden Computerprogrammen, Hörbücher, Vorlesen, und Lernprogramme, die reichhaltig angeboten werden für lese- und schreibschwache Schüler; Unterstützung durch Programme mit automatischer Fehlerkorrektur und/oder Vorlese-Programme, bei dem der Computer das Lesen übernimmt; bereitstellen von Büchern und Texten mit Textvergrößerung und/oder mit farblichen Hervorhebungen; Ausarbeiten von Alltagsstrukturen für den Legastheniker (Tagesablauf und Lernstruktur).

Mit dem erfindungsgemäßen Diagnoseverfahren ist es möglich die Diagnose eines erhöhten Legasthenierisikos unabhängig von anderen Diagnoseverfahren durchzuführen. Weiterhin ist das erfindungsgemäße Detektionsverfahren nur auf genetisches Material angewiesen; daher ist vorzugsweise kein weiterer Beitrag des zu testenden Individuums notwendig. Allerdings kann es hilfreich sein, die Diagnose gemäß der vorliegenden Erfindung durch weitere Verfahren zu unterstützen. Das erfindungsgemäße Verfahren kann zur Legasthenierisikodiagnose genutzt werden, zum Beispiel wenn es während einer Routinevorsorgeuntersuchung für Kinder verwendet wird. In einer Ausführungsform kann das Verfahren zur Behandlung von Legasthenie zwischen Schritt d) und e) einen weiteren Schritt enthalten, in dem weitere Verfahren zur Diagnose eines Legasthenierisikos durchgeführt werden. Dies kann auch psychometrische Ergänzungsverfahren beinhalten. Solche Verfahren sind dem Fachmann bekannt (Breuer, H. & Weuffen, M. (1994). Lernschwierigkeiten am Schulanfang. Schuleingangsdiagnostik zur Früherkennung und Frühförderung. Weinheim, Beltz; und ; Jansen, H., Mannhaupt, G., Marx, H., Skowronek, H. (2006) BISC Bielefelder Screening zur Früherkennung von Lese-Rechtschreibschwierigkeiten. 2. überarbeitete Auflage. Göttingen, Hogrefe).

Darüber hinaus kann zwischen Schritt d) und e) des Verfahrens zur Behandlung gemäß der vorliegenden Erfindung ein oder mehrere Schritte erfolgen, in dem das Vorliegen von Aufmerksamkeitsdefizitsyndrom (ADS) und/oder Hyperaktivität und/oder eines IQ von weniger als 80, vorzugsweise ein IQ von weniger als 85, ausgeschlossen wird. In einer Ausführungsform des Diagnoseverfahrens gemäß der vorliegenden Erfindung umfasst das Diagnoseverfahren weiter einen oder mehrere Schritte zum Ausschluss von Aufmerksamkeitsdefizitsyndrom (ADS) und/oder Hyperaktivität und/oder ADHS und/oder eines IQ von weniger als 80, vorzugsweise weniger als ein IQ von 85. Solche Schritte und Verfahren sind auf dem Fachgebiet bekannt.

Im Kontext der vorliegenden Erfindung wird der Begriff "Legasthenie" so verwendet, wie er auf dem Gebiet der Medizin bekannt und definiert ist. "Legasthenie" beschreibt eine komplexe, durch unerwartete Schwierigkeiten beim Erlernen von Lesen und/oder Schreiben und/oder Verstehen von Wörtern/Texten und/oder Buchstabieren charakterisierte Störung bei ansonsten normaler Intelligenz. Die Krankheit kann sich in verschiedenen Schweregraden manifestieren, von denen alle durch den hier benutzten Begriff "Legasthenie" umfasst werden sollen. Es kann bevorzugt sein, den Begriff "Legasthenie" wie hier verwendet mit Leseschwäche zu assoziieren. Der Begriff "Legasthenie" wie hier verwendet kann jedoch auch mit allen Störungen und/oder Problemen beim Schreiben assoziiert werden. Im Falle, dass eine genetische Komponente für die Anfälligkeit eines Individuums für eine Krankheit gezeigt wurde, kann es mehrere Vorteile geben, eine Diagnose eines erhöhten Risikos auf besagter genetischer Komponente basieren zu lassen. Dies wird insbesondere durch die vorliegende Erfindung bereitgestellt.

Weiter ist ein Kit zur Durchführung eines erfindungsgemäßen Verfahrens offenbart, insbesondere zur Durchführung des erfindungsgemäßen Diagnoseverfahrens, wobei der Kit Mittel zur Bestimmung des Genotyps für mindestens eine chromosomale Region umfasst, wobei besagte Region ausgewählt ist aus der Gruppe bestehend aus der Region von Nukleotid 12091000 bis 12200000 des Chromosoms 1, der Region von Nukleotid 89066000 bis 89088000 des Chromosoms 15, der Region von Nukleotid 41930000 bis 42032000 des Chromosoms 2 und der Region von Nukleotid 51508000 bis 51885000 des Chromosoms 2 (Positionen gemäß hg18). In einer bevorzugten Ausführungsform umfasst das erfindungsgemäße Kit einen oder mehrere Polynukleotidprimer und/oder mehrere Polynukleotidsonden. Diese Polynukleotidprimer und/oder Polynukleotidsonde haben in einer besonders bevorzugten Ausführungsform die Merkmale der Polynukleotidprimer bzw. - sonden, wie hierin ausgeführt.

Weiter ist eine Polynukleotidsonde offenbart, die zur Bestimmung der
zu detektierenden Genotypen geeignet ist.
Die Polynukleotidsonde kann eine Sequenz ausgewählt aus der Gruppe bestehend
aus SEQ ID NO. 11, SEQ ID NO. 15, SEQ ID NO. 19 und SEQ ID NO. 23 sein. Dem Fachmann ist ersichtlich, dass die Länge der Polynukleotidsonden entsprechend des verwendeten Verfahrens angepasst werden kann. Dabei ist lediglich zu beachten, dass eine spezifische Hybridisierung an den zu detektierenden Genotyp erfolgt. Diese Hybridisierung hat spezifisch unter den gewählten Bedingungen zu erfolgen. Dem Fachmann ist bewusst, dass die Detektion von Polymorphismen mittels einer Polynukleotidsonde unter Bestimmung der Stärke der Hybridisierung der Sonde an die Zielsequenz erfolgen kann. Beispielsweise erniedrigt sich die Schmelztemperatur, wenn es innerhalb der Sonde einen Mismatch gibt. Dies ist z.B. dann der Fall, wenn die Sonde die Sequenz des komplementären Strangs des "Major"-Zustands eines SNPs aufweist, während die Probe den "Minor"-Zustand aufweist. Dem Fachmann sind Verfahren und Mittel geläufig die Schmelztemperatur (Tₘ) einer Sonde zu bestimmen. Insbesondere können hierzu die Techniken der quantitativen PCR verwendet werden. Dabei können zur Detektion der Hybridisierung verschiedene Mittel eingesetzt werden, wie z.B. interkalierende Farbstoffe oder modifizierte Sonden.

Die Polynukleotidsonden ausgewählt aus der Gruppe bestehend aus SEQ ID NO. 11, SEQ ID NO. 15, SEQ ID NO. 19 und SEQ ID NO. 23 können weitere Modifikationen ausgewählt aus der Gruppe bestehend aus Fluoreszenzfarbstoffen, wie FAM/TAMRA (Blanchard, P., Guillot, S., Antùnez, K., Köglberger, H., Kryger, P., de Miranda, J.R., Franco, S,, Chauzat, M.P., Thiéry, R., Ribière, M. (2014) Development and validation of a real-time two-step RT-qPCR TaqMan(®) assay for quantitation of Sacbrood virus (SBV) and its application to a field survey of symptomatic honey bee colonies. J Virol Methods, 197, 7-13), TET/MGB (Yoshitomi, K.J., Jinneman, K.C., Weagant, S.D. (2003). Optimization of a 3'-minor groove binder-DNA probe targeting the uidA gene for rapid identification of Escherichia coli O157:H7 using real-time PCR. Mol Cell Probes, 17(6), 275-80), VIC/QSY (Nicklas, J.A., Buel, E. (2003). Development of an Alu-based, QSY 7-labeled primer PCR method for quantitation of human DNA in forensic samples. J Forensic Sci, 48(2),282-91), Biotin, und Photospaltstellen umfassen.

Dem Fachmann ist ersichtlich, dass es möglich ist, die in dieser Offenbarung beschriebenen Zustände bestimmter SNPs und das damit korrelierende Risiko für Legasthenie nicht direkt über die benannten SNPs zu identifizieren, sondern über korrelierende Reporter-SNPs. Solche korrelierenden Reporter-SNPs zeichnen sich dadurch aus, dass sie in ihrem Zustand mit einem bestimmten Zustand anderer SNPs korrelieren. Diese Korrelation wird auch mit "Kopplungsungleichgewicht" beziehungsweise dem englischen Begriff "linkage disequilibrium" bezeichnet. Dies ist eine nicht zufällige Assoziation von Allelen verschiedener Loci, wobei eine andere statistische Korrelation zwischen den Allelen verschiedener Loci vorliegt, als die, die man für unabhängige, zufällige Verteilung der Allele aufgrund ihrer individuellen Allelfrequenz annehmen würde (siehe auch Slatkin, Montgomery (June 2008). "Linkage disequilibrium - understanding the evolutionary past and mapping the medical future", Nature Reviews Genetics 9 (6): 477-485). Das Kopplungsungleichgewicht D zwischen zwei Allelen A und B wird berechnet, indem man die Wahrscheinlichkeit des Auftretens von Allel A in einer Population, multipliziert mit der Wahrscheinlichkeit des Auftretens von Allel B in dieser Population von der Wahrscheinlich des gemeinsamen Auftretens von Allel A und Allel B subtrahiert. Weicht das erhaltene Ergebnis signifikant von 0 ab, so besteht ein Kopplungsungleichgewicht. Um ein von der jeweiligen Allelfrequenz unabhängiges Maß zu erhalten, bildet man auf Basis von D den Korrelationskoeffizienten R der betreffenden Loci. Dafür wird D dividiert durch die Wahrscheinlichkeit des Auftretens von Allel A multipliziert mit der Gegenwahrscheinlichkeit von Allel A multipliziert mit der Wahrscheinlichkeit des Auftretens von Allel B multipliziert mit der Gegenwahrscheinlichkeit von Allel B. Bevorzugt für Reporter-SNPs werden dabei Werte für R² von ≥ 0.0, bevorzugt ≥0,1, weiter bevorzugt ≥0,5, besonders bevorzugt ≥0.8.

Zudem offenbart ist die Verwendung eines beschriebenen Kits, eines beschriebenen Polynukleotidprimers oder einer beschriebenen Polynukleotidsonde zur Diagnose eines Legasthenierisikos. Dem Fachmann wird ersichtlich sein, dass sich die Merkmale des Kits, der Polynukleotidprimer und der Polynukleotidsonden aus dem tatsächlich im Verfahren zur Diagnose verwendeten Bestimmungsverfahrens zur Bestimmung des Genotyps ergeben.

Der Tabelle 4 sind die Sequenzen ebenfalls zu entnehmen. Darin ist auch dargestellt, für die Bestimmung welcher Genotypen diese besonders geeignet sind. Demnach kann für die Bestimmung des Genotyps das Vorhandensein eines der Genotypen aus Tabelle 4 bestimmt werden, d.h. einer oder mehrerer der Genotypen der SEQ ID NO. 1 oder 2 für die chromosomale Region von Nukleotid 12091000 bis 12200000 des Chromosoms 1, der SEQ ID NO. 3 oder 4 für die chromosomale Region von Nukleotid 89066000 bis 89088000 des Chromosoms 15, der SEQ ID NO 5 oder 6 für die chromosomale Region von Nukleotid 41930000 bis 42032000 des Chromosoms 2 und SEQ ID NO. 7 oder 8 für die chromosomale Region von Nukleotid 51508000 bis 51885000 des Chromosoms 2.

In besagtem Aspekt der Erfindung und in weiteren Aspekten wie nachfolgend erwähnt (wenn nicht explizit angegeben), kann die Bereitstellung der Probe des Menschen außerhalb des menschlichen Körpers stattfinden. Das Verfahren ist bevorzugt ein *in vitro* Diagnoseverfahren.

Wie hier verwendet bezeichnet der Begriff "Polymorphismus" Variationen innerhalb des Genoms von Individuen einer Population. Polymorphismen werden beispielsweise mit Sequenziertechniken detektiert. Jedoch können Polymorphismen auch mittels anderen sogenannten Genotypisierungstechniken, wie z.B. RFLP Analyse, bestimmt werden und wurden es bereits. Im Falle, dass nur ein Nukleotid innerhalb einer bestimmten Region abweicht, bezeichnet man dies als "Single Nucleotide Polymorphism" oder "SNP". Ein SNP kann für die Mehrheit der Population bezogen auf ein definiertes Nukleotid/Base den Zustand A haben ("major"), wohingegen in der Minderheit einer Population Zustand B ("minor") bezogen auf ein anderes Nukleotid/Base vorliegt. Jedoch kann sich die Region auch über zwei oder mehrere Nukleotide erstrecken, und sie kann eine Insertion oder Deletion sein. Polymorphismen im menschlichen Genom sind nicht notwendigerweise mit einem bestimmten Genotyp, d.h. einer Krankheit, verbunden. Jedoch stellen sie interessante "Ziel"-Stellen dar, die mit einer Krankheit wie Legasthenie assoziiert sein können, da sie Regionen darstellen, die in einer Population variieren, die unter anderem legasthene und nicht legasthene Individuen umfasst. Die Erfinder haben festgestellt, dass Polymorphismen in den erfindungsgemäßen Regionen mit dem Vorliegen von Legasthenie assoziiert sind.

Wie schon oben erwähnt können bestimmte Deletionen, Insertionen, Punktmutationen und/oder Translokationen in der DNS für bedeutende nachfolgende Effekte verantwortlich sein. Im Falle, dass ein Polymorphismus einen solchen nachfolgenden Effekt zu verursachen scheint (d.h. ein SNP führt zu einem Aminosäureaustausch an einer wesentlichen Stelle eines Proteins oder beeinflusst regulatorische Prozesse), kann er mit einer Krankheit assoziiert sein und kann vorzugsweise mit einem Verfahren gemäß der Erfindung analysiert werden. Es kann bevorzugt sein, Deletionen, Insertionen, Punktmutationen, Inversionen und/oder Translokationen in den kodierenden Regionen von Genen in den erfindungsgemäßen chromosomalen Regionen für die Analyse auszuwählen.

Man kann daher identifizierte und etablierte Polymorphismen in mindestens einer der genannten Regionen der wie oben erwähnt an Legasthenie beteiligten Gene analysieren. Daten über solche Polymorphismen können aus öffentlichen Datenbanken wie NCBI (http://www.ncbi.nlm.nih.gov) ermittelt werden. Es ist sehr wahrscheinlich, dass die Daten über Polymorphismen in chromosomalen Regionen während der nächsten Jahre zunehmen werden, wobei die Kosten für besagte Analysen sinken werden. Daher kann man sich bei der Analyse der in Tabelle 1 aufgelisteten Regionen nicht nur auf die gegenwärtig bekannten Polymorphismen beziehen, sondern auch alle Polymorphismen einschließen, die der Öffentlichkeit in Zukunft zugänglich sein werden. Im nächsten Schritt, wie schon oben für die Analyse erwähnt, kann man den Genotyp des zu testenden Individuums mit dem Genotyp eines Referenzindividuums (Kontrolle) beziehungsweise vorzugsweise einer großen Gruppe von Referenzindividuen (Kontrolle) vergleichen, die nicht an Legasthenie erkrankt sind. In den letzten Jahren, sogar Jahrzehnten, wurden große Anstrengungen unternommen, um solche Einzelnukleotid-Polymorphismen in Human-Genomen zu identifizieren. Inzwischen sind Datenbanken mit einer großen Anzahl von identifizierten SNPs dem Fachmann geläufig. Eine solche Datenbank ist die Datenbank dbSNP des NCBI (URL: www.ncbi.nlm.nih.gov/projects/snp/). Die in der vorliegenden Offenbarung angegebenen rs-Nummern beziehen sich auf die Einträge in der dbSNP NCBI-Datenbank.

Diese sind auch dem Fachmann geläufig.

Die besagten chromosomalen Regionen, ausgewählt aus der oben aufgelisteten Gruppe von Regionen, können hinsichtlich der Präsenz von Abweichungen, ausgewählt aus der Gruppe, bestehend aus Einzelnukleotid-Polymorphismen (engl. "Single nucleotide polymorphism" (SNP)), Deletionen, Insertionen, Translokationen und Inversionen analysiert werden. Diese Abweichungen können zu veränderten Genexpressionsleveln, Leserasterverschiebungen, Aminosäuresubstitutionen oder vorzeitigen Stopkodons und/oder anderen Mutationen führen. Solche Abweichungen können zu verschiedenen Resultaten führen. Beispielsweise kann eine Punktmutation in einem kodierenden Bereich dazu führen, dass das Genprodukt, d. h. das kodierte Protein, eine abweichende Aminosäure-Sequenz aufweist. Hierdurch kann das Protein in seiner Funktion beeinträchtigt oder gänzlich inaktiviert werden. Eine solche Mutation kann jedoch auch eine "Silent"-Mutation sein, d. h. nicht zu einer Änderung der Sequenz des kodierten Proteins führen oder zu einer Änderung der Proteinsequenz führen, die keine Auswirkung auf deren Funktion hat. Ebenso kann eine Änderung der Aminosäure-Sequenz des Proteins dazu führen, dass das Protein eine gesteigerte Aktivität im Hinblick auf seine Funktion ausübt. Darüber hinaus können die genannten Abweichungen auch in regulatorischen Bereichen der genannten chromosomalen Region liegen, d. h. zum Beispiel zur Veränderung der Sequenz von Bereichen führen, die die Expression der kodierten Gene regulieren. Dies kann zu einer Deregulierung der Expression führen, d. h. die Expressionslevel sind erhöht oder erniedrigt. In einer Ausführungsform der Erfindung führen die Abweichungen zu einer Inaktivierung, d. h. einer Inaktivierung des Genprodukts und/oder der Expression des Gens. Die Erfinder der vorliegenden Erfindung haben festgestellt, dass insbesondere eine Abweichung in einem der SNPs, ausgewählt aus der Gruppe bestehend aus rs496888, rs7162960, rs2715079 und rs2374341, die Diagnose eines Legasthenierisikos in einem frühem Stadium erlaubt. Daher ist in einer bevorzugten Ausführungsform besagte Abweichung ein SNP, ausgewählt aus der Gruppe bestehend aus rs496888, rs7162960, rs2715079 und rs2374341. In einer bevorzugten Ausführungsform wird des Vorliegen eines Genotyps an rs496888 mit einen cis-Effekt auf die Gene *TNFRSF1B* und/oder *MFN2* der Präsenz eines Legasthenierisikos in der zu diagnostizierenden Person zugeschrieben. In einer bevorzugten Ausführungsform wird das Vorliegen eines Genotyps an rs7162960 mit einem cis-Effekts auf *BLM* der Präsenz eines Legasthenierisikos in der zu diagnostizierenden Person zugeschrieben, ist. In einer bevorzugten Ausführungsform wird das Vorliegen eines Genotyps an rs2374341 mit einem cis-Effekt auf das Gen *EML4* der Präsenz eines Legasthenierisikos in der zu diagnostizierenden Person zugeschrieben. Der cis-Effekt ist vorzugsweise eine Erhöhung (bzgl. Gen *EML4* (Kirsten, H., Al-Hasani, H., Holdt, L., Gross, A., Beutner, F., Krohn, K., Horn, K., Ahnert, P., Burkhardt, R., Reiche, K., Hackermüller, J., Löffler, M., Teupser, S., Thiery, J., Scholz, M. (2015) Dissecting the Genetics of the Human Transcriptome identifies novel trait-related trans-eQTLs and corroborates the regulatory relevance of non-protein coding loci. Hum Mol Genet, pii: ddv194 [Epub ahead of print])) bzw. einer Erniedrigung der Expression des Gens (bzgl. Gene *TNFRSF1B* (Westra, H.J., Peters, M.J., Esko, T., Yaghootkar, H., Schurmann, C., Kettunen, J, Christiansen, M.W., Fairfax, B.P., Schramm, K., Powell, J.E., Zhernakova, A., Zhernakova, D.V., Veldink, J.H., Van den Berg, L.H., Karjalainen, J., Withoff, S., Uitterlinden, A.G., Hofman, A., Rivadeneira, F., 't Hoen, P.A., Reinmaa, E., Fischer, K., Nelis, M., Milani, L., Melzer, D., Ferrucci, L., Singleton, A.B., Hernandez, D.G., Nalls, M.A., Homuth, G., Nauck, M., Radke, D., Völker, U., Perola, M., Salomaa, V., Brody, J., Suchy-Dicey, A., Gharib, S.A., Enquobahrie, D.A., Lumley, T., Montgomery, G.W., Makino, S., Prokisch, H., Herder, C., Roden, M., Grallert, H., Meitinger, T., Strauch, K., Li, Y., Jansen, R.C., Visscher, P.M., Knight, J.C., Psaty, B.M., Ripatti, S., Teumer, A., Frayling, T.M., Metspalu, A., van Meurs, J.B., Franke, L. (2013) Systematic identification of trans eQTLs as putative drivers of known disease associations. Nat Genet, 45(10), 1238-1243.), *MFN2, BLM*).
Die oben in Schritt c) erwähnte Kontrolle kann einen oder mehrere nicht-legasthenen Menschen darstellen oder
zumindest mehrere Menschen, die zum überwiegenden Anteil nicht-legasthen sind. Der Fachmann wird erkennen, dass die Kontrolle auch Daten sein können, die zuvor gesammelt wurden. Diese Daten wurden vorzugsweise von erwiesenermaßen nicht-legasthenen Menschen gesammelt. Da sich der Genotyp im Verlauf eines Menschenlebens üblicherweise nicht ändert, können diese Proben auch von Menschen stammen, die nicht im selben Alter sind, wie die zu diagnostizierende Person. Es kann von Vorteil sein, wenn die Kontrolle von Menschen stammt, die bereits das Erwachsenenalter erreicht haben. Bei solchen Probanden ist es mit höherer Sicherheit möglich anhand von Anamnese das Vorliegen einer Legasthenie auszuschließen. Die Kontrolle kann jedoch auch parallel mit dem erfindungsgemäßen Verfahren durchgeführt werden. Auch dann kann es von Vorteil sein, einen Menschen im Erwachsenenalter als Kontrolle zu wählen, um auch hier Legasthenie der Kontrolle auszuschließen. Die Erfinder haben überraschenderweise festgestellt, dass für bestimmte SNP in den hierin genannten chromosomalen Regionen eine Diagnose eines Legasthenierisikos ohne direkten Vergleich mit einer Kontrolle möglich ist. Dies folgt aus der bereitgestellten Lehre, dass das von den Erfindern beobachtete gehäufte Auftreten eines Zustandes einer genetischen Variante in Legasthenikern im Vergleich zu nicht-legasthenen Menschen eine Aussage dazu ermöglicht, ob der diagnostizierte Mensch ein erhöhtes Legasthenierisiko aufweist. In einer Ausführungsform des erfindungsgemäßen Verfahrens wird das Vorliegen eines anderen Nukleotids als G für rs496888, Vorliegen eines anderen Nukleotids als T für rs7162960, Vorliegen eines anderen Nukleotids als T für rs2715079 oder das Vorliegen eines anderen Nukleotids als C für rs2374341 der Präsenz eines Legasthenierisikos in der zu diagnostizierenden Person zugeschrieben. Der Fachmann wird erkennen, dass nicht immer alle vier der genannten SNPs gemeinsam untersucht werden müssen, sondern, dass vielmehr auch einzelne SNPs untersucht werden können. In einer Ausführungsform wird das Vorliegen eines anderen Nukleotids als G für rs496888 der Präsenz eines Legasthenierisikos in der zu diagnostizierenden Person zugeschrieben. In einer Ausführungsform wird das Vorliegen eines anderen Nukleotids als T für rs7162960 der Präsenz eines Legasthenierisikos in der zu diagnostizierenden Person zugeschrieben. In einer Ausführungsform wird das Vorliegen eines anderen Nukleotids als T für rs2715079 der Präsenz eines Legasthenierisikos in der zu diagnostizierenden Person zugeschrieben. In einer Ausführungsform wird das Vorliegen eines anderen Nukleotids als C für rs2374341 der Präsenz eines Legasthenierisikos in der zu diagnostizierenden Person zugeschrieben.

Die Erfinder haben weiter festgestellt, dass die Präsenz von bestimmten allelischen Zuständen an den genannten SNPs besonders hohe Prädiktive Eigenschaften haben
Das Vorliegen eines A für rs496888, das Vorliegen eines C für rs7162960, das Vorliegen eines C für rs2715079 oder das Vorliegen eines T für rs2374341 kann der Präsenz eines Legasthenierisikos in der zu diagnostizierenden Person zugeschrieben. Der Fachmann wird erkennen, dass nicht immer alle vier der genannten SNPs gemeinsam untersucht werden müssen, sondern, dass vielmehr auch einzelne SNPs untersucht werden können. In einer Ausführungsform wird das Vorliegen eines A für rs496888 der Präsenz eines Legasthenierisikos in der zu diagnostizierenden Person zugeschrieben. In einer Ausführungsform wird das Vorliegen eines C für rs7162960 der Präsenz eines Legasthenierisikos in der zu diagnostizierenden Person zugeschrieben. In einer Ausführungsform wird das Vorliegen eines C für rs2715079 der Präsenz eines Legasthenierisikos in der zu diagnostizierenden Person zugeschrieben. In einer Ausführungsform wird das Vorliegen eines T für rs2374341 der Präsenz eines Legasthenierisikos in der zu diagnostizierenden Person zugeschrieben.

Weiterhin ist ein Verfahren zur Diagnose eines Legasthenierisikos bei einem Menschen beschrieben, umfassend die Schritte: a) Bereitstellung einer Probe
von besagtem Menschen, wobei diese Probe DNS umfasst; b) Bestimmung des Genotyps von mindestens einem in besagter DNS vorhandenen SNP ausgewählt aus der Gruppe bestehend aus rs496888, rs7162960, rs2715079 und rs2374341; c) Vergleich des Genotyps von besagtem mindestens einem SNP mit Legasthenie assoziierten Genotypen, wobei das Vorliegen eines oder mehrerer der Genotypen ausgewählt aus der Gruppe bestehend aus homozygot minor für rs496888, homozygot minor für rs7162960, homozygot minor für rs2715079 und homozygot minor für rs2374341 mit Legasthenie assoziiert sind; und d) Zuweisung von Legasthenierisiko zu besagtem Menschen basierend auf besagtem Vergleich. Weiterhin ist ein Verfahren zur Diagnose eines Legasthenierisikos beschrieben, umfassend die Schritte: a) Bereitstellung einer
Nukleinsäure umfassenden Probe von einem zu diagnostizierenden Menschen; b) Bestimmung des Genotyps von mindestens einem in besagter Nukleinsäure vorhandenen SNP ausgewählt aus der Gruppe von SNPs, bestehend aus rs496888, rs7162960, rs2715079 und rs2374341; c) Zuschreiben einer Präsenz eines Legasthenierisikos in der zu diagnostizierenden Person bei Vorliegen eines Genotypen, ausgewählt aus der Gruppe, bestehend aus homozygot C für rs7162960, homozygot A für rs496888, homozygot C für rs2715079 und homozygot T für rs2374341.
Der Fachmann ist mit seinem Fachwissen und der vorliegenden Erfindung in der Lage, Kombinationen der offenbarten "Biomarker" für Legasthenie zu wählen. Wie hierin dargelegt, kann es zur Erhöhung der Aussagekraft wünschenswert sein, den Genotyp von mehr als einer der genannten Regionen, bzw. SNPs zu bestimmen. Dem Fachmann sind statistische Verfahren geläufig anhand derer er die Anzahl und Kombinationen der erfindungsgemäßen Regionen und/oder SNPs zur Erzielung der gewünschten Aussagekraft bestimmen kann. Beispielsweise kann ein Summenscore gebildet werden, wobei das Auftreten einer Krankheit oder der Wahrscheinlichkeit des Vorliegens von Legasthenie mit der Anzahl von Abweichungen gemäß der vorliegenden Erfindung in der jeweiligen Bevölkerungsgruppe korreliert ist. Nach dieser Analyse haben Patienten (Probanden) bei denen in drei dieser SNPs mindestens ein Risikoallel vorliegt ein Risiko quantifiziert als Odds Ratio von 2.1 (95%Konfidenzintervall 1.16-4.12) und Patienten (Probanden), bei denen in vier dieser SNPs mindestens ein Risikoallel vorliegt ein Risiko quantifiziert als Odds Ratio von 3.1 (95%Konfidenzintervall 1.57-6.25). Mehr vorliegende Risikovarianten bedeutet damit eine höhere Wahrscheinlichkeit für das Vorliegen von Legasthenie. Der Fachmann kann die zu bestimmende Anzahl anpassen, bevorzugt liegt diese zum Beispiel bei mindestens einem Risikoallel in vier oder drei Riskovarianten. Durch die Verwendung von mehr vorliegenden Risikovarianten als reduziert man die Zahl von falsch-positiven Probanden, allerdings erhöht dies auch das Risiko von falsch-negativen Probanden. Der Fachmann wird also abwägen, welchen "Cut-off"-Wert er wählen muss. Dabei wird er abwägen, ob er möglichst alle Probanden mit Legasthenie identifizieren will und dabei das Risiko eingeht mehr falschpositive mit einzuschließen, oder ob er sicherer sein will, dass eine höherer Anteil der als ein Legasthenierisiko habend diagnostizierten Probanden auch tatsächlich an Legasthenie leiden und dabei das Risiko eingeht mehr falsch-negative Ergebnisse einzuschließen. Weitere mathematische Verfahren, um eine individuelle Risiko durch die Verwendung mehrerer der beschriebenen chromosomalen Regionen, bzw. SNPs zu berechnen sind beispielsweise der NRI (Net Reclassification Index) oder der IDI (Integrated Discrimination Index). Die Beurteilung der prädiktiven Fähigkeit eines neuen Marker, bzw. eines Markersets erfolgt auch über die Fläche unter der ROC-Kurve. Verfahren zur statistischen Bestimmung sind auch in Pencina MJ, D'Agostino RB, Vasan RS. Statistical methods for assessment of added usefulness of new biomarkers. Clin Chem Lab Med. 2010 Dec; 48(12):1703-1711 offenbart.

Die Sensitivität und Spezifität eines diagnostischen Tests hängt von mehr als nur die analytische "Qualität" des Tests, sondern auch auf die Definition dessen, was als abnormes Ergebnis angesehen wird. Wie oben ausgeführt, kann es nötig sein mehr als einen erfindungsgemäßen Genotyp zu bestimmen, d.h. den Genotyp von 2 oder mehr der erfindungsgemäßen Regionen oder bspw. SNP zu bestimmen. In der Praxis werden ROC-Kurven verwendet, typischerweise werden diese ROC-Kurven durch Auftragen des Werts einer Variablen (z.B. Anzahl der Abweichungen im Genotyp) in ihrer relativen Häufigkeit der als "normal" eingestuften (d.h. scheinbar gesunden) und als "krank" eingestuften Population (d.h. Patienten, die Legasthenie haben) berechnet. Abhängig von der jeweiligen Diagnose-Frage die es zu beantworten gilt, muss die Referenz-Gruppe nicht unbedingt "Normal", das heißt völlig gesund, sondern vielmehr kann hier jede erfindungsgemäße Kontrollgruppe in Frage kommen. So auch zum Beispiel Patienten, die einen niedrigen IQ und/oder ADS und/oder ADHS haben. Für einen bestimmten Marker, kann es vorkommen, dass eine Abweichung vom "nicht-risikoassoziierten"-Genotyp bei Personen mit und ohne Legasthenie vorliegt, wobei die Wahrscheinlichkeit für das Vorliegen der Legasthenie signifikant erhöht ist. Unter solchen Bedingungen wird der Test nicht unbedingt mit 100% Genauigkeit "normal" von "krank" unterscheiden. Daher kann es nötig sein einen "Schwellenwert" für die Anzahl an Abweichungen im Genotyp festzulegen, oberhalb dessen der Test als abnormal, d.h. "krank" und unterhalb dessen der Test als normal, d.h. "gesund" eingestuft wird. Die Fläche unter der ROC-Kurve ist ein Maß für die Wahrscheinlichkeit, dass die erhaltende Anzahl die korrekte Einteilung in "normal" und "krank" ermöglicht. ROC-Kurven können verwendet werden, wenn die Testergebnisse für sich genommen nicht zu einem eindeutigen Ergebnis führen. Solange man die Ergebnisse einstufen (engl. "ranken") kann, kann man eine ROC-Kurve erstellen. Zum Beispiel könnten die Ergebnisse einer Diagnose auf "Krankheit" weiter graduell (zB 1 = gering, 2 = normal, 3 = hoch) eingestuft werden. Dieses Ranking kann mit der "normalen" Bevölkerung korreliert werden, und eine ROC-Kurve erstellt werden. Diese Verfahren sind dem Fachmann bekannt (siehe z. B. Hanley et al. (1982), Radiology 143: 29-36).

Die horizontale Achse der ROC-Kurve stellt (1-Spezifität) dar. Dieser Wert ist umso größer, je höher die Rate der falsch-positiven Ergebnisse ist. Die vertikale Achse der Kurve bildet die Empfindlichkeit (Sensitivität) ab. Dieser Wert ist umso größer, je höher die Trefferwahrscheinlichkeit für korrekt-positive Ergebnisse. So können für einen bestimmten Schwellenwert für die Anzahl an Abweichungen, der Wert von (1-Spezifität) und eine entsprechende Sensitivität bestimmt werden. Die Fläche unter der ROC-Kurve ist ein Maß für die Wahrscheinlichkeit, dass die Anzahl der Abweichungen geeignet ist die Diagnose eines Legasthenierisikos zu ermöglichen. Somit kann die Fläche unter der ROC-Kurve verwendet werden, um die Wirksamkeit der Diagnose zu bestimmen. Vorzugsweise wird ein Schwellenwert ausgewählt, um eine ROC-Kurve mit einer Fläche unter der Kurve von signifikant größer als 0.5, mehr bevorzugt größer als 0.7 bereitzustellen, besonders bevorzugt größer als 0,8..

Auch können Odds Ratio (OR) oder Chancenverhältnis als Risikomaß verwendet werden. Dies stellt ein Assoziationsmaß dar, welches die Chance zweier Individuen oder Populationen vergleicht, dass ein Ereignis z. B. eine Krankheit wie Legasthenie, eintritt. Das OR spielt besonders in Assoziationsstudien im Fall-Kontroll-Design eine Rolle. Das Odds Ratio gibt an, um wieviel höher für Merkmalsträger die Chance ist, zu erkranken, als für Personen ohne diesen Risikofaktor. Das OR wird berechnet, indem die Anzahl der erkrankten Individuen einer Population, die Risikoträger sind, mit der Anzahl der erkrankten Individuen einer Population, die keine Risikoträger sind, multipliziert wird. Das erhaltene Ergebnis wird dividiert durch die Anzahl der gesunden Individuen einer Population, die Risikoträger sind, multipliziert mit der Anzahl der gesunden Individuen einer Population, die keine Risikoträger sind. Ein Odds Ratio von eins bedeutet, dass die Chancen zu erkranken für beide Populationen gleich sind. Anders als das relative Risiko bezieht sich das Odds Ratio auf Chancen (Risiken) und nicht auf Wahrscheinlichkeiten (retrospektiv). ORs sind dem Fachmann hinlänglich bekannte (siehe z.B. Ziegler, Andreas. A statistical Approach to Genetic Epidemiology. WILEY-VCH, 2006).
In einer weiteren bevorzugten Ausführungsform der vorliegenden Erfindung wird Legasthenie einem Menschen mit einer Wahrscheinlichkeit zugewiesen, die von der Anzahl der Unterschiede in den analysierten Regionen abhängig ist, d.h. je höher die Anzahl der Unterschiede, desto höher die Wahrscheinlichkeit von Legasthenie bei besagtem Menschen.

Die Erfinder haben überraschenderweise festgestellt, dass Abweichungen des Genotyps einer der erfindungsgemäßen chromosomalen Regionen prädiktiv für die Diagnose eines Legasthenierisikos sind. Unter Umständen kann es jedoch wünschenswert sein, den prädiktiven Wert, d.h. die Sensitivität weiter zu erhöhen. Hierzu ist es Vorteilhaft, wenn die Zuschreibung von Legasthenie dann erfolgt, wenn Abweichungen im Genotyp zur Kontrolle in mindestens zwei oder mehr der erfindungsgemäßen chromosomalen Regionen vorliegen. In einer Ausführungsform des erfindungsgemäßen Verfahrens erfolgt das Zuschreiben einer Präsenz eines Legasthenierisikos in der zu diagnostizierenden Person bei Vorliegen von je einer Abweichung des bestimmten Genotyps vom Genotyp besagter Kontrolle in mindestens zwei der besagten Regionen. In einer weiteren Ausführungsform des erfindungsgemäßen Verfahrens erfolgt das Zuschreiben einer Präsenz eines Legasthenierisikos in der zu diagnostizierenden Person bei Vorliegen von je einer Abweichung des bestimmten Genotyps vom Genotyp besagter Kontrolle in allen vier der besagten Regionen. In einer Ausführungsform erfolgt das Zuschreiben einer Präsenz eines Legasthenierisikos in der zu diagnostizierenden Person bei Vorliegen von je einer Abweichung des bestimmten Genotyps vom Genotyp besagter Kontrolle in der Region von Nukleotid 12091000 bis 12200000 des Chromosoms 1 der Region von Nukleotid 89066000 bis 89088000 des Chromosoms 15 oder in der Region von Nukleotid 12091000 bis 12200000 des Chromosoms 1 und in der Region von Nukleotid 41930000 bis 42032000 des Chromosom 2 oder in der Region von Nukleotid 12091000 bis 12200000 des Chromosoms 1 und in der Region von Nukleotid 51508000 bis 51885000 des Chromosom 2. In einer Ausführungsform des erfindungsgemäßen Verfahrens erfolgt das Zuschreiben einer Präsenz eines Legasthenierisikos in der zu diagnostizierenden Person bei Vorliegen von je einer Abweichung des bestimmten Genotyps vom Genotyp besagter Kontrolle in mindestens zwei SNP, ausgewählt aus der Gruppe bestehend aus rs7162960, rs496888, rs2715079 und rs2374341, vorzugsweise bei Vorliegen von je einer Abweichung in rs7162960 und rs496888 oder in rs7162960 und rs2715079 oder in rs7162960 und rs2374341 oder in rs496888 und rs2715079 oder in rs496888 und rs2374341 oder in rs2715079 und rs237434. Wie oben ausgeführt, haben die Erfinder festgestellt, dass für die genannten SNPs das Auftreten eines bestimmten oben erwähnten genetischen Zustandes prädiktiv für das Vorliegen von Legasthenie ist. Daher ist es eine Ausführungsform der vorliegenden Erfindung, dass das Zuschreiben einer Präsenz von Legasthenie in der zu diagnostizierenden Person bei Vorliegen je einer Abweichung von den folgenden Nukleotiden in mindestens zwei SNP erfolgt, ausgewählt aus der Gruppe bestehend aus G für rs496888, T für rs7162960, T für rs2715079 und C für rs2374341, vorzugsweise bei Vorliegen von je einer Abweichung von den Nukleotiden G für rs496888 und T für rs7162960 oder G für rs496888 und T für rs2715079 oder G für rs496888 und C für rs2374341 oder T für rs7162960 und T für rs2715079 oder T für rs7162960 und C für rs2374341 oder T für rs2715079 und C für rs2374341. In einer Ausführungsform des erfindungsgemäßen Verfahrens erfolgt das Zuschreiben einer Präsenz eines Legasthenierisikos in der zu diagnostizierenden Person bei Vorliegen folgender Nukleotide in mindestens zwei SNP, ausgewählt aus der Gruppe bestehend aus A für rs496888, C für rs7162960, C für rs2715079 und T für rs2374341, vorzugsweise bei Vorliegen von je den Nukleotiden A für rs496888 und C für rs7162960 oder A für rs496888 und C für rs2715079 oder A für rs496888 und T für rs2374341 oder C für rs7162960 und T für rs2715079 oder C für rs7162960 und T für rs2374341 oder C für rs2715079 und T für rs2374341.

Es kann auch zur weiteren Erhöhung der Sensitivität und/oder Spezifität wünschenswert sein den Genotyp von mindestens drei der bevorzugten SNP zu Bestimmen. In einer Ausführungsform erfolgt daher das Zuschreiben einer Präsenz eines Legasthenierisikos in der zu diagnostizierenden Person bei Vorliegen von je einer Abweichung des bestimmten Genotyps vom Genotyp besagter Kontrolle in mindestens drei SNP, ausgewählt aus der Gruppe bestehend aus rs496888, rs7162960, rs2715079 und rs2374341, vorzugsweise bei Vorliegen von je einer Abweichung in rs496888 und rs7162960 und rs2715079 oder in rs496888 und rs7162960 und rs2374341 oder in rs496888 und rs2715079 und rs2374341 oder in rs7162960 und rs2715079 und rs2374341, vorzugsweise bei Vorliegen von je einer Abweichung in rs7162960, rs496888, rs2715079 und rs2374341. Wie oben ausgeführt, haben die Erfinder festgestellt, dass für die genannten SNPs Abweichungen vom Risikozustand (vgl. Tabelle 2.1.) prädiktiv für das Vorliegen von Legasthenie sind. Daher ist es eine Ausführungsform der vorliegenden Erfindung, dass das Zuschreiben einer Präsenz eines Legasthenierisikos in der zu diagnostizierenden Person bei Vorliegen je einer Abweichung von den folgenden Nukleotiden in mindestens drei SNP erfolgt, ausgewählt aus der Gruppe bestehend aus G für rs496888, T für rs7162960, T für rs2715079 und C für rs2374341, vorzugsweise bei Vorliegen von je einer Abweichung von den Nukleotiden G für rs496888 und T für rs7162960 und T für rs2715079 oder G für rs496888 und T für rs7162960 und C für rs2374341 oder G für rs496888 und T für rs2715079 und C für rs2374341 oder T für rs7162960 und T für rs2715079 und C für rs2374341. In einer weiteren Ausführungsform des erfindungsgemäßen Verfahrens erfolgt die Zuschreibung der Präsenz eines Legasthenierisikos in der zu diagnostizierenden Person bei Vorliegen von je einer Abweichung von den Nukleotiden G für rs496888 und T für rs7162960 und T für rs2715079 und C für rs2374341. In einer Ausführungsform der vorliegenden Erfindung, erfolgt das Zuschreiben einer Präsenz eines Legasthenierisikos in der zu diagnostizierenden Person bei Vorliegen folgender Nukleotide in mindestens drei SNP, ausgewählt aus der Gruppe bestehend aus A für rs496888, C für rs7162960, C für rs2715079 und T für rs2374341, vorzugsweise bei Vorliegen von je den Nukleotiden A für rs496888 und C für rs7162960 und C für rs2715079 oder A für rs496888 und C für rs7162960 und T für rs2374341 oder A für rs496888 und C für rs2715079 und T für rs2374341oder C für rs7162960 und C für rs2715079und T für rs2374341. In einer weiteren Ausführungsform des erfindungsgemäßen Verfahrens erfolgt die Zuschreibung der Präsenz eines Legasthenierisikos in der zu diagnostizierenden Person bei Vorliegen von je einer Abweichung von den Nukleotiden A für rs496888 und C für rs7162960 und C für rs2715079 und T für rs2374341.

In einer Ausführungsform wird die Nukleinsäure vor der Bestimmung des Genotyps amplifiziert und/oder aufgereinigt. Vor Bestimmung des Genotyps bedeutet, dass dies vor der Ermittlung der Sequenz oder Teilen davon geschieht. Dies bedeutet, dass dies in Schritt b) des erfindungsgemäßen Verfahrens durchgeführt werden kann. Daher umfasst das erfindungsgemäße Verfahren in einer Ausführungsform in Schritt b) die Aufreinigung und/oder Amplifizierung der Nukleinsäure in besagter Probe, bevorzugt die Amplifizierung besagter Regionen oder Teilen davon.

Im Wesentlichen kommt es bei der Bestimmung des Genotyps auf die Bestimmung der Sequenz an. Sequenz in diesem Zusammenhang kann sich auf einen längeren Nukleinsäureabschnitt beziehen, das heißt auf die Abfolge bestimmter Nukleotide. In einer Ausführungsform der vorliegenden Erfindung betrifft die Bestimmung des Genotyps die Verwendung bekannter Verfahren, die geeignet sind die Sequenz von Nukleinsäuren zu bestimmen. Dies inkludiert auch massenspektroskopische Verfahren. In einer Ausführungsform des erfindungsgemäßen Verfahrens erfolgt die Bestimmung des Genotyps unter Verwendung einer Technik, ausgewählt aus der Gruppe bestehend aus Nukleinsäuren-Sequenzierung, Mikroarray, Real-time PCR, Restriktionslängenpolymorphismus (RLFP) und Massenspektrometrie.

Wie hierin ausgeführt, eignet sich das erfindungsgemäße Verfahren überraschenderweise besonders gut zur Diagnose eines Legasthenierisikos bevor die zu diagnostizierende Person erkennbare Sprachentwicklung zeigt, z.B. im Säuglingsalter oder gar als Fetus oder Embryo. Insbesondere auch bevor die zu diagnostizierende Person das Schulalter erreicht hat.
Daher kann besagter zu diagnostizierender Mensch ein Alter von 0 bis 8, vorzugsweise von 0 bis 7, weiter bevorzugt von 0 bis 6 haben.
Wobei hier vorzugsweise auf die Vollendung eines Lebensjahres abgestellt wird.

Offenbart ist ebenfalls ein Kit zur Durchführung des erfindungsgemäßen Verfahrens. Der Fachmann wird erkennen, dass das Kit dermaßen ausgestaltet sein muss, dass die Bestimmung des jeweiligen Genotyps gewährleistet ist. Daher kann das Kit Mittel zur Bestimmung des Genotyps für mindestens eine chromosomale Region umfassen,
wobei besagte Region ausgewählt ist aus der Region von Nukleotid 12091000 bis 12200000 des Chromosoms 1, der Region von Nukleotid 89066000 bis 89088000 des Chromosoms 15, der Region von Nukleotid 41930000 bis 42032000 des Chromosom 2 und der Region von Nukleotid 51508000 bis 51885000 des Chromosom 2. Diese Mittel können ein oder mehrere Polynukleotidsonden umfassen, wobei besagte Polynukleotidsonden jeweils spezifisch an SNPs hybridisieren, ausgewählt aus der Gruppe bestehend aus rs496888, rs7162960, rs2715079 und rs237434.
Das beschriebene Kit kann eine oder mehrere beschriebene Polynukleotidsonden, wie hierin beschrieben enthalten. Es ist beschrieben, dass das
Kit einen oder mehrere Polynukleotidprimer zur Amplifizierung besagter Region enthält, vorzugsweise zur Amplifizierung eines Teils besagter Region, wobei besagter Teil mindestens einen der bevorzugten SNP umfasst, ausgewählt aus der Gruppe bestehend aus rs496888, rs7162960, rs2715079 und rs237434. Es ist auch beschrieben, dass das Kit einen oder mehrere Polynukleotidprimer umfasst, wie hierin beschrieben. Es ist beschrieben, dass das Kit eine oder mehrere Polynukleotidsonden und einen oder mehrere Polynukleotidprimer umfasst, vorzugsweise mindestens zwei Polynukleotidprimer. Der Fachmann wird erkennen, dass die Polynukleotidprimer und die Polynukleotidsonden vorzugsweise aufeinander abgestimmt sind, d.h. dass, wenn eine Polynukleotidsonde umfasst ist, die beispielsweise spezifisch an rs496888 hybridisiert, die ebenfalls enthaltenden Polynukleotidprimer geeignet sind einen Bereich zu amplifizieren, der rs496888 umfasst. Dies gilt, *mutatis mutandis,* auch für die übrigen SNPs oder Regionen. Eine Ausführungsform des offenbarten Kits betrifft eine Mikroarray Vorrichtung. Diese umfasst bevorzugt: a) eine Kammer zur Aufnahme einer Nukleinsäure beinhaltenden Flüssigprobe; b) ein oder mehrere verschiedene Polynukleotidsonden, die auf einer inneren Oberflächen der Kammer immobilisiert sind, wobei besagte Polynukleotidsonden jeweils spezifisch ein oder mehrere SNP detektieren, ausgewählt aus der Gruppe bestehend aus rs496888, rs7162960, rs2715079 und rs2374341. Der Fachmann ist in der Lage die Länge und Sequenz der Polynukleotidsonden zu wählen. Insbesondere aus dem hierin Beschriebenen für die Polynukleotidsonden.

Die Polynukleotidsonden des Kits können eine Länge von 10 bis 45 Nukleotiden aufweisen, vorzugsweise weisen sie eine Länge von 20 bis 35 Nukleotiden auf, weiter vorzugsweise eine Länge von 25 bis 30 Nukleotiden. Aufweisen einer Länge wird hier als bestehend aus der genannten Anzahl an Nukleotiden aufgefasst. Es ist beschrieben, dass die Polynukleotidsonden des Kits eine Sequenz ausgewählt aus der Gruppe bestehend aus SEQ ID NO. 11, SEQ ID NO. 12, SEQ ID NO. 15, SEQ ID NO. 16, SEQ ID NO. 19 und SEQ ID NO. 23 umfassen.

In einer bevorzugten Ausführungsform von besagtem Aspekt der Erfindung bezieht sich das Verfahren auf die Bereitstellung einer Probe von besagtem Menschen außerhalb des menschlichen Körpers.

In einer weiteren bevorzugten Ausführungsform der vorliegenden Erfindung hinsichtlich des Aspekts der Erfindung hinsichtlich der Bestimmung von SNPs wird Legasthenie einem Menschen mit einer Wahrscheinlichkeit zugewiesen, die von der Anzahl der Abweichungen zwischen den bestimmten Genotypen und den Genotypen der Kontrollgruppe abhängig ist, d.h. je höher die Anzahl der Abweichungen, desto höher die Wahrscheinlichkeit von Legasthenie bei besagtem Menschen. Dem Fachmann wird ersichtlich sein, dass die Diagnose oder Präsenz "eines Legasthenierisikos" der Diagnose bzw. Präsenz eines "erhöhten Legasthenierisikos" entspricht und die Begriffe synonym verwendet werden. Die Erhöhung bezieht sich auf das durchschnittliche Risiko einer Population.

Der zu diagnostizierende Mensch kann ein kaukasischer Mensch sein. Besagter Kaukasier gehört in einer bevorzugten Ausführungsform zu einer Indo-Germanisch sprechenden Population; weiter bevorzugt gehört dieser Kaukasier zu einer West-Germanisch sprechenden, Hochdeutsch oder Deutsch sprechenden Population. Der Fachmann wird verstehen, dass ein Mensch auch dann als zu einer entsprechenden Population zugehörig anzusehen ist, wenn er selbst noch nicht sprechen kann. Vielmehr ist dies dahingehend zu verstehen, dass zu erwarten ist, dass dieser Mensch in einer Population aufwächst, in der diese Sprachen die vorwiegend benutzte Kommunikationssprache ist. Dies ergibt sich auch aus dem Umstand, dass das erfindungsgemäße Verfahren unerwarteterweise eben ein Diagnoseverfahren für Legasthenie bereitstellt, mit der die entsprechende Diagnose schon vor Erlernen der Sprache erfolgen kann.

In einer bevorzugten Ausführungsform in der vorliegenden Erfindung ist die Nukleinsäure, die in der bereitgestellten Probe vorhanden ist, eine Nukleinsäure ausgewählt aus der Gruppe bestehend aus RNS, mRNS, DNS, genomischer DNS und cDNS. In einer bevorzugten Ausführungsform ist die Nukleinsäure genomische DNS. In einer weiteren Ausführungsform wird der Genotyp anhand der exprimierten Nukleinsäuren bestimmt, d.h. beispielsweise mRNS. Daher ist in einer Ausführungsform die Nukleinsäure eine mRNS oder cDNS.

Der Fachmann wird erkennen, dass es notwendig ist, dass die Nukleinsäure zumindest die genomische Region, bzw. zumindest den Bereich um den SNP enthalten muss, deren Genotyp in Verfahren bestimmt werden soll oder mit ihr korreliert. Der Fachmann kann sich verschiedener Techniken bedienen, um dies zu gewährleisten. Beispielsweise kann er die genomische Region, bzw. den Bereich um das jeweilige SNP, deren Genotyp zu bestimmen ist, mittels bekannter Verfahren amplifizieren. In einer Ausführungsform umfasst das Verfahren daher in oder vor Schritt b) die Amplifikation besagter genomischer Region. In einer weiteren Ausführungsform, in der ein oder mehrere SNPs bestimmt werden, umfasst das erfindungsgemäße Verfahren die Amplifikation eines Bereichs um den SNP dessen Genotyp zu bestimmen ist. Dieser Bereich um den SNP besteht in einer Ausführungsform aus 20 bis 300 Nukleotiden, in einer weitere Ausführungsform aus 20 bis 50 Nukleotiden. Der Fachmann wird erkennen, dass es bevorzugt sein kann, dass der SNP, dessen Genotyp zu bestimmen ist einen gewissen Abstand zu den Enden des amplifzierten Bereichs aufweisen soll. Die beiden Enden des Amplifikationsprodukts haben deshalb in einer Ausführungsform einen Abstand von mindestens 150Nukleotiden zum zu bestimmenden SNP, vorzugsweise einen Abstand von mindestens 20 Nukleotiden. Ein hierzu geeignetes Verfahren ist beispielsweise die Polymerase Kettenreaktion (engl. Polymerase chain reaction (PCR). Es können auch andere Amplifikationsverfahren zur Anwendung kommen, diese können ausgewählt sein aus der Gruppe bestehend aus "Rolling Circle Amplification" (wie in LIU, et al., "Rolling circle DNA synthesis: Small circular oligonucleotides as efficient templates for DNA polymerases," J. Am. Chem. SOC. 118:1587-1594 (1996) beschrieben), die isothermale Amplifikation (wie in Walker, et al., "Strand displacement amplification--an isothermal, in vitro DNA amplification technique," Nucleic Acids Res. 20(7):1691-1696 (1992), und Ligase Ketten Reaktion (wie in Landegren, et al., "A Ligase-Mediated Gene Detection Technique," Science 241:1077-1080 (1988) oder in Wiedmann, et al., "Ligase Chain Reaction (LCR)-Overview and Applications," PCR Methods and Applications (Cold Spring Harbor Laboratory Press, Cold Spring Harbor Laboratory, NY (1994) pp. S51-S64 beschrieben). In einer bevorzugten Ausführungsform des Verfahrens der vorliegenden Erfindung wird die in der Probe des Menschen enthaltene Nukleinsäure zuerst gereinigt und dann vorzugsweise amplifiziert, und zwar außerhalb des menschlichen Körpers.

Für ein Verfahren der vorliegenden Erfindung ist es weiterhin bevorzugt, die Analyse so auszuführen, dass sie unter Nutzung von mindestens einer Technik durchgeführt wird, ausgewählt aus der Gruppe an Techniken bestehend aus Nukleinsäuresequenzierung (Pettersson, E., Lundeberg, J., Ahmadian, A. (2009). Generations of sequencing technologies. Genomics, 93(2), 105-111), Mikroarray (Müller, H.-J. & Röder, T. (2004). Der Experimentator: Microarrays. Spektrum Akademischer Verlag, Heidelberg), Real-time PCR (Navarro, E., Serrano-Heras, G., Castano, M.J., Solera, J. (2015). Real-time PCR detection chemistry. Clinica chimica acta, 439, 231-250), Restriktionslängenpolymorphismusanalyse (Feuilhade de Chauvin, M. (2005). New diagnostic techniques. Journal of the European Academy of Dermatology and Venereology, 19, Suppl 1, 20-24) und Massenspektrometrie (Wenzel, T., Elssner, T., Fahr, K., Bimmler, J., Richter, S., Thomas, I., Kostrzewa, M. (2003). Genosnip: SNP genotyping by MALDI-TOF MS using photocleavable oligonucleotides. Nucleosides, nucleotides & nucleic acids, 22 (5-8), 1579-1581)]. Jedoch kann jedes andere Verfahren, das für die Analyse von Nukleinsäure und die Bestimmung eines Genotyps geeignet ist, genutzt werden.

Real-time PCR Verfahren können auch verwendet werden, um die erfindungsgemäßen Regionen und SNP zu analysieren. Allgemein gesagt basiert Real-time PCR auf dem Einbau von Doppelstrang-spezifischen Farbstoffen in die DNS, während besagte DNS amplifiziert wird. Besagte Farbstoffe werden nur in dem Falle, dass sie eingebaut sind, detektiert. Folglich ist das Detektionssignal des entsprechenden Farbstoffes umso höher, je mehr DNS amplifiziert wird. Durch entsprechende Gestaltung der Primer und/oder durch Hinzufügung von geeigneten Polynukleotidsonden, die nur mit einer spezifischen DNS-Sequenz hybridisieren (und folglich in der Lage sind, zwischen SNPs zu unterscheiden) und die Verwendung von spezifischen Hybridisierungsbedingungen können DNS-Regionen und bevorzugt Polymorphismen analysiert werden.

Die Bestimmung des Genotyps kann mittels der beschriebenen Polynukleotidprimer und/oder der beschriebenen Polynukleotidsonden ausgeführt werden.
Hierbei gibt es prinzipiell verschiedene Verfahren aus denen sich der Fachmann bedienen kann. Bei der Verwendung der beschriebenen Polynukleotidsonden kann die Bestimmung des Genotyps, z.B. eines SNPs, mittels Schmelzkurvenanalyse erfolgen. Bei einer Schmelzkurvenanalyse wird der Doppelstrang bestehend aus zu analysierender Nukleinsäure und Polynukleotidsonde aufgeschmolzen, indem die Temperatur langsam kontinuierlich erhöht wird (z.B. von 50 °C auf 95 °C). Bei einer für das Fragment spezifischen Schmelztemperatur denaturiert der Doppelstrang zu zwei einzelsträngigen Molekülen. Die verschiedenen bekannten Verfahren setzen auf eine Veränderung der Fluoreszenz nach Auftrennung des Doppelstrangs. Dies kann zum Beispiel mittels interkalierendem Farbstoff geschehen. Dabei wird ein Fluoreszenzfarbstoff (z. B. SYBR Green I) bei Auftrennung des Doppelstrangs freigesetzt und eine Änderung der Fluoreszenz registriert. Eine andere Möglichkeit besteht darin, den Fluorescence resonance energy transfer (FRET) auszunutzen. Ein Donor-Fluorochrom, das durch eine Lichtquelle angeregt wird, gibt einen Teil seiner Energie an ein in ausreichender Nähe befindliches Akzeptor-Fluorochrom ab. Nimmt der Abstand zwischen Akzeptor und Donor zu, so nimmt FRET und somit das Fluoreszenzsignal des Akzeptors ab, während das des Donors zunimmt. Diese Methode ist zwar aufwendig und teuer, bietet aber die Vorteile der hohen Spezifität des Assays. Dem Fachmann sind Modifikationen von Sonden bekannt, um FRET zu nutzen. Beispielsweise umfassen diese LightCycler Sonden, bei denen zwei Sonden eingesetzt werden, die in unmittelbarer Nähe an die Zielnukleinsäure binden. Die beiden Sonden sind jeweils mit einem FRET-Donor bzw. FRET-Akzeptor (hier Reporter) markierte Oligonukleotide, die nebeneinander an die Ziel-Sequenz binden und damit die Fluorochrome in eine für den FRET ausreichende Nähe bringen. Eine weitere Möglichkeit der Echtzeit-Quantifizierung von PCR-Produkten unter Ausnutzung des FRET bietet die Nutzung von sogenannten "Molecular Beacons" als Sonden. Molecular Beacons sind Polynukleotidsonden, die sowohl mit einem Reporter-Fluorophor als auch mit einem Quencher gekoppelt sind. Die Nucleotide am 5'-Ende der Sonde sind zu denen am 3'-Ende komplementär, so dass sich eine für Molecular Beacons charakteristische Sekundärstruktur ausbilden kann. In diesem als stem loop (Haarnadelstruktur) bezeichneten Zustand zeigt der Reporter durch seinen geringen Abstand zum Quencher keine Fluoreszenz. Durch Anlagerung der Schleifen-Region an eine komplementäre DNS-Sequenz während eines PCR-Zyklus wird der Abstand zwischen Quencher und Reporter vergrößert. Eine Reporter-Fluoreszenz kann somit beobachtet werden. Die Sonde ist weiter so konstruiert, dass sie an eine Zielsequenz innerhalb der zu analysierenden Nukleinsäure bindet, z.B. die Sequenz, die den SNP umfasst, spezifisch hybridisiert (Abravaya K, Huff J, Marshall R, Merchant B, Mullen C, Schneider G, Robinson J (April 2003). "Molecular beacons as diagnostic tools: technology and applications". Clin. Chem. Lab. Med. 41 (4): 468-74). Weitere bekannte Verfahren sind TaqMan basierte (McGuigan FE, Ralston SH (September 2002). "Single nucleotide polymorphism detection: allelic discrimination using TaqMan". Psychiatr. Genet. 12 (3): 133-136; Syvänen AC (December 2001). "Accessing genetic variation: genotyping single nucleotide polymorphisms". Nat. Rev. Genet. 2 (12): 930-42), Oligonucleotide Ligation Assay (Macdonald SJ. Genotyping by Oligonucleotide Ligation Assay (OLA). CSH Protoc. 2007 Sep 1; 2007:pdb.prot4843), Temperature gradient gel electrophoresis (TGGE) oder temperature gradient capillary electrophoresis (TGCE) (Harbron S, Rapley R (2004). Molecular analysis and genome discovery. London: John Wiley & Sons Ltd. ISBN 0-471-49919-6), Denaturing high performance liquid chromatography (DHPLC) (Oefner PJ, Underhill PA (1995). "Comparative DNA sequencing by denaturing high-performance liquid chromatography (DHPLC)". Am J Hum Genet 57: 103-10), Detektion mittels Mismatch bindender Proteine (Drabovich AP, Krylov SN (March 2006). "Identification of base pairs in single-nucleotide polymorphisms by MutS protein-mediated capillary electrophoresis". Anal. Chem. 78 (6): 2035-8), Amplicon Schmelzpunktanalyse (Gundry CN, Vandersteen JG, Reed GH, Pryor RJ, Chen J, Wittwer CT (March 2003). "Amplicon melting analysis with labeled primers: a closed-tube method for differentiating homozygotes and heterozygotes". Clin. Chem. 49 (3): 396-406), Harbron S, Rapley R (2004). Molecular analysis and genome discovery. London: John Wiley & Sons Ltd. ISBN 0-471-49919-6), SNP microarrays (Affimetrix 2007; Harbron S, Rapley R (2004). Molecular analysis and genome discovery. London: John Wiley & Sons Ltd. ISBN 0-471-49919-6), Flap endonuclease (FEN) oder auch Invader Assay (Olivier M (June 2005). "The Invader assay for SNP genotyping". Mutat. Res. 573 (1-2): 103-10) oder unter Verwendung des SNPlex™ (Applied Biosystems). Diese Verfahren können auch dazu verwendet warden, festzustellen, ob ein bestimmter Genotyp homozygot oder heterozygote im zu untersuchenden Individuum vorliegt (siehe bspw. Gundry CN, Vandersteen JG, Reed GH, Pryor RJ, Chen J, Wittwer CT (March 2003). "Amplicon melting analysis with labeled primers: a closed-tube method for differentiating homozygotes and heterozygotes". Clin. Chem. 49 (3): 396-406; oder unter Verwednung zweier Beacon-Sonden nach Abravaya K, Huff J, Marshall R, Merchant B, Mullen C, Schneider G, Robinson J (April 2003). "Molecular beacons as diagnostic tools: technology and applications". Clin. Chem. Lab. Med. 41 (4): 468-74; oder Harbron S, Rapley R (2004). Molecular analysis and genome discovery. London: John Wiley & Sons Ltd. ISBN 0-471-49919-6). Die aufgeführten Verfahren stellen jedoch keine abschließende Auflistung möglicher Verfahren zur Bestimmung des Genotyps dar und dienen dem Fachmann lediglich als Anhaltspunkt.

Offenbart ist auch die Bestimmung des Genotyps unter Zuhilfenahme von Massenspektrometrie. Auch Massenspektrometrie (MS) kann verwendet werden, um die die Nukleinsäure, bzw. DNS umfassenden Proben zu analysieren. In MALDI-MS wird eine Probe mit einer Matrixmaterial enthaltenden Lösung gemischt, und ein Tropfen der Flüssigkeit wird auf der Oberfläche einer Metallplatte platziert. Die Matrixlösung co-kristallisiert dann z.B. mit der biologischen Probe, und die Metallplatte wird in das Massenspektrometer eingeführt, und dann wird Laserenergie auf die Metallplattenoberfläche gerichtet, wo sie durch die biologischen Moleküle absorbiert wird und die biologischen Moleküle ionisiert, ohne sie signifikant zu fragmentieren. In anderen Ausführungsformen der MALDI-MS kann die Matrix zuerst als dünner Film kristallisiert werden, wobei die biologische Probe später hinzugefügt wird, oder umgekehrt.

Im Kontext der vorliegenden Erfindung kann es bevorzugt sein, für die Detektion keine klassische MALDI-MS Analyse durchzuführen, sondern sich auf Weiterentwicklungen der MALDI-MS zu stützen. Diese Weiterentwicklungen, welche z.B. "surface enhanced Laser Desorptions/Ionisations Massenspektrometrie (SELDI-MS)" einschließen, kombinieren das oben erwähnte Prinzip der Probenvorbereitung und Prä-Fraktionierung mit den Prinzipien der MALDI-MS. In SELDI-MS kann die Probenoberfläche z.B. so modifiziert werden, dass sie aktiv am Probenvorbereitungsprozess beteiligt ist. In einer Variante kann diese Probenoberfläche daher mit Affinitätsmatrizes derivatisiert werden, die eine Ionenaustauschcharakteristik liefern, um die Präfraktionierung der biologischen Probe zu erlauben.

Weitere Weiterentwicklungen der MALDI/SELDI Technologie sind im Fachgebiet als surface-enhanced affinity capture (SEAC), surface-enhanced need disorption (SEND) oder surface-enhanced photo label attachment and release (SEPAR) bekannt. Ein Fachmann wird all diese Massenspektrometrieansätze für die Analyse der DNS bei einer von einem Individuum gewonnenen Probe gemäß vorliegender Erfindung in Erwägung ziehen.
Für die Durchführung der Bestimmung des Genotyps können Polynukleotidsonden und massenspektrometrische Verfahren verwendet werden. Hierzu kann es notwendig sein, dass die Polynukleotidsonden Modifikationen vorweisen. Solche Verfahren sind dem Fachmann bekannt (Wenzel, T., Elssner, T., Fahr, K., Bimmler, J., Richter, S., Thomas, I., Kostrzewa, M. (2003). Genosnip: SNP genotyping by MALDI-TOF MS using photocleavable oligonucleotides. Nucleosides, nucleotides & nucleic acids, 22 (5-8), 1579-1581). Daher umfassen die beschriebenen Polynukleotidsonden in einer Ausführungsform eine Modifikation wie Biotin. In einer Ausführungsform ist besagte Modifikation am 5'-Ende der Polynukleotidsonde angebracht. Weiter kann die beschriebene Polynukleotidsonde eine Spaltstelle umfassen. Solche Spaltstellen sind Modifikationen des Phosphat-(Deoxy)Ribose Rückrads. So kann beispielsweise zwischen zwei Zuckerresten besagten Rückgrats das Nukleotid durch ein spaltbares anderes Molekül ersetzt sein. Vorzugsweise ist diese Gruppe durch Bestrahlung mit einer elektromagnetischen Strahlung bestimmter Wellenlänge oder eines bestimmten Wellenlängenbereichs spaltbar. Solche Stellen werden als Photospaltstelle bezeichnet. Geeignete Reste hierfür sind dem Fachmann bekannt (Niemeyer, D., Elssner, T., Fahr, K., Peters, D., Wenzel, T., Petkovski, E., Kostrzewa (2004). Detection of genetic variation by MALDI-TOF mass spectrometry: rapid SNP genotyping using the GENOLINK system. International Congress Series, 1291, 9-11).

Daher kann die beschriebene Polynukleotidsonde eine Photospaltstelle umfassen, die sich vorzugsweise zwischen zwei Nukleotiden befindet, die 8 bis 15 Nukleotide vom 3' Ende entfernt liegen. Dem Fachmann erschließt sich, dass die Photospaltstelle zwischen zwei Nukleotiden vorliegen muss, d.h. innerhalb der Polynukleotidsonde. Die Position innerhalb der Sonde kann vom Fachmann gewählt werden. Die Polynukleotidsonde kann einen Biotinrest am 5'-Ende und eine Photospaltstelle haben, besonders bevorzugt hat die Polynukleotidsonde eine Sequenz gemäß SEQ ID NO. 12 oder SEQ ID NO. 16.

Offenbart ist auch die Nutzung von wie oben beschriebenen Verfahren. Ein wie oben beschriebenes Verfahren kann in einer Vorsorgeuntersuchung eines Menschen in einem Entwicklungsstadium genutzt werden, das zu früh für das Auftreten von manifesten Symptomen von Legasthenie ist.

Die Vorsorgeuntersuchung kann eine routinemäßige Vorsorgeuntersuchung für Kinder im Alter von zwölf Monaten sein.

In einer weiteren Ausführungsform zur Nutzung eines Verfahrens gemäß der Erfindung kann das wie oben beschriebene Verfahren genutzt werden, um eine frühe Diagnose zu erreichen und Kindern mit einem Risiko einer Legasthenie spezielle medizinische und/oder pädagogische Behandlung zukommen zu lassen.

In einer weiteren bevorzugten Ausführungsform kann ein wie oben beschriebenes Verfahren genutzt werden, um die Legastheniediagnose eines schon mit herkömmlichen Verfahren wie Lese- und Rechtschreibtests getesteten Menschen zu erhärten.

Die Erfinder haben herausgefunden, dass es möglich ist, bei einem Menschen ein Legasthenierisiko zu diagnostizieren, basierend auf der Bestimmung des Genotyps des besagten Menschen in bestimmten Regionen, ausgewählt aus der erwähnten Gruppe von chromosomalen Regionen,. Nach dem Vergleich des so bestimmten Genotyps mit dem Genotyp einer Kontrolle ist es möglich, einem Menschen ein Legasthenierisiko zuzuweisen. Weiterhin haben die Erfinder herausgefunden, dass besagte Diagnose auf der Bestimmung von mindestens einem Genotyp eines wie in Tabelle 2.1 aufgeführten SNPs und dem Vergleich von besagtem Genotyp mit dem nicht mit Legasthenie korrespondierenden Genotyps basieren kann.

Einzelnukleotid-Polymorphismen (engl. Single nucleotide polymorphism (SNP)) sind dem Fachmann bekannt, insbesondere werden Variationen einzelner Basenpaare in einem DNS-Strang so bezeichnet. Vorzugsweise liegt ein SNP dann vor, wenn mindestens 1% der Population eine Abweichung in der Base des SNPs ausweist. Je nach Basenaustausch kann die Information des Kodons in einer kodierenden Sequenz verändert sein. Kodiert das Triplet weiterhin für die gleiche Aminosäure, spricht man von einem synonymen SNP. Bei einem nicht synonymen SNP erfolgt ein Aminosäurewechsel, d. h. das durch die Sequenz kodierte Protein weist eine abweichende Aminosäure-Sequenz auf. Liegt ein nicht synonymer SNP in einer kodierenden Region, auch als cSNP (engl. coding SNP) kann eine veränderte Base eine Auswirkung auf die Proteinfunktion haben und physiologische Defekte nach sich ziehen. In einer Ausführungsform liegt der SNP in einer regulatorischen Region, d. h. die Genregulation wird durch den SNP gestört. Solche SNPs werden üblicherweise als rSNP (regulatory SNP) bezeichnet. Diese liegen in regulatorischen Regionen und beeinflussen die Transkription und folglich die Proteinexpression und die daraus resultierende Proteinkonzentration. Weiter kann durch SNPs die RNS-Prozessierung gestört werden. Solche SNPs werden als srSNP (engl. structural RNA-SNP) bezeichnet. Zum Beispiel kann eine Basenänderung an einer Spleißstelle zu einer mRNS führen, die für funktionsloses Protein kodiert.

Die Erfinder haben nun festgestellt, dass die vier oben genannten SNPs mit dem Vorliegen von Legasthenie korrelieren, d. h., dass man anhand der SNPs eine diagnostische Aussage treffen kann. Daher ist es eine bevorzugte Ausführungsform der vorliegenden Erfindung, dass in dem Verfahren zur Diagnose eines Legasthenierisikos der Genotyp in einem der SNPs, ausgewählt aus der Gruppe, bestehend aus rs7162960, rs496888, rs2715079 und rs2374341 bestimmt wird. Der Datenbank ist zu entnehmen, dass SNP rs7162960 in der Region des Gens BLM liegt, dass rs496888 in der Region des Gens TNFRSF1B liegt und dass die beiden SNPs rs2715079 und rs2374341 auf Chromosom 2 liegen.

In einer Ausführungsform der vorliegenden Erfindung beinhaltet das Verfahren zur Diagnose die Bestimmung des Genotyps der Nukleinsäure der Probe für einen SNP, ausgewählt aus der oben genannten Gruppe. Deshalb ist es offenbart, folgende Verfahren bereitzustellen: Verfahren zur Diagnose eines Legasthenierisikos umfassend die folgenden Schritte: a) Bereitstellung einer Nukleinsäure beinhaltenden Probe von einem zu diagnostizierenden Menschen; b) Bestimmung des Genotyps der Nukleinsäure der Probe für mindestens einen SNP, ausgewählt aus der Gruppe, bestehend aus rs7162960, rs496888, rs2715079 und rs2374341; c) Vergleich des in Schritt b) bestimmten Genotyps mit dem Genotyp einer Kontrolle zur Feststellung von Abweichungen des bestimmten Genotyps vom Genotyp der Kontrolle; d) Zuschreiben einer Präsenz eines Legasthenierisikos in der zu diagnostizierenden Person bei Vorliegen mindestens einer festgestellten Abweichung des bestimmten Genotyps vom Genotyp besagter Kontrolle in mindestens einer der besagten Regionen. Offenbart ist auch ein Verfahren zur Diagnose eines Legasthenierisikos umfassend die Schritte: a) Bereitstellen einer Nukleinsäure beinhaltenden Probe von einem zu diagnostizierenden Menschen; b) Bestimmung des Genotyps der DNS der Probe für rs7162960; c) Vergleichen des in Schritt b) bestimmten Genotyps mit dem Genotyp einer Kontrolle zur Feststellung von Abweichungen des bestimmten Genotyps vom Genotyp der Kontrolle; d) Zuschreiben einer Präsenz eines Legasthenierisikos in der zu diagnostizierenden Person bei Vorliegen einer festgestellten Abweichung des bestimmten Genotyps vom Genotyp besagter Kontrolle. Eine weitere Ausführungsform betrifft ein Verfahren zur Diagnose eines Legasthenierisikos umfassend die Schritte: a) Bereitstellen einer Nukleinsäure beinhaltenden Probe von einem zu diagnostizierenden Menschen; b) Bestimmung des Genotyps der Nukleinsäure der Probe für rs496888; c) Vergleichen des in Schritt b) bestimmten Genotyps mit dem Genotyp einer Kontrolle zur Feststellung von Abweichungen des bestimmten Genotyps vom Genotyp der Kontrolle; d) Zuschreiben einer Präsenz eines Legasthenierisikos in der zu diagnostizierenden Person bei Vorliegen einer festgestellten Abweichung des bestimmten Genotyps vom Genotyp besagter Kontrolle.

Offenbart ist auch ein Verfahren zur Diagnose eines Legasthenierisikos umfassend die Schritte: a) Bereitstellen einer Nukleinsäure beinhaltenden Probe von einem zu diagnostizierenden Menschen, b) Bestimmung des Genotyps der Nukleinsäure der Probe für rs2715079; c) Vergleichen des in Schritt b) bestimmten Genotyps mit dem Genotyp einer Kontrolle zur Feststellung von Abweichungen des bestimmten Genotyps vom Genotyp der Kontrolle; d) Zuschreiben einer Präsenz eines Legasthenierisikos in der zu diagnostizierenden Person bei Vorliegen einer festgestellten Abweichung des bestimmten Genotyps vom Genotyp besagter Kontrolle. Offenbart ist auch ein Verfahren zur Diagnose eines Legasthenierisikos umfassend die Schritte: a) Bereitstellen einer Nukleinsäure beinhaltenden Probe von einem zu diagnostizierenden Menschen, b) Bestimmung des Genotyps der Nukleinsäure der Probe für rs2374341; c) Vergleichen des in Schritt b) bestimmten Genotyps mit dem Genotyp einer Kontrolle zur Feststellung von Abweichungen des bestimmten Genotyps vom Genotyp der Kontrolle; d) Zuschreiben einer Präsenz eines Legasthenierisikos in der zu diagnostizierenden Person bei Vorliegen einer festgestellten Abweichung des bestimmten Genotyps vom Genotyp besagter Kontrolle.
Offenbart ist auch ein Verfahren zur Diagnose eines Legasthenierisikos umfassend die Schritte: a) Bereitstellen einer Nukleinsäure beinhaltenden Probe von einem zu diagnostizierenden Menschen; b) Bestimmung des Genotyps der Nukleinsäure der Probe für mindestens einer SNP, ausgewählt aus der Gruppe bestehend aus rs2715079 und rs2374341; c) Vergleichen des in Schritt b) bestimmten Genotyps mit dem Genotyp einer Kontrolle zur Feststellung von Abweichungen des bestimmten Genotyps vom Genotyp der Kontrolle; d) Zuschreiben einer Präsenz eines Legasthenierisikos in der zu diagnostizierenden Person bei Vorliegen mindestens einer festgestellten Abweichung des bestimmten Genotyps vom Genotyp besagter Kontrolle in mindestens einer der besagten Regionen.

Der Fachmann wird unmittelbar feststellen, dass die Kontrolle im Zusammenhang mit der vorliegenden Erfindung vorzugsweise ein oder mehrere Menschen sind, die nicht an Legasthenie leiden. Als Legasthenie versteht der Fachmann eine umschriebene und bedeutsame Beeinträchtigung in der Entwicklung der Lesefertigkeiten, die nicht allein durch das Entwicklungsalter, visuelle Probleme oder unangemessene Beschulung erklärbar ist. Legasthenie wird auch als Lese-und Rechtschreibstörung bzw. Dyslexie bezeichnet. Nach der internationalen statistischen Klassifikation der Krankheiten und verwandter Gesundheitsprobleme (ICD, engl. international statistical classification of disease and related health problems) in der 2015 gültigen Ausgabe ICD-10 wird der Dyslexie der Code ICD-10-F81.0 (ICD-10-R48) zugewiesen (Pettersson, E., Lundeberg, J., Ahmadian, A. (2009). Generations of sequencing technologies. Genomics, 93(2), 105-111). Eine Dyslexie wird üblicherweise bisher durch folgende Verfahren diagnostiziert. Ein dem Fachmann geläufiger Lese-Rechtschreib-Test ist der KNUSPEL-L (Marx, H. (1998). Knuspels Leseaufgaben (KNUSPEL-L). Göttingen, Hogrefe). Der Fachmann wird erkennen, dass das erfindungsgemäße Verfahren auch in Kombination mit bekannten Verfahren zur Diagnose von Legasthenie verwendet werden kann. Darüber hinaus wird in einer Ausführungsform der zu diagnostizierende Mensch auch auf das Nichtvorliegen von Aufmerksamkeitsdefizit (Hyperaktivitäts)-Syndrom (F90.0, F90.1) und einem IQ von unter 80, vorzugsweise unter 85, untersucht. Der zu diagnostizierende Mensch ist bevorzugt ein Mensch in einem Alter von 0 bis 8 Jahren, vorzugsweise von 0 bis 7 Jahren, weiter bevorzugt von 0 bis 6 Jahren. In einer weiteren Ausführungsform ist besagter zu diagnostizierender Mensch jünger als 12 Monate. Der Fachmann wird erkennen, dass die vorliegende Erfindung ein Verfahren bereitstellt, das Vorliegen einer Legasthenie in sehr frühen Stadien zu erkennen. Deshalb ist es auch möglich, mit dem erfindungsgemäßen Verfahren eine Legasthenie im Zuge einer Präimplantationsdiagnostik (PID) festzustellen. Auch dies ist einer möglichen Ausführungsform der vorliegenden Erfindung. In einer weiteren Ausführungsform wird das vorliegende Verfahren nicht im Zuge einer Präimplantationsdiagnostik verwendet. Dies gilt insbesondere bei ethischen Bedenken.

Im Kontext der vorliegenden Erfindung bezeichnet der Begriff "ungefähr" und "circa" ein Zuverlässigkeitsintervall, das ein Fachmann so versteht, dass der technische Effekt des in Frage stehenden Merkmals noch erreicht wird. Der Begriff bezeichnet "typischerweise" eine Abweichung des bezeichneten numerischen Werts von ±10% und bevorzugt ±5%.

Der Begriff "umfassend" darf nicht als limitierend verstanden werden. Für den Zweck der vorliegenden Erfindung wird der Begriff "bestehend aus" als bevorzugte Ausführungsform von "umfassend" angesehen. Falls nachstehend eine Gruppe dahingehend definiert ist, dass sie mindestens eine bestimmte Anzahl an Ausführungsformen umfasst, bezeichnet dies auch eine Gruppe, die vorzugsweise nur aus diesen Ausführungsformen besteht.

Im Kontext der vorliegenden Erfindung wird der Begriff "Legasthenie" so verwendet, wie er auf dem Gebiet der Medizin bekannt und definiert ist. "Legasthenie" soll eine komplexe, durch unerwartete Schwierigkeiten beim Erlernen von Lesen und/oder Schreiben und/oder Verstehen von Wörtern/Texten und/oder Buchstabieren charakterisierte Störung bei ansonsten normaler Intelligenz beschreiben. Die Krankheit kann sich in verschiedenen Schweregraden manifestieren, von denen alle durch den hier benutzten Begriff "Legasthenie" umfasst werden sollen. Es kann bevorzugt sein, den Begriff "Legasthenie" wie hier verwendet mit Leseschwäche zu assoziieren. Der Begriff "Legasthenie" wie hier verwendet kann jedoch auch mit allen Störungen und/oder Problemen beim Schreiben assoziiert werden.

Da das Verfahren der vorliegenden Erfindung die Analyse von genetischem Material betrifft, ist es in einem ersten Schritt notwendig, genetisches Material des zu testenden Individuums bereitzustellen. In den meisten Fällen kann besagtes Individuum ein Kind im Alter von 12 Monaten oder sogar jünger sein.

Zunächst ist es einfach, eine Nukleinsäure beinhaltende Probe zu erhalten, um die hierin offenbarten chromosomalen Regionen, insbesondere die SNP, zu analysieren, die an der Krankheit beteiligt sind. Weiterhin ist es aufgrund der Fortschritte, die in den letzten Jahren bei den Techniken für solch eine Analyse, d. h. Bestimmungen von Genotypen, gemacht wurden, möglich, sehr verlässliche Resultate zu erhalten. Besagte Bestimmungen sind heutzutage Routineverfahren und dem Fachmann bekannt. Die Analyse einer Probe kann in einer Chargenauswertung zusammen mit anderen Proben durchgeführt werden. Der Markt für besagte Detektionsverfahren ist hochkompetitiv, und daher sind die Kosten niedrig. In einer Ausführungsform wird eine Gewebeprobe des betreffenden Individuums als eine Nukleinsäure enthaltende Probe verwendet, wie z.B. Blut, Haut oder Speichel des besagten Individuums. Jede andere Probe kann auch verwendet werden, jedoch muss besagte Probe genetisches Material, d.h. DNS des Individuums, enthalten. Die Proben der zu diagnostizierenden Person können verschieden gewählt werden, solange sichergestellt ist, dass diese Nukleinsäure der Person enthalten. Vorzugsweise enthält die Probe Zellen der Person. In eine Ausführungsform ist die die Nukleinsäure enthaltende Probe ausgewählt aus der Gruppe bestehend aus Gewebeproben, Körperflüssigkeiten, Urin, Blut, Serum, Haut und Speichel. Jedes dem Fachmann bekannte Verfahren zur Sammlung und Bereitstellung besagter Probe kann verwendet werden. Eine Blutprobe kann zum Beispiel mittels einer mit einem sterilen Röhrchen verbundenen sterilen Nadel aus einer Vene eines Arms eines Individuums genommen werden. Eine Speichelprobe kann zum Beispiel genommen werden, indem ein steriles Wattestäbchen verwendet und Speichel aus dem Mund- und Rachenraum eines Individuums gesammelt wird, oder indem besagter Raum gespült und besagte Spüllösung gesammelt wird. Eine Speichelprobe kann jedoch auch Speichel enthalten, welcher von dem Individuum gespuckt und gesammelt wurde. Besagter erster Schritt des der Erfindung gemäßen Verfahrens kann direkten Kontakt mit dem Körper des zu untersuchenden Individuums umfassen. Jedoch, wie z.B. im Falle der Sammlung des gespuckten Speichels, involviert besagter erster Schritt nicht notwendigerweise Kontakt mit dem Körper des Individuums. In jedem Fall können alle weiteren Schritte außerhalb des menschlichen Körpers ausgeführt werden.

Sobald eine Nukleinsäure enthaltende Probe vom zu diagnostizierenden Menschen gewonnen wurde, kann es hilfreich sein, die Nukleinsäure, wie z.B. vorzugsweise DNS vor der Bestimmung des Genotyps aufzureinigen. Für diesen Aufreinigungsschritt kann jedes dem Fachmann bekannte Verfahren verwendet werden. Dies kann Zentrifugationsschritte, Ausfällungsschritte, Dialyseschritte, Erhitzungs- und Kühlschritte, Denaturierungsschritte und so weiter umfassen. Üblicherweise liegt am Ende solcher Aufreinigungsprotokolle die Nukleinsäure, vorzugsweise die DNS des zu diagnostizierenden Menschen in einem geeigneten Puffer in einer geeigneten Konzentration und einem Reinheitsgrad vor, der für die weitere Analyse ausreicht. In speziellen Fällen kann man die Menge und Reinheit der DNS nach den Aufreinigungsschritten bestimmen, z.B. mittels UV-Spektrometrie wie dem Fachmann bekannt. Auf diese Weise kann jede Verunreinigung aufgrund von noch in der Lösung vorhandenen Proteinen durch Messung bei einer Wellenlänge von 280 Nanometern definiert werden, wobei die Reinheit und Konzentration der DNS durch Verwendung von 260 Nanometern gefolgt von der Analyse der entsprechenden Höchstwerte analysiert werden kann. Jedoch kann jedes Standardprotokoll zur Aufreinigung von DNS mit dem Endziel der Bereitstellung einer für die verwendete Analysemethode geeigneten DNS-Probe verwendet werden. Da verschiedene wie unten diskutierte Verfahren verwendet werden können, kann die Aufreinigung auch gemäß der in späteren Schritten genutzten Methode variieren.

In einer Ausführungsform wird die Nukleinsäure des zu diagnostizierenden Menschen nicht nur aufgereinigt, sondern auch amplifiziert. Dies geschieht vorzugsweise während oder vor Schritt b) des erfindungsgemäßen Verfahrens. Die Amplifizierung kann also auch in Schritt a) geschehen. Mit der Amplifikation kann auch eine Umschreibung der Nukleinsäure einhergehen. Dies ist zum Beispiel geboten, wenn der Genotyp anhand der mRNS bestimmt werden soll. Dann erfolgt die Umschreibung in sogenannte cDNS mittels der Verwendung reverser Transkriptase, einem Verfahren, das dem Fachmann hinreichend bekannt ist (wie z.B. aus H. M. Temin, S. Mizutani: RNA-dependent DNA polymerase in virions of Rous sarcoma virus. In: Nature (1970), Band 226(5252), S. 1211-3. Erratum in: Nature. 1970 227(5253):102; Baltimore D: RNA-dependent DNA polymerase in virions of RNA tumour viruses. In: Nature. 226, Nr. 5252, Juni 1970, S. 1209-11; S. Spiegelman, K. F. Watson, D. L. Kacian: Synthesis of DNA complements of natural RNAs: a general approach. In: Proc Natl Acad Sci U S A. (1971), Band 68(11), S. 2843-2845; A. Efstratiadis, F. C. Kafatos, A. M. Maxam, T. Maniatis: Enzymatic in vitro synthesis of globin genes. In: Cell (1976), Band 7(2), S. 279-88A. M. Carothers, G. Urlaub, J. Mucha, D. Grunberger, L. A. Chasin: Point mutation analysis in a mammalian gene: rapid preparation of total RNA, PCR amplification of cDNA, and Taq sequencing by a novel method. In: Biotechniques (1989), Band 7(5), S. 494-6, 498-9; A. L. Shaffer, W. Wojnar, W. Nelson: Amplification, detection, and automated sequencing of gibbon interleukin-2 mRNA by Thermus aquaticus DNA polymerase reverse transcription and polymerase chain reaction. In: Anal Biochem. (1990), Band 190(2), S. 292-6T. W. Myers, D. H. Gelfand: Reverse transcription and DNA amplification by a Thermus thermophilus DNA polymerase. In: Biochemistry (1991), Band 30(31), S. 7661-6; M. J. Moser, R. A. DiFrancesco, K. Gowda, A. J. Klingele, D. R. Sugar, S. Stocki, D. A. Mead, T. W. Schoenfeld: Thermostable DNA polymerase from a viral metagenome is a potent RT-PCR enzyme. In: PLoS One (2012), Band 7(6), S. e38371). Dem Fachmann sind viele verschiedene Methoden der Amplifikation bekannt; die meisten basieren auf der PCR-Technik. Daher kann man Primer entwerfen, die stromaufwärts und stromabwärts der interessierenden Region gelegen sind (einer hybridisiert mit dem Watson-Strang, einer hybridisiert mit dem Crick-Strang), um besagte Region zu umfassen. Durch Hinzufügung geeigneter Enzyme, wie Polymerasen, und Verwendung mehrerer Temperaturzyklen, die Denaturierung, Anlagerung und Reaktionstemperaturen umfassen, kann die interessierende Region amplifiziert werden. Die PCR-Technik *per se* ist dem Fachmann bekannt und er ist in der Lage, je nach Template und interessierende Region die Parameter wie Temperaturen, Zykluslänge, Menge an Enzymen, Sequenz der Primer etc. zu wählen. Nach der Amplifikation können weitere Schritte folgen, um den Genotyp zu bestimmen. Solche Schritte können sein, z. B. DNS-Sequenzierung oder aber Detektionsmethoden, die auf Hybridisierung basieren. In Fällen, wo auf Hybridisierung basierende Detektionsmethoden in späteren Schritten benutzt werden, kann es bevorzugt sein, markierte Primer und/oder markierte Nukleotide in die Amplifikationsreaktion einzuschließen, um die interessierenden DNS-Regionen schon in diesem initialen Schritt für eine einfachere zweckmäßigere Detektion besagter Regionen in den entsprechenden folgenden Assays, wie DNS-Mikroarrays, zu markieren.

Somit umfassen die ersten Schritte des Verfahrens zur Detektion eines Legasthenierisikos in einem Menschen die Bereitstellung einer Probe, die die DNS des Individuums enthält, vorzugsweise die Aufreinigung besagter DNS aus der Probe sowie vorzugsweise die Amplifizierung der interessierenden Regionen besagter DNS vor den folgenden Analyseschritten. Alle diese bisher erwähnten Schritte und Verfahren sind dem Fachmann bekannte Routineverfahren, und jedes bekannte für den entsprechenden Zweck geeignete Verfahren kann verwendet werden.

Im folgenden Schritt kann die optional aufgereinigte und amplifizierte DNS nun in bestimmten DNS-Regionen mittels eines Verfahrens analysiert werden, das weiter unten nach der Diskussion der interessierenden genomischen Regionen ausgeführt ist.

Die Erfinder haben überraschenderweise chromosomale Regionen identifiziert, die bei Legasthenie involviert sind. In zweien der Regionen liegen annotierte Gene vor, die in Tabelle 1 aufgelistet sind und an der Anfälligkeit für Legasthenie beteiligt sind. Diese Gene sind zusammen mit den EntrezGene GeneID-Nummern, wie in der NCBI Datenbank (http://www.ncbi.nlm.nih.gov/Genbank/index.html) verwendet, und den entsprechenden Alias-Namen der Gene in Tabelle 1 aufgelistet.

**Tabelle 1: Gene, die an der Anfälligkeit für Legasthenie beteiligt sind**

| | Gene | EntrezGene Zugangskode (GenID-Nummer) | Alias |
|---|---|---|---|
| 1 | TNFRSF1B | 7133 | CD120b, TBPII, TNF-R-II, TNF-R75, TNFBR, TNFR1B, TNFR2, TNFR80, p75, p75TNFR, TNF-R2; TNF-RII; p75 TNF receptor; p80 TNF-alpha receptor; soluble TNFR1B variant 1; tumor necrosis factor beta receptor; tumor necrosis factor binding protein 2; tumor necrosis factor receptor 2; tumor necrosis factor receptor superfamily member 1B; tumor necrosis factor receptor type II |
| 2 | BLM | 641 | BS, RECQ2, RECQL2, RECQL3, Bloom syndrome protein; DNA helicase, RecQ-like type 2; recQ protein-like 3 |

Offenbart ist somit die Diagnose eines Legasthenierisikos auf Grund von Abweichungen im Genotyp der zu diagnostizierenden Person in einem der Gene aus Tabelle 1 verglichen mit dem Genotyp einer Kontrolle in besagten Genen.

In einer sehr vereinfachten Sichtweise eines "gesunden" und eines "kranken" Zustands in Bezug auf eine genetische Komponente kann man die Assoziation eines Genotyps mit einer Krankheit wie folgt erklären.

Für besagten Genotyp kann ein "Wildtyp" Zustand definiert werden. Besagter Zustand kann die Expressionsregulation eines Gens oder Genprodukt selbst umfassen, wie hierin im Detail definiert. Getrennt vom Wildtyp-Zustand kann auch ein "Mutanten"-Zustand definiert werden. Besagter Zustand bezieht sich auf eine vom Wildtyp abweichende Situation. Bei SNP wird der Wildtyp-Zustand auch als der Zustand bezeichnet, der hierin ebenfalls definiert ist als der Zustand, der bei den meisten Individuen einer Population vorliegt, die nicht an der zu diagnostizierenden Krankheit leiden. Weiterhin ist eine chromosomale Region oder SNP in jedem Individuum in zwei Allelen vorhanden. Bei der Bezugnahme auf die zwei Allele besagter chromosomaler Region oder SNP bezieht man sich üblicherweise auf den Genotyp des besagten Allels. Die Begriffe "Allel" und "Genotyp des besagten Allels" oder einfach "Genotyp" werden in vorliegender Erfindung wie dem Fachmann bekannt verwendet. Folglich sind in jedem Individuum zwei Allele eines Gens vorhanden, und besagte zwei Allele können identisch sein, beispielsweise im Wildtyp-Zustand; in diesem Fall wird der Genotyp als "homozygoter Wildtyp" für besagtes Allel bezeichnet. Im Falle, dass sich besagte zwei Allele in entweder einer einzelnen Base oder einer bestimmten Region unterscheiden, wird der Genotyp als "heterozygot" für besagte Region des besagten Allels bezeichnet. Wie oben dargelegt kann ein "Wildtyp-Zustand" eines Gens die Regulation und Expression eines Gens umfassen. Im Folgenden werden die Begriffe "Regulation" und "Expression" angesprochen.

Die Regulation eines Gens hängt, zumindest teilweise, von Proteinbindungspartnern (wie Transkriptionsfaktoren) ab, die an regulatorische DNS-Regionen besagten Gens binden. Besagte regulatorische Regionen unterscheiden sich üblicherweise von den kodierenden Regionen eines Gens, d.h. sie können zum Beispiel in einer Region weit weg von der kodierenden Region selbst lokalisiert sein. Die Bindung von Proteinen an DNS hängt üblicherweise von einer bestimmten Sequenz von Nukleotiden in besagter Region ab; folglich kann eine definierte Sequenz Andockstellen für Transkriptionsfaktoren schaffen. An diesen Andock- oder Bindestellen können Proteinkomplexe assemblieren, die die "Expression des Gens" regulieren. Folglich können bestimmte regulatorische Sequenzen, wie beispielsweise Promotoren, eine wichtige Rolle für den Expressionsgrad eines Gens spielen. Im Kontext der vorliegenden Erfindung kann jede Sequenz, die nicht das Genprodukt kodiert, aber Teil besagten Gens ist, als "regulatorische Region" bezeichnet werden; auch sowohl stromaufwärts als auch -abwärts gelegene regulatorische Regionen und Introns werden als "regulatorische Region" bezeichnet.

Der wie hier verwendete Begriff "Expression eines Gens" umfasst mindestens zwei Schritte. Die Transkription des interessierenden Gens in entsprechende RNS ist ein Schritt der Genexpression. Im Falle, dass ein Protein durch das Gen kodiert wird, wird besagte RNS als mRNS bezeichnet. Die mRNS kann weiter verarbeitet (z.B. gespleißt) werden, und in einem zweiten Schritt wird die mRNS in ein Protein translatiert. Proteine können die Endprodukte der Genexpression sein. Die Menge eines Proteins als Endprodukt kann mit dem Grad der Genexpression korrelieren. Jedoch können auch RNS-Moleküle, wie mikroRNS, die Endprodukte der Genexpression sein. Besagte RNS können in einem zweiten Schritt auch Gegenstand von Verarbeitung und Modifikation sein. Im Kontext der vorliegenden Erfindung können Proteine als Genendprodukte bevorzugt sein und sind nachfolgend diskutiert. Jedoch werden andere Genendprodukte einschließlich Spleiß-Varianten und dergleichen auch von der vorliegenden Erfindung umfasst.

Die kodierende Region eines Gens bestimmt eine definierte Sequenz von Aminosäuren entsprechend dem genetischen Kode. Besagte Aminosäuresequenz scheint selbst die Gesamtstruktur des letztendlichen Proteins zu definieren und zu bestimmen.

Die kodierende Sequenz eines Gens muss nicht notwendigerweise ununterbrochen sein; es kann dazwischen nicht-kodierende Abschnitte geben, die regulatorische Funktion haben können. Typischerweise bezeichnet man die Exons eines Gens als die kodierenden Regionen, wohingegen Introns als nicht-kodierende Regionen bezeichnet werden. Wie oben erwähnt, können besagte Regionen regulatorische Funktionen haben. Jedoch sind beide Regionsarten Teil eines Gens. Im Kontext von Proteinen als Genendprodukt soll der Begriff "kodierende Region" oder "kodierende Sequenz" eine DNS-Sequenz umfassen, welche am 5'-Ende mit dem Startkodon, d.h. ATG, beginnt. Dieses Triplett kodiert die erste Aminosäure, ein Methionin, des entsprechenden Proteins, wenn die Translation der mRNS initiiert wird.

Entsprechend endet die kodierende Region am 3'-Ende mit einem Stopkodon, z.B. TAG, um die Proteintranslation zu beenden. Die Translation der mRNS resultiert in einem Protein, welches sich aus verschiedenen Domänen/Polypeptiden/Teilen zusammensetzen kann.

Es kann folglich eine definierte DNS-Sequenz eines Gens geben, die aus regulatorischen und kodierenden Regionen zusammengesetzt ist. Dies kann zu einer definierten und regulierten Expression besagten Gens führen, wobei das Genprodukt z.B. ein Protein mit einer definierten Aminosäuresequenz und Funktion ist. Dies kann als der Wildtyp-Zustand besagten Gens bezeichnet werden.

Besagtes Gen kann jedoch nicht im Wildtyp-Zustand vorliegen, sondern vielmehr in einem mutierten Zustand, z.B. hinsichtlich der Expression oder der Struktur des Genprodukts.

Mutationen in den regulatorischen Regionen eines Gens können zur Fehlregulation der Genexpression führen; Mutationen z.B. in Promotorsequenzen können die Bindestellen für in der Genregulation wichtige Transkriptionsfaktoren stören oder Bindungsaffinitäten beeinflussen, was entweder zu einem höheren oder erniedrigten Expressionsgrad führen kann. Obwohl die Endprodukte der Genexpression, z.B. ein Protein, so funktional wie das Wildtyp-Protein sein können, kann die Fehlregulation zu einem Erkrankungszustand führen.

Abgesehen von der Fehlregulation können "Fehler" im Genendprodukt auch die Ursache für eine Krankheit sein. Wenn ein Protein das Endprodukt ist, kann es sich folglich vom Wildtyp-Protein in einer oder mehreren Aminosäuren unterscheiden. Im Falle, dass eine für die Gesamtstruktur des Proteins wichtige Aminosäure betroffen ist und diese von einer Aminosäure ersetzt wird, die die strukturellen Erfordernisse nicht erfüllt, kann die Gesamtstruktur gestört und das Protein nicht funktional sein. Ferner können Bindungsaffinitäten eines Proteins aufgrund von unterschiedlichen, in dem mutierten Protein vorhandenen Oberflächen gestört sein. Weiterhin gibt es definierte Positionen für bestimmte für die enzymatische Funktion wichtige Aminosäuren, deren Austausch das gesamte Enzym inaktiviert. Ein Protein kann auch inaktiviert werden, indem es in unterschiedlichem Grade verkürzt wird oder indem eine neue Domäne und/oder ein Teil eines anderen Proteins eingefügt wird.

Die Basis für ein nicht funktionales Protein können Mutationen in der für besagte Region kodierenden DNS sein. Im Falle, dass beispielsweise ein Nukleotid in der kodierenden Sequenz fehlt oder ein Nukleotid zusätzlich eingefügt wurde, wird der aus Nukleotidtripletts zusammengesetzte genetische Kode verschoben und kodiert folglich in den meisten Fällen eine Nonsense-Aminosäuresequenz. Dies wird üblicherweise als Leserasterverschiebung bezeichnet. Jedoch können andere Mutationen, wie Punktmutationen, zu Aminosäureaustauschen oder zur Erzeugung von Stopkodons führen, was wiederum zu verkürzten Proteinen führt. Zusätzliche Mutationen auf DNS-Ebene umfassen Deletionen, Insertionen, Inversionen und Translokationen, die alle zu einer neuen Sequenz verglichen mit der Wildtyp-Situation führen. Solche Effekte können verständlicherweise für schwere Funktionsverluste im Produkt der Genexpression verantwortlich sein.

Folglich können in der kodierenden Region eines Gens vorhandene Punktmutationen, Insertionen, Deletionen, Inversionen und/oder Translokationen (und dergleichen, im Folgenden zusammengefasst als "Mutationen") zu einem nicht funktionellen Genexpressionsprodukt führen; das Vorhandensein besagter Mutationen in regulatorischen Regionen kann zu einer Fehlregulation führen.

Falls ein Gen eine wichtige Funktion in der Zelle erfüllt, kann ein mutierter Zustand von besagtem Gen direkt mit einer Krankheit verbunden sein, die aus der Fehlregulation und/oder Fehlfunktion besagten Gens resultiert.

Jedoch führt nicht jede Mutation in besagtem Gen notwendigerweise zu einer Krankheit. Erstens kann die Mutation den Zustand des Gens nicht drastisch verändern. Zweitens kann die Mutation durch eine zweite Mutation in besagtem Gen kompensiert werden. Weiterhin können andere Gene die Funktion besagten Gens übernehmen und so weiter. Wie oben erklärt sind in einem Individuum zwei Allele eines Gens vorhanden; so kann in einigen Fällen ein Allel im Wildtyp-Zustand in der Lage sein, das mutierte Allel zu kompensieren. Daher ist es eine Ausführungsform der vorliegenden Erfindung, dass das Verfahren die Bestimmung umfasst, ob die Abweichung homozygot oder heterozygot vorliegt. Der Fachmann ist in der Lage solche Bestimmungen durchzuführen, insbesondere anhand der hierin offenbarten Verfahren und Referenzen. In einer Ausführungsform erfolgt also das Zuschreiben einer Präsenz eines Legasthenierisikos in der zu diagnostizierenden Person bei homozygotem Vorliegen besagter Abweichung(en) des bestimmten Genotyps vom Genotyp besagter Kontrolle erfolgt.

Die Assoziation eines Gens mit einer Krankheit scheint folglich stark von seiner Funktion abhängig zu sein; falls ein Gen in einen bestimmten zellularen Prozess verwickelt ist und dort essentiell zu sein scheint, kann die Inaktivierung von entweder einem oder beiden Allelen besagten Gens aufgrund der Unterbrechung besagten zellularen Prozesses direkt zu einer Krankheit führen. Weiterhin scheint die Assoziation eines Gens im mutierten Zustand abhängig zu sein von in anderen Genen vorhandenen Mutationen, z.B. in demselben Signalweg. Besagte Gene können parallele Faktoren in einem Signalweg sein oder essentielle stromaufwärts- und stromabwärts gelegene Faktoren eine zellulären Signalwegs; Es muss also, wenn eines der zwei parallelen Genen nicht funktional ist, nicht zu einem Erkrankungszustand kommen; jedoch können Mutationen in beiden Genen bei einem Individuum zu besagter Krankheit führen. Weiterhin wird die Situation vom allelischen Zustand der besagten Gene beeinflusst, d.h. ob der Genotyp homozygot für die Mutation ist oder heterozygot.

Zusammenfassend gesagt kann ein spezifischer Genotyp eines Gens einer chromosomalen Region mit einer Krankheit assoziiert sein.

Wenn ein bestimmter Satz von Genen in einen zellulären Prozess verwickelt ist, dessen Störung eine spezifische Krankheit zu erklären scheint, kann man folglich spezifische Genotypen von besagten mit besagter Krankheit assoziierten Genen finden, indem man die im zu testenden Individuum gefundenen Genotypen mit der Situation eines nicht an der Krankheit leidenden Individuums vergleicht (Kontrolle). Selbstverständlich kann man sich nicht nur auf ein einzelnes Referenzindividuum als Kontrolle beziehen, das nicht mit der Krankheit behaftet ist, sondern vielmehr auf eine große Population von gesunden Referenzindividuen. In einer bevorzugten Ausführungsform der vorliegenden Erfindung ist die Kontrolle daher eine Population von Referenzindividuen, die keine Legasthenie aufweisen. Dabei können Informationen über besagten Satz von Genen und deren entsprechende Genotypen in besagter Referenzpopulation gemittelt und dann mit der spezifischen Situation bei einem zu testenden Individuum verglichen werden. Wenn mindestens ein essentielles Gen aus dem Satz von Genen mehr mutierte Genotypen verglichen mit besagter gemittelter Referenzpopulation zeigt, kann das Individuum als vermutlich unter der Krankheit leidend kategorisiert werden. Jedoch ist es wichtig zu beachten, dass im Allgemeinen auch Wildtyp-Genotypen mit einer Krankheit assoziiert sein können.

Zusammenfassend gesagt kann man die regulatorischen und/oder kodierenden Regionen eines Gens auf das Vorhandensein von Punktmutationen, Deletionen, Insertionen und/oder Translokationen analysieren, um Unterschiede zur "Wildtyp-Situation" zu finden, d.h. Unterschiede zur Situation in Individuen ohne erhöhtes Legasthenierisiko. Weiterhin kann man die Genotypen besagter Allele analysieren, d.h. entweder homozygot oder heterozygot für besagte Mutation. Jedoch führt, wie oben dargelegt, nicht jede Mutation, die in einem Gen gefunden wurde, das allgemein in einen mit der Krankheit assoziierten Prozess verwickelt ist, notwendigerweise zu der Krankheit. Daher ist der Vergleich mit einer Referenzanalyse (d.h. mit einer Probe von mindestens einem nicht von besagter spezieller Krankheit betroffenen Individuum) essentiell, um zu bestimmen, ob der Genotyp mit der Krankheit assoziiert zu sein scheint.

Wie oben erwähnt haben die Erfinder herausgefunden, dass alle in Tabelle 1 aufgelisteten chromosomalen Regionen/Gene bei der Anfälligkeit für Legasthenie eine Rolle spielen.

Mit den oben gegebenen Definitionen und Erklärungen für "Wildtyp" und "mutierten" Zustand, sollte die DNS des zu testenden Individuums in mindestens einer der genannten Regionen analysiert werden. Besagte Analyse sollte auf alle wie oben dargelegten Mutationen fokussiert sein, die entweder einen Effekt auf die Regulation oder das Endprodukt der Genexpression, beispielsweise ein Protein, zu haben scheinen. Dasselbe gilt für die unten aufgelisteten bevorzugten Ausführungsformen. Wie oben dargelegt scheint der Vergleich mit einem nicht legasthenen Referenzindividuum und vorzugsweise einer großen Gruppe von nicht legasthenen Individuen entscheidend.

Es kann weiterhin möglich sein, nur ein Gen oder einen intergenischen Marker zu analysieren, falls eine entsprechend stärkere Assoziation besagten Gens mit Legasthenie vorliegt. Es ist offenbart, dass die, in den der Region von Nukleotid 41930000 bis 42032000 des Chromosom 2 und der Region von Nukleotid 51508000 bis 51885000 des Chromosom 2 gelegenen, intergenischen Marker rs2715079 und rs2374341 für die Analyse ausgewählt werden können.

Im Schritt c) des erfindungsgemäßen Verfahrens wird das Resultat besagter Analyse aus Schritt b) mit dem Resultat einer Analyse eines nicht legasthenen Referenzindividuums (Kontrolle) verglichen. Falls ein mutierter Genotyp im getesteten Individuum auch in nicht legasthenen Referenzindividuum gefunden wird, steht besagter mutierter Genotyp mit einer geringeren Wahrscheinlichkeit mit Legasthenie in Verbindung stehen. Falls jedoch das nicht legasthene Referenzindividuum eine homozygote Wildtyp-Situation zeigt, wohingegen das getestete Individuum eine mutierte Situation, die z.B. zu einem deregulierten und/oder nicht funktionalen oder dysreguliertem Genprodukt führt, zeigt, zeigt dies eine hohe Wahrscheinlichkeit einer Assoziation mit Legasthenie, insbesondere wenn besagte Mutation homozygot ist. In einer bevorzugten Ausführungsform der vorliegenden Erfindung kann besagtes nicht legasthene Referenzindividuum nicht ein einzelnes Individuum, sondern vielmehr eine große Gruppe von Individuen sein, die nicht an Legasthenie leiden. Besagte Gruppe von nicht legasthenen Individuen kann mindestens 100, mindestens 125, mindestens 150, mindestens 200, mindestens 500, mindestens 1000, mindestens 5000, mindestens 7000, mindestens 10.000 nicht legasthene Individuen umfassen. Dem Fachmann ist ersichtlich, dass durch die vorliegende Erfindung auch eine Referenz bereitgestellt wird, sodass im Verfahren keine parallele Testung von Referenzindividuen erfolgen muss, sondern gegen eine vorhandene Referenz verglichen wird, vorzugsweise zu den hierin offenbarten Referenzen. Anders gesagt, es wird überprüft, ob eine Abweichung zu den hierin offenbarten Sequenzen vorliegt.

In Schritt d) eines Verfahrens gemäß der vorliegenden Erfindung wird Legasthenie einem Menschen basierend auf dem oben erwähnten Vergleich zugewiesen. Allgemein basiert besagte Zuweisung auf der Anzahl der Unterschiede zwischen dem getesteten Individuum und dem Referenzindividuum beziehungsweise der nicht legasthenen Referenzgruppe. Je höher also die Anzahl an Abweichungen zwischen den Resultaten des getesteten Individuums und den Resultaten von nicht legasthenem Referenzindividuum beziehungsweise -gruppe, desto höher ist die Wahrscheinlichkeit einer Anfälligkeit für Legasthenie im getesteten Individuum.
Es ist auch offenbart, dass man die Gene TNFRSF1B, BLM sowie die mit Legathenie assoziierte Region DYX3 um die intergenischen Marker rs2715079 und rs2374341 auf das Vorhandensein von etablierten SNPs untersuchen kann. Es kann bevorzugt sein, SNPs aus den SNPs in den kodierenden Regionen besagter Gene auszuwählen.

Ein Weg, mit Legasthenie assoziierende Genotypen experimentell zu bestimmen, ist der Vergleich besagter Genotypen in mehrheitlich legasthenen und mehrheitlich nicht legasthenen Individuen. Zu diesem Zweck kann man in zwei aus entweder Legasthenikern oder Nicht-Legasthenikern zusammengesetzten Gruppen Informationen über die entsprechende Region sammeln (z.B. durch Bestimmung des Genotyps eines SNPs). Statistische Analyse wird dann für die Berechnung verwendet, ob ein Genotyp besagter analysierter Region mit Legasthenie assoziiert oder nicht.

Es versteht sich, dass es essentiell für die vorliegende Erfindung ist, zu bestimmen, ob ein SNP im wie oben definierten risikoassoziierten oder nicht-risikoassoziierten Zustand vorliegt. Informationen über besagten Zustand, d.h. risikoassoziiert oder nicht-risikoassoziiert, sind die für die Legasthenierisikodiagnose relevanten Informationen; folglich ist in der Spalte "Typ" der mit Legasthenie assoziierende Typ jedes SNPs aufgelistet; selbstverständlich ist dem Fachmann bewusst, dass besagter risikoassoziierter oder nicht-risikoassoziierter Zustand mit zwei unterschiedlichen komplementären Allelen an der entsprechenden Position des SNPs angegeben werden kann, abhängig davon, wie die Analyse durchgeführt wird. Da DNS aus zwei komplementären Strängen besteht, hängt das den besagten Zustand an der SNP-Position angebende Nukleotid, d.h. ein A, ein T, ein G, oder ein C vom analysierten Strang ab: wenn der kodierende Strang analysiert wird, kann ein G das Nukleotid sein, das entweder mit einem minor oder major Zustand assoziiert ist. Wenn jedoch der komplementäre nicht-kodierende Strang analysiert wird, wird ein C das Nukleotid sein, das entweder mit einem risikoassoziierten oder nicht-risikoassoziierten Zustand (und umgekehrt) assoziiert ist. Dasselbe trifft selbstverständlich für ein T oder ein A zu. Folglich kann ein T an der Position des SNPs auf dem kodierenden Strang mit Legasthenie assoziiert sein (z.B. als risikoassoziierter Typ). Wenn jedoch der nicht-kodierende Strang analysiert wird, wird ein A als mit Legasthenierisiko assoziiert interpretiert, da es eben auch den risikoassoziierten Typ abbildet.

Daher ist es wichtig anzumerken, dass in der vorliegenden Erfindung durchgehend alle Nukleotide, die an spezifischen SNP-Positionen entweder den risikoassoziierten oder nicht-risikoassoziierten Zustand (z.B. Spalte "korr. Base" in Tabelle 2.1) bezeichnen, auf dem kodierenden Strang der DNS lokalisiert sind. Folglich ist das angegebene Nukleotid nur mit entweder dem risikoassoziierten oder nicht-risikoassoziierten Zustand assoziiert, wenn besagter kodierender Strang analysiert wird. Es ist dem Fachmann klar, dass das entsprechende komplementäre Nukleotid besagten risikoassoziierten oder nicht-risikoassoziierten Zustand bezeichnen wird, wenn der nicht-kodierende DNS-Strang analysiert wird.

Zusammenfassend gesagt wird der Zustand eines SNPs, d.h. risikoassoziierten oder nicht-risikoassoziierten, bestimmt werden. Das Nukleotid an besagter exakter Position kann variieren, abhängig davon, ob der kodierende oder nicht-kodierende Strang analysiert wird. Beide, die hier angegebenen Nukleotide auf dem kodierenden Strang oder deren komplementäre Nukleotide auf dem nicht-kodierenden Strang, können für besagte Analyse verwendet werden und sind von der vorliegenden Erfindung umfasst.

Die Erfinder haben festgestellt, dass für die bevorzugten SNPs der Zustand minor mit dem Vorliegen einer Legasthenie in dem zu diagnostizierenden Menschen assoziiert. Das heißt, in einer bevorzugten Ausführungsform der vorliegenden Erfindung ist besagte Abweichung ein "risikoassoziierten"-Zustand für einen SNP, ausgewählt aus der Gruppe, bestehend aus rs496888, rs7162960, rs2715079 und rs2374341. Es wurde darüber hinaus festgestellt, dass der "nicht-risikoassoziierte"-Zustand für rs496888 das Nukleotid C für den Referenzstrang im Sinne des "GRCh37.p10 reference assembly (GCF 000001405.22)" kodierenden Strang, für rs7162960 das Nukleotid A für den kodierenden Strang ist, für rs2715079 das Nukleotid A für den kodierenden Strang und für rs2374341 das Nukleotid C für den kodierenden Strang. Daher betrifft die vorliegende Erfindung in einer Ausführungsform ein Verfahren zur Diagnose eines Legasthenierisikos, umfassend die Schritte: a) Bereitstellung einer DNS beinhaltenden Probe von einem zu diagnostizierenden Menschen; b) Bestimmung des Genotyps der DNS der Probe für mindestens ein SNP, ausgewählt aus der Gruppe, bestehend aus rs496888, rs7162960, rs2715079 und rs2374341; c) Zuschreiben einer Präsenz eines Legasthenierisikos in der zu diagnostizierenden Person bei Vorliegen eines anderen Nukleotids als C für rs496888, Vorliegen eines anderen Nukleotids als A für rs7162960, Vorliegen eines anderen Nukleotids als A für rs2715079 oder das Vorliegen eines anderen Nukleotids als C für rs2374341 der Präsenz eines Legasthenierisikos in der zu diagnostizierenden Person zugeschrieben wird.

In einer bevorzugten Ausführungsform erfolgt das Zuschreiben der Präsenz eines Legasthenierisikos bei mindestens zwei der abweichenden Nukleotiden der zuvor genannten SNPs.

Insbesondere wurde von den Erfindern festgestellt, dass das Vorliegen eines C für rs7162960, das Vorliegen eines A für rs496888, das Vorliegen eines C für rs2715079 und/oder das Vorliegen eines T für rs2374341 die Präsenz eines Legasthenierisikos in der zu diagnostizierenden Person anzeigt. Daher ist es eine bevorzugte Ausführung der vorliegenden Erfindung ein Verfahren zur Diagnose eines Legasthenierisikos bereitzustellen, das folgende Schritte umfasst: a) Bereitstellen einer DNS beinhaltenden Probe von einem zu diagnostizierenden Menschen; b) Bestimmung des Genotyps der DNS der Probe für mindestens ein SNP, ausgewählt aus der Gruppe, bestehend aus rs7162960, rs496888, rs2715079 und rs2374341; c) Zuschreiben einer Präsenz eines Legasthenierisikos in der zu diagnostizierenden Person bei Vorliegen eines A für rs496888, bei Vorliegen eines C für rs7162960, bei Vorliegen eines C für rs2715079 oder bei Vorliegen eines T für rs2374341.

In einer besonders bevorzugten Ausführungsform erfolgt das Zuschreiben der Präsenz eines Legasthenierisikos bei Vorliegen mindestens zweier der genannten Nukleotide, besonders bevorzugt bei mindestens drei der genannten Nukleotide, weiter bevorzugt bei Vorliegen aller vier genannten Nukleotide für die entsprechenden Positionen

In Tabelle 2.1 sind einige SNPs in den Genen TNFRSF1B, BLM sowie die intergenischen Marker rs2715079 und rs2374341 aus Tabelle 1 mit entsprechenden p-Werten oder r-Werten <0.05 angegeben. Wie unten erklärt haben die Erfinder herausgefunden, dass besagte SNPs mit Legasthenie assoziiert sind.

Ein spezifischer Genotyp eines der besagten SNPs, der in einem zu testenden Individuum vorliegt, kann folglich direkt als mit Legasthenie assoziiert oder eben nicht zugewiesen werden. In diesem Fall wird der Vergleich nicht mit einer nicht legasthenen Referenzgruppe durchgeführt, sondern mit den Resultaten von Daten, welche für assoziierte SNPs gefunden wurden, und zwar unter Verwendung des Versuchsaufbaus des Vergleichs legasthener und nicht legasthener Individuen. Es ist wichtig anzumerken, dass man Legasthenie auch direkt basierend auf besagter Identifikation beziehungsweise den assoziierenden Regionen zuweisen kann, sobald weitere assoziierende Regionen in den 4 genomischen loci in Tabelle 1 identifiziert wurden.

Die in der vorliegenden Offenbarung erwähnten SNPs sind gemäß der rs-Nomenklatur klassifiziert. Jede rs-Nummer korrespondiert mit exakt einer Position im Genom, wo ein SNP detektiert wurde. Besagtes Resultat wurde als Variation an die dbSNP-Datenbank übermittelt. Die dbSNP-Datenbank ist für die Öffentlichkeit zugänglich, und es kann via NCBI darauf zugegriffen werden (http://www.ncbi.nlm.nih.gov/). Besagte SNPs können folglich vorzugsweise aus der Gruppe der in Tabelle 2.1 aufgelisteten SNPs 1 bis 4 ausgewählt werden, die rs7162960, rs496888, rs2715079 und rs2374341 umfasst.
Offenbart ist das Zuschreiben einer Präsenz eines Legasthenierisikos in der zu diagnostizierenden Person bei Vorliegen von je einer Abweichung des bestimmten Genotyps vom Genotyp besagter Kontrolle in mindestens zwei SNP, ausgewählt aus der Gruppe bestehend aus rs7162960, rs496888, rs2715079 und rs2374341, erfolgt, vorzugsweise bei Vorliegen von je einer Abweichung in rs7162960 und rs496888 oder in rs7162960 und rs2715079 oder in rs7162960 und rs2374341 oder in rs496888 und rs2715079 oder in rs496888 und rs2374341 oder in rs2715079 und rs2374341.

Besagte Liste von SNPs basiert auf den in Beispiel 1 gezeigten Resultaten sowie auf der Vorhersage weiterer assoziierter SNPs (Beispiel 2).

Die Erfinder haben herausgefunden, dass der in Tabelle 2.1 aufgelistete Genotyp (minor oder major) mit Legasthenie assoziiert ist. Dies korrespondiert mit einem Nukleotid/Base an besagter exakter Position und kann wie aufgelistet homozygot oder heterozygot sein. Besagtes Resultat basiert auf dem Vergleich von Daten von legasthenen Individuen mit Daten einer nicht legasthenen Referenzgruppe. Es wurde beispielsweise herausgefunden, dass ein risikoassoziierter Genotyp, der einem G auf dem Referenzstrang im Sinne des "GRCh37.p10 reference assembly (GCF_000001405.22) Strang bei SNP rs7162960 an Position -3211 von beiden Allelen von BLM entspricht (und folglich dem Genotyp homozygot risikoassoziiert an dieser Position) mit Legasthenie assoziiert ist. Folglich weist ein C an besagter Position des Referenzstrangs bei beiden Allelen von BLM in einem getesteten Individuum auf eine Anfälligkeit für Legasthenie hin.

In Schritt d) des besagten Verfahrens gemäß der vorliegenden Erfindung wird einem Menschen Legasthenie basierend auf dem Vergleich des gefundenen (im Sinne von "experimentell bestimmten") Genotyps eines SNPs (z.B. Genotyp homozygot minor (d.h. C/C) bei rs7162960) mit einem Resultat zugewiesen, das auf eine Assoziation mit Legasthenie hinweist (z.B. Genotyp homozygot minor (d.h. CC) bei rs7162960). Auf besagte Resultate kann man auch dahingehend verweisen, dass sie auf ein Risiko für Legasthenieentwicklung hinweisen, und man kann folglich "Zuweisung von Legasthenie" diesbezüglich als "Zuweisung von Legasthenie basierend auf dem assoziierenden Risiko" interpretieren. Im Falle, dass die beiden Resultate übereinstimmen, wird dies als Hinweis auf Legasthenie in dem getesteten Individuum interpretiert. Besagte Zuweisung kann jedoch nicht nur auf einem Resultat basieren.

In den hierin gezeigten experimentellen Resultate wurden unter Verwendung von Daten von 255 ausgewählten Legasthenikern und 397 nicht legasthenen Individuen ermittelt, wobei die Zahlen für jeden individuell analysierten SNP aufgrund der Realisierbarkeit der Resultate schwanken können. Aus früheren experimentellen Studien scheint es, dass unterschiedliche genetische Regionen mit Legasthenie assoziiert sein können, abhängig von der Herkunft, bzw. dem spezifischen Hintergrund der analysierten Legastheniker/betroffenen Familien., d. h. es gibt eine Assoziation mit der Sprache. Es kann bevorzugt sein, die mit einem der vorliegenden Erfindung gemäßen Verfahren zusammengetragenen Resultate im Kontext von kaukasischen Menschen zu interpretieren. Weiterhin können besagte Resultate im Kontext von kaukasischen Menschen interpretiert werden, die zu einer Indogermanisch sprechenden Population gehören. Ein Kriterium für die Auswahl von Individuen mit Legasthenie für die Studien der Erfinder war unter anderem, dass die Individuen keine Konzentrationsschwierigkeiten und einen Mindest-IQ von 85 hatten. Folglich können besagte Resultate im Kontext von nicht mit besagten Schwierigkeiten assoziierten Formen von Legasthenie interpretiert werden.
Offenbart ist, dass, wie oben erwähnt, andere Krankheiten und Auffälligkeiten, wie ADS oder ADHS auszuschließen. Des Weiteren ist es offenbart, dass das Verfahren nur mit Proben von Personen durchgeführt wird, die einen IQ von 80 oder mehr haben, vorzugsweise einen IQ von 85 oder mehr. Verfahren zum Ausschluss, bzw. Diagnose von Aufmerksamkeitsdefizitsyndrom (ADS) und/oder Hyperaktivität und/oder ADHS und/oder eines IQ von weniger als 80 sind dem Fachmann bekannt. Zum Beispiel kann ADS durch psychometrische Verfahren (Döpfner, M., Lehmkuhl, G., Steinhausen, H.-C. (2006). KIDS1 - Aufmerksamkeits- und Hyperaktivitätsstörung. Göttingen, Hogrefe) diagnostiziert werden. ADS kann auch kombiniert sein mit Hyperaktivität, dann spricht der Fachmann von ADHS. Als spezifische psychodiagnostische Testverfahren für ADHS steht beispielsweise von Manfred Döpfner, Gerd Lehmkuhl und H.-C. Steinhausen eine Diagnose-Checkliste in Form von Fragebögen und Checklisten für Aufmerksamkeitsdefizit-/Hyperaktivitätsstörungen (DCL-ADHS), der Fragebogen zum Hyperkinetischen Syndrom und Therapieleitfaden von Klein sowie Conners 3TM von C. K. Conners zur Verfügung. Zur grundlegenden Diagnostik gehören daher neben der Befragung des betroffenen Kindes, der Eltern bzw. Erzieher und Lehrkräfte auch eine gründliche psychologische Testdiagnostik, eine neurologische Untersuchung sowie Verhaltensbeobachtung (M. Döpfner, G. Lehmkuhl, H.-C. Steinhausen: Kinder-Diagnostik-System (KIDS), Band 1: Aufmerksamkeitsdefizit- und Hyperaktivitätsstörungen (ADHS). Hohgrefe, Göttingen 2006; diese Veröffentlichung ist durch Referenz in die vorliegende Anmeldung einbezogen). Eine Diagnose sollte sich auf Informationen aus unterschiedlichen Quellen stützen, da ein einzelner Test oder Lebensumfeld nicht die komplette Differenzialdiagnostik abdecken kann. Zur grundlegenden Diagnostik gehören daher neben der Befragung des betroffenen Kindes, der Eltern bzw. Erzieher und Lehrkräfte auch eine gründliche psychologische Testdiagnostik, eine neurologische Untersuchung sowie Verhaltensbeobachtung. Konzentrationsstörungen, wie sie bei ADHS vorliegen, können auf ganz unterschiedliche biologische und psychologische Umstände und Ursachen zurückgeführt werden. Da sich die ADHS-Leitsymptome (z. B. Hyperaktivität, geringe Ausdauer, schlechtes Schriftbild, Ängste, Depressionen, Konzentrationsschwierigkeiten) und die eines Magnesiummangels überlappen, kann eine genaue Überprüfung der Ursachen geboten sein (D.-H. Liebscher, K. Baerlocher, H.-G. Classen, U. C. Liebscher, G.-W. Ratzmann, W. Vierling, A.Weigert, K. Kisters: Magnesiummangel und -therapie bei ADHS. In: Nieren- und Hochdruckkrankheiten, 40. Jg., Nr. 3, Mai 2011, ISSN 0300-5224, S. 123-128; diese Veröffentlichung ist durch Referenz in die vorliegende Anmeldung einbezogen). Für die Fremdurteile (Lehrkräfte, Eltern) steht eine Reihe von Fragebogenverfahren zur Verfügung. Besser ist jedoch die direkte Beobachtung des Kindes in der Schule und zu Hause; diese sollte zusätzlich erfolgen. Bei der Diagnose von ADHS sollten die Kriterien der Weltgesundheitsorganisation eingehalten werden. Vorzugsweise liegen die Symptome seit mindestens sechs Monaten in mindestens zwei Lebensbereichen vor. Weiter ist es bevorzugt, dass die Symptome von ADHS erstmals vor dem siebten Lebensjahr aufgetreten sind. Die Symptome von ADHS bei zu diagnostizierenden, bzw. behandelnden Menschen weiblichen Geschlechts können sich erst in der Pupertät zeigen. Es darf zum Beispiel keine tiefgreifende Entwicklungsstörung, keine Schizophrenie und keine andere psychotische Störung vorliegen. Die aktuelle Forschung relativiert diese Ausschließlichkeit allerdings deutlich. So weiß man inzwischen zum Beispiel, dass - entgegen früherer Annahmen - ADHS und Autismus sich nicht gegenseitig ausschließen (Kirsten Stollhoff: Autismus und ADHS häufig gepaart. In: Pädiatrie Hautnah, 23. Jg., Nr. 6, 2011, ISSN 1437-1782, S. 447; diese Veröffentlichung ist durch Referenz in die vorliegende Anmeldung einbezogen). Eine testpsychologische Untersuchung dauert üblicherweise ein bis zwei Stunden, um auch eine gründliche Verhaltensbeobachtung in der Testsituation zu gewährleisten. Zusätzlich können weiterer Tests durchgeführt werden, z. B. der Denkfertigkeiten ("Intelligenztest" zur Bestimmung des Intelligenzquotienten (IQ)). Hierbei können die Untertests einen Aufschluss über die Stärken und Schwächen liefern und eine Hilfe in der Diagnosestellung bieten. Weiter kann eine Magnet-Resonanz-Tomographie (MRT) angefertigt werden. Ein EEG kann zusätzlich durchgeführt, um zu klären, ob andere Erkrankungen vorliegen, z.B. Epilepsie.

Im Falle, dass in Tabelle 2.1 ein p-Wert angegeben ist, wurde der SNP gemäß der in Beispiel 1 angegebenen Daten analysiert und für mit Legasthenie assoziierend befunden.

Es kann bevorzugt sein, sich nicht nur auf die in Tabelle 2.1 aufgelisteten SNPs zu verlassen, sondern auch weitere Polymorphismen wie weitere SNPs, Deletionen, Inversionen, Translokationen und/oder Insertionen, die eine Korrelation mit Legasthenie zeigen, zu bestimmen.
Die Genotypen können von mindestens einem der folgenden 4 in den Genen TNFRSF1B und BLM sowie in der mit Legasthenie assoziierten Regionen von Nukleotid 41930000 bis 42032000 und von Nukleotid 51508000 bis 51885000 des Chromosoms 2 SNPs zur Diagnose eines Legasthenierisikos bei einem Menschen bestimmt werden: rs496888 in TNFRSF1B, rs7162960 in BLM sowie rs2715079 in der chromosomalen Region von Nukleotid 41930000 bis 42032000 des Chromosoms 2 und rs2374341 in der chromosomalen Region von Nukleotid 51508000 bis 51885000 des Chromosom 2. Man kann dann gemäß folgendem Schema die für die SNPs gewonnenen Resultate einer Anfälligkeit für Legasthenie zuweisen: Die Genotypen homozygot major (d.h. AA) bei rs496888, homozygot minor (d.h. CC) bei rs7162960, homozygot major (d.h. CC) bei rs2715079 und homozygot minor (d.h. TT) bei rs2374341 sind mit Legasthenie assoziiert.

Vorzugsweise kann man den Genotyp von mindestens einem der besagten 4 SNPs in der folgenden Reihenfolge bestimmen: rs496888, rs2715079, rs7162960 und rs2374341. Besagte Klassifikation basiert auf den sogenannten "p-Werten", welche für Wahrscheinlichkeiten kennzeichnend sind, die mit besagtem Genotyp assoziiert sind. Wenn ein einzelner SNP von den für eine Analyse aufgelisteten SNPs verwendet wird, z.B. in Kombination mit anderen Markern, kann seine Wahrscheinlichkeit gemäß dem p-Wert klassifiziert werden, wobei der niedrigste p-Wert die höchste Wahrscheinlichkeit kennzeichnet.

Folglich kann die Auflistung am Beginn dieses Absatzes auch für eine Klassifikation genutzt werden, wie signifikant individuelle SNPs sind, beginnend mit dem signifikantesten SNP.

Im Falle, dass zwei der 2 SNPs verwendet werden, kann man vorzugsweise rs496888 und rs2715079 auswählen. Im Falle, dass drei der 4 SNPs verwendet werden, kann man vorzugsweise rs496888, rs2715079 und rs7162960 auswählen und so weiter.

Weiterhin sind isolierte Oligonukleotide zur Detektion von mindestens einem Gen ausgewählt aus der in Tabelle 1 aufgelisteten Gruppe von Genen, einschließlich Nukleinsäuremolekülen, die spezifisch Regionen in besagten mit Legasthenie assoziierten Regionen bzw. SNP detektieren offenbart.

Folglich sind Nukleinsäuremoleküle beschrieben, die spezifisch Regionen in TNFRSF1B (SEQ ID No: 1) detektieren, die mit Legasthenie assoziiert sind. Weiterhin sind Nukleinsäuremoleküle offenbart, die spezifisch Regionen in BLN (SEQ ID No: 2) detektieren, die mit Legasthenie assoziiert sind.
Weiterhin sind Nukleinsäuremoleküle offenbart, die spezifisch Regionen in der Region von Nukleotid 41930000 bis 42032000 des Chromosoms 2 und der Region von Nukleotid 51508000 bis 51885000 des Chromosoms 2 detektieren, die mit Legasthenie assoziiert sind.

Besagte Nukleinsäuremoleküle können im Bereich von ungefähr 12 bis ungefähr 20 Nukleotiden liegen, wobei 15 Nukleotide bevorzugt sind, die die Regionen in besagten mit Legasthenie assoziierten Genen überspannen. Vorzugsweise können besagte Regionen die im Folgenden aufgelisteten SNPs sein.

Die Regionen, die die in Tabelle 2.1 aufgelisteten SNPs umfassen, können mittels der wie oben definierten dbSNP-Datenbank, worin jeder SNP eine definierte Position im Genom hat, identifiziert und beschafft werden. Wenn man sich auf ein Gen bezieht, worin ein SNP gelegen ist, kann man weiterhin die Position des besagten SNPs in Bezug auf das ATG des besagten Gens definieren. In den folgenden Absätzen werden die SNP-Positionen gemäß besagter Definition angegeben, wobei ein "+" eine Position stromaufwärts des ATG und ein "-" eine Position stromabwärts des ATG des entsprechenden Gens bezeichnet.

Folglich ist eine Polynukleotidsonde beschrieben, die spezifisch den SNP rs496888 an Position 501 in SEQ ID NO: 1 oder SEQ ID NO: 2 (TNFRSF1B-Region) detektieren, worin ein G an besagter Position das minor Allel repräsentiert und ein A das major Allel repräsentiert. Die SNP Position entspricht Nukleotid -20148 gesehen vom ATG des TNFRSF1B Gens.

Offenbart ist weiterhin eine Polynukleotidsonde, die spezifisch den SNP rs7162960 an Position 501 in SEQ ID NO: 3 oder SEQ ID NO: 4 (BLM-Region) detektieren, worin ein G an besagter Position das minor Allel (SEQ ID NO: 4) repräsentiert und ein A das major (Allel SEQ ID NO: 3) repräsentiert. Die SNP Position entspricht Nukleotid -3211 gesehen vom ATG des TNFRSF1B Gens.
Offenbart ist weiterhin eine Polynukelotidsonde, die spezifisch den SNP rs2715079 an Position 501 in SEQ ID Nr. 5 oder SEQ ID NO: 6 (Region von Nukleotid 41930000 bis 42032000 des Chromosom 2) detektieren, worin ein T an besagter Position das minor Allel (SEQ ID NO: 6) repräsentiert und ein C das major Allel repräsentiert (SEQ ID NO: 5).
Offenbart ist weiterhin eine Polynukelotidsonde, die spezifisch den SNP rs2374341 an Position 501 in SEQ ID Nr. 7 oder SEQ ID NO: 8 (Region von Nukleotid 51508000 bis 51885000 des Chromosom 2) detektieren, worin ein T an besagter Position das minor Allel (SEQ ID NO: 9) repräsentiert und ein C das major Allel (SEQ ID NO: 8) repräsentiert.

Besagte Polynukleotidsonden können als Sonden in einem Mikroarray wie hierin beschrieben verwendet werden (und somit als "Sondennukleotide" bezeichnet werden). Es gelten alle hierin angegebenen Ausführungsformen für die Polynukleotidsonden.

Wie hierin erwähnt, kann es nötig sein, dass die chromosomale Region, deren Genotyp es zu bestimmen gilt vor der eigentlichen Bestimmung des Genotyps zunächst amplifiziert werden muss. Dem Fachmann sind verschiedene Verfahren zur Amplifikation von chromosomalen Regionen bekannt. Viele dieser Amplifikationsverfahren bedienen sich kurzer Oligonukleotide, die an die zu amplifizierende Region flankierende Sequenzen spezifisch binden, auch Primer oder Polynukleotidprimer genannt. Die Erfinder der vorliegenden Erfindung haben hierfür bevorzugte Oligonukleotide entwickelt, die besonders gut geeignet sind relevante Genotypen im Zusammenhang mit der vorliegenden Erfindung zu bestimmen, d.h. die entsprechenden chromosomalen Regionen zu amplifizieren.
Daher sind Polynukleotidprimer beschrieben, umfassend eine Sequenz ausgewählt aus der Gruppe bestehend aus SEQ ID NO. 9, SEQ ID NO. 10, SEQ ID NO. 13, SEQ ID NO. 14, SEQ ID NO. 17, SEQ ID NO. 18, SEQ ID NO. 20 und SEQ ID NO. 21. Dem Fachmann ist geläufig, dass es für die Amplifikation mittels Polynukleotidprimer vorwiegend auf dessen Spezifität ankommt, um zu gewährleisten, dass die Primer nur an der vorgesehenen Stelle hybridisieren und somit sicherstellen, dass nur das gewünschte Amplifikationsprodukt erhalten wird. Auch ist dem Fachmann bewusst, dass zur Gewährleistung der Elongation des Primers im Amplifikationsverfahren, die Bindung des 3'Endes des Primers von Bedeutung ist. Hier kann es vorteilhaft sein, dass eine bestimmte Anzahl der Nukleotide am 3'Ende des Primers eine 100%tige Identität mit dem Gegenstrang der Sequenz, an die der Primer Binden soll, aufweisen. Die Länge dieser Identität kann variieren und der Fachmann ist in der Lage, entsprechende Gesichtspunkte bei der Wahl der primer zu berücksichtigen. Insbesondere ist dem Fachmann bewusst, dass dies von Faktoren, wie der Sequenz und dem GC-Gehalt des entsprechenden Abschnitts abhängt.
Die Sequenz des 3'-Endes des Polynukleotidprimers kann aus der Sequenz der letzten mindestens 6 Nukleotide des 3'-Endes
einer Sequenz, ausgewählt aus der Gruppe bestehend aus SEQ ID NO. 9, SEQ ID NO. 10, SEQ ID NO. 13, SEQ ID NO. 14, SEQ ID NO. 17, SEQ ID NO. 18, SEQ ID NO. 20 und
SEQ ID NO. 21 bestehen. Offenbart ist auch ein Polynukleotidprimer bestehend aus der Sequenz ausgewählt aus der Gruppe bestehend aus SEQ ID NO. 9, SEQ ID NO. 10, SEQ ID NO. 13, SEQ ID NO. 14, SEQ ID NO. 17, SEQ ID NO. 18, SEQ ID NO. 20 und SEQ ID NO. 21. Weiter ist dem Fachmann ersichtlich, dass die Länge des Polynukleotidprimers ein weiterer Faktor ist, den es zu beachten gilt ist.
Offenbart ist auch, dass der Polynukleotidprimer eine Länge von 10 bis 50 Nukleotiden, vorzugsweise eine Länge von 15 bis 30 Nukleotiden, besonders bevorzugt eine Länge von 20 bis 30 Nukleotiden hat. Die Sequenz des 3'-Endes des Polynukleotidprimers kann aus der Sequenz der letzten mindestens 6 Nukleotide des 3'-Endes einer Sequenz bestehen, ausgewählt aus der Gruppe bestehend aus SEQ ID NO. 9, SEQ ID NO. 10, SEQ ID NO. 13, SEQ ID NO. 14, SEQ ID NO. 17, SEQ ID NO. 18, SEQ ID NO. 20 und SEQ ID NO. 21, und der Polynukleotidprimer hat eine Länge von 20 bis 30 Nukleotiden.

Die Verfahren, welche verwendet werden können, um Nukleinsäuren zu analysieren, können aus der Gruppe von Verfahren ausgewählt sein, die Sequenzierung, Mikroarray, Real-time PCR und Massenspektrometrie umfasst. Selbstverständlich kann jedes andere dem Fachmann bekannte Verfahren verwendet werden, um den Genotyp eines Individuums zu bestimmen.

Sequenzierung oder auch DNS-Sequenzierung ist ein Routineverfahren, das durchgeführt wird, um die Sequenz einer bestimmten Region zu bestimmen. Es verwendet Primer, die so gestaltet sind, dass sie die zu analysierende Region zusammen mit markierten Nukleotiden in einem PCR-ähnlichen Aufbau flankieren. Mittels Analyse der Markierungen an den entsprechenden Positionen ist es möglich, die Sequenz der DNS zu bestimmen, beginnend ab der Region, mit welcher der Primer hybridisiert.

DNS-Mikroarray Techniken sind dem Fachmann auch sehr gut bekannt. Besagte Techniken basieren auf Hybridisierungsereignissen zwischen der Test-DNS und sogenannten "Sonden", die auf definierten Punkten eines Mikroarrays in einer Kammer immobilisiert sind. Heutzutage werden solche Mikroarrays routinemäßig verwendet, um DNS-Sequenzen zu bestimmen, sogar bis hinab zur Ebene von Einzelbasen und somit zur Detektion von SNPs. Dies ist durch entsprechende Auswahl der Sonden und die Verwendung spezifischer Hybridisierungsbedingungen möglich. Bei besagten Techniken kann die DNS für Detektionszwecke markiert werden. Routinemäßig können Sonden, die die verschiedenen Sequenzen an der Position eines SNPs abdecken, in Kombination mit entsprechenden Kontrollen verwendet werden; folglich kann auch der Genotyp des entsprechenden SNPs analysiert werden. Auch Next-Generation-Sequencing-Verfahren können angewandt werden und sind wohl bekannt.

Der Begriff "Kammer" eines Arrays bezeichnet einen geschlossenen Raum, worin die Sonden so angeordnet sind, dass deren aufnehmende (im Sinne von hybridisierende) Polynukleotidsonden (getrennt von den verankernden Teilen) so in dem definierten Raum präsentiert werden, dass sie in der Lage sind, andere in besagtem Raum vorhandene Moleküle zu kontaktieren. Selbstverständlich kann besagter Raum oder Kammer für einen bestimmten Schritt während des Versuchsablaufs, wie das Laden mit einer Flüssigkeitsprobe in besagten Raum, geöffnet sein. Zur Analyse von Interaktionen zwischen den Sonden und anderen in besagtem Raum vorhandenen Molekülen, kann jedoch die Kammer geschlossen sein, um ein geschlossenes Reaktionssystem mit definierten Reaktionsparametern, wie der Konzentration von bestimmten Molekülen innerhalb besagter Kammer, zu ergeben.

Alle besagten Techniken können mit der oben dargelegten vorherigen Aufreinigung und/oder Amplifikation der DNS kombiniert werden, aber auch mit bestimmten Restriktionsverdauen, um DNS zu schneiden und es zu ermöglichen, kleinere DNS-Fragmente zu analysieren.

Alle Aspekte und Ausführungsformen eines Verfahrens gemäß der vorliegenden Erfindung können verwendet werden, um bei einem Menschen Legasthenie zu diagnostizieren und/oder eine Legastheniediagnose in einem Menschen zu erhärten, welcher schon mit herkömmlichen Verfahren wie Lese- und Rechtschreibtests getestet wurde.

Die einzige Voraussetzung für besagtes Verfahren ist die Bereitstellung einer Probe eines zu testenden Menschen, die Nukleinsäure, wie z.B. DNS umfasst. Folglich kann das Verfahren gemäß der Erfindung unabhängig vom Alter des zu testenden Menschen verwendet werden. Dies kann während einer Routineuntersuchung für Kinder geschehen, um Legasthenie so früh wie möglich zu detektieren. Entsprechende Hilfe kann dann daraufhin zu einem frühen Entwicklungsstadium erfolgen.

Es versteht sich, dass die Beispiele und Abbildungen nicht als limitierend aufzufassen sind. Dem Fachmann ist es natürlich möglich, sich weitere Modifikationen zu den hier dargelegten Prinzipien vorzustellen. Die folgenden Beispiele dienen lediglich der Veranschaulichung der Erfindung.

### BEISPIELE

### Beispiel 1:

*Identifikation von mit Legasthenie identifizierten SNPs mittels der Bestimmung der Sequenz der entsprechenden Regionen in legasthenen Patienten sowie in gesunden Referenzindividuen und Analyse der für besagte zwei Gruppen zusammengetragenen Resultate mittels statistischer Bestimmung, ob es eine Assoziation der entsprechenden SNPs mit Legasthenie gibt.*

### Ethikvotum

Das Ethikvotum wurde bei der Ethikkommission der Universität Leipzig und dem Regionalschulamt Leipzig sowie dem Sächsischen Staatsministerium für Kultus eingeholt. Eine schriftliche Einverständniserklärung wurde bei den Eltern der Probanden eingeholt.

### Studiengruppe

Die Studiengruppe bestand aus 255 Legasthenikern von deutscher Abstammung. Kontrollen waren 397 gesunde Blutspender von deutscher Abstammung.

Die Erhebung der Daten von Kindern mit Legasthenie erfolgte durch Kontakte zu Schulen mit speziellen Legasthenieklassen, die auf die Unterrichtung von Kindern mit Leseschwierigkeiten spezialisiert sind. Einschlusskriterien für Probanden waren ein IQ ≥ 85, keine Hinweise auf Aufmerksamkeits-Hyperaktivitätssyndrom (ADHS) und die verifizierte Legastheniediagnose. Der IQ wurde mittels des nicht sprachbasierten CFT-20 (Weiß, 1998) getestet, ADHS mit dem Konzentrationstest d2 (Brickenkamp 1994). Für die spätere Analyse von Legasthenie führten wir den Lese- und Rechtschreibtest KNUSPEL-L (Marx 1998) durch. Alle Kinder gingen entweder in die dritte oder vierte Klasse.

### Genotypisierung

DNS wurde aus den jeweiligen Speichelproben nach Herstellerangaben extrahiert (Qiagen Blood & Tissue kit, QIAGEN, Hilden). Die bevorzugten SNPs wurden mittels eines kombinierten Tagging- (Haploview) und funktionalen Ansatzes (mittels der Software Pupasuite, Reumers, J., Conde, L., Medina, I., Maurer-Stroh, S., Van Durme, J,, Dopazo, J., Rousseau, F., Schymkowitz, J. (2008). Joint annotation of coding and non-coding single nucleotide polymorphisms and mutations in the SNPeffect and PupaSuite databases. Nucleic Acids Res, 36(Database issue), D825-9) ausgewählt. Der Haploview-Tagger (siehe: http:///www.broad.mit.edu/mpg/tagger/) ist ein Werkzeug zur Auswahl und Evaluation von tag-SNPs aus Genotypisierungsdaten, wie denen des Internationalen HapMap Projekts. PCR-Primer Entwurf wurde unter Verwendung von NCBI *dbSNP* und Ensembl-Datenbanken und den Computerprogrammen muPlex (Rachlin et al., 2005), ePCR (Schuler 1997), HumanBlat (Kent 2002) und Netprimer (PREMIER Biosoft International, Palo Alto, CA) implementiert. Alle PCR-Primer wurden von MWG-Biotech (Ebersberg, Deutschland) bezogen.

SNP-Genotypisierung wurde unter Verwendung des GenoSNIP Verfahrens mit leichten Modifikationen (Wenzel et al., 2003; Kirsten et al., 2007) durchgeführt. PCR-Reaktionen wurden unter Standardbedingungen durchgeführt. Das resultierende PCR-Produkt, wurde bei 37°C für 1h im selben Röhrchen verdaut, indem 2µl eines Gemisches zugegeben wurden, das Exonuklease I (0.2U) und Shrimp alkalische Phosphatase (0.3U) enthielt. Enzyme wurden bei 80°C für 20 min inaktiviert.

Primer für Einzelbasenverlängerungsreaktionen (SBE) wurden mit photospaltbaren Basen entworfen (Kirsten et al. 2006). Alle SBE-Primer wurden von Biotez (Berlin, Deutschland) bezogen. SBE-Reaktionen wurden unter folgenden Bedingungen durchgeführt: Initiale Denaturierung bei 95°C für 4 min, 44 Zyklen mit Denaturierung bei 94°C für 10s, Primer Hybridisierung bei 60°C für 30s und Elongation bei 72°C für 10s. Die Reaktionen bestanden aus 1µl Puffer C 10x, 1µl MgCl₂ 100mM, 0.2 TermiPol (alle von Solis Biodyne, Tartu, Estland), 0.9µl ddNTP 4x10mM (Carl Roth GmbH), 12µl verdautes PCR-Produkt, komplettiert zu 18µl mit Primer und aqua bidest. SBE-Produkte wurden mittels MALDI-TOF Massenspektrometrie (Bruker Daltonics, Leipzig, Deutschland) gemäß Standardprotokollen detektiert. Die Genotypbestimmung wurde mittels GenoTools (Pusch et al. 2001) und unter Berechnung statistischer Parameter der Genotypenverteilung durchgeführt.

### Statistik

Alle Genotypen wurden mittels Standardstatistiken für Odds Ratio und Lathrop genetisches relatives Risiko Berechnung (Lathrop 1983) auf eine Assoziation mit Legasthenie hin analysiert. Eine Analyse des Hardy-Weinberg-Gleichgewichts wurde zur Evaluation der Genotypisierungsqualität durchgeführt. Zur Haplotypanalyse wurde die Software *Haploview* 3.32 verwendet. Um die Signifikanz von Unterschieden in Allel- oder Haplotypfrequenzen zu testen, wurden Chi-Quadrat Statistiken oder, wenn angemessen, Fishers Exakter Test verwendet. Bei der Haplotypanalyse wurden die p-Werte für multiples Testen mit der Anzahl der Haplotypen korrigiert.

### Beispielhafte Ergebnisse für rs7162960 in BLM

SNP rs7162960 ist an Position -3211 vor dem BLM Gen etabliert, wobei ein C an besagter Position das minor Allel darstellt und ein T an besagter Position das major Allel darstellt. 255 Legastheniker und 394 gesunde Referenzindividuen wurden getestet.

Die Sequenzanalyse offenbarte, dass 130 von insgesamt 510 Allelen in den Proben der legasthenen Patienten (Fälle) ein C an besagter Position hatten. Folglich war die Frequenz des minor Allels 25% (Fälle_Minor_Allel_Frequenz). 380 Allele hatten ein T an der entsprechenden Position (entsprechend 75%; Fälle_Major_Allel_Frequenz). 20 legasthene Patienten waren homozygot für das minor Allel (Fälle_Homo_Minor), 145 homozygot für das major Allel (Fälle_Homo_Major) und 90 waren heterozygot (Fälle Hetero).

In den Referenzproben (Kontrollen), hatten 168 Allele ein C und 620 Allele ein T an der entsprechenden Position. Folglich war die Frequenz des minor Allels 21% (Fall_Minor_Allel_Frequenz). 18 Referenzindividuen waren homozygot für das minor Allel (Kontroll_Homo_Minor), 244 homozygot für das major Allel (Kontroll_Homo_Major) und 132 waren heterozygot (Kontroll_Hetero).

Unter Verwendung der oben dargelegten statistischen Methoden ist es möglich, zu bestimmen, ob ein bestimmter Genotyp mit Legasthenie assoziiert ist. Ein entsprechender p-Wert gibt an, wie signifikant die Assoziation ist, wobei kleinere p-Werte einen höheren Grad an Signifikanz angeben. Im vorliegenden Beispiel von rs7162960 in BLM führte besagte Analyse zu dem Resultat, dass der homozygote minor Allel Zustand mit einem p-Wert von 0.025 mit Legasthenie assoziiert ist. Dies entspricht einem C an Position -3211 auf beiden Allelen.

Unter Verwendung des oben beschriebenen Versuchsaufbaus wurden die 4 SNP in Tabelle 3.1 und 3.2 als mit Legasthenie assoziierend gefunden. Die entsprechenden p-Werte besagter SNPs sind in Tabelle 2.1 aufgelistet.

**Tabelle 2.1: Überblick über mit Legasthenie assoziierte SNPs**

| # | SNP | Im Gen | Typ | Korr. Base | p-Wert oder r<0.05 (HapMap) | GRR/OR [95% KI] |
|---|---|---|---|---|---|---|
| 1 | rs7162960 | BLM | homozygot minor | C | 0.025 | 1.83 [1.08-3.10] |
| 2 | rs496888 | TNFRSF1B | heterozygot oder homozygot major allel | A | 0.013 | 2.06 [1.16-3.66] |
| 3 | rs2715079 | intergenisch in DYX3-Region | heterozygot oder homozygot major allel | C | 0.038 | 1.56 [1.03-2.36] |
| 4 | rs2374341 | intergenisch in DYX3-Region | heterozygot oder homozygot minor | T | 0.031 | 1.43 [1.04-1.97] |

| | | | | | | |
|---|---|---|---|---|---|---|
| SNP: Single nucleotide polymorphism; GRR: Genetic relative risk [Relatives genetisches Risiko]; OR: Odds ratio [Chancenverhältnis] Korr. Base: [korrespondierende Base] bzw. Allel, das mit einem Risiko assoziiert ist | | | | | | |

**Tabelle 2.2**

| Auswahlkriterium der Subgruppe | Fälle | Kontrollen | OR [95% KI] | p-Wert |
|---|---|---|---|---|
| Referenzgruppe (0/4 oder 1/4 SNPs haben mind. ein Risikoallel) | 15 (5.9%) | 46 (11.6%) | Referenzgruppe | |
| 2/4 SNPs haben mind. ein Risikoallel | 70 (27.5%) | 132 (33.2%) | 1.63 [0.86 - 3.20] | 0.14 |
| 3/4 SNPs haben mind. ein Risikoallel | 112 (43.9%) | 161 (40.6%) | 2.13 [1.16 - 4.12] | 0.019 |
| 4/4 SNPs haben mind. ein Risikoallel | 58 (22.7%) | 58 (14.8%) | 3.07 [1.57 - 6.25] | 0.0014 |

| | | | | |
|---|---|---|---|---|
| OR: Odds ratio [Chancenverhältnis] bzgl. der Referenz, definiert als die Gruppe aller Individuen, die bei nur 0 oder 1 SNP mind. ein Risikoallel tragen). Die 4 SNPs entsprechen rs7162960, rs496888, rs2715079 und rs2374341. Zur Berechnung wurde ein standardlogistisches Regressionsmodell verwendet, in dem der Legastheniestatus die abhängige Variable darstellte und das Legasthenierisiko von Trägern von Risikovarianten in 2/4, 3/4 bzw. 4/4 SNPs mit dem Legasthenierisiko von Trägern von 0/4 bzw. 1/4 Risikovarianten (Referenz) als unabhängige Variablen verglichen wurde. | | | | |

**Tabelle 3.1**

| rs-Nummer | Fälle_Minor_Allel_Frequenz | Fälle_Major_Allel_Frequenz | Fälle_Homo_Minor | Fälle_Homo_Major | Fälle_Hetero |
|---|---|---|---|---|---|
| rs7162960 | 25% | 75% | 20 | 145 | 90 |
| rs496888 | 28% | 72% | 17 | 129 | 109 |
| rs2715079 | 43% | 57% | 39 | 74 | 142 |
| rs2374341 | 38% | 62% | 34 | 97 | 123 |

**Tabelle 3.2**

| **rs-Nummer** | **Kontroll_Minor_Allel_Frequenz** | **Kontroll**_**Major_Allel_Frequenz** | **Kontroll_Homo_Minor** | **Kontroll_Homo**_**Major** | **Kontroll_Hetero** |
|---|---|---|---|---|---|
| rs7162960 | 21% | 79% | 18 | 244 | 132 |
| rs496888 | 34% | 66% | 51 | 177 | 169 |
| rs2715079 | 48% | 52% | 87 | 104 | 205 |
| rs2374341 | 32% | 68% | 45 | 186 | 166 |

**Tabelle 4 (Hierin offenbarte Sequenzen)**

| **SEQ ID NO.** | **Sequenz** | **Beschreibung** |
|---|---|---|
| 1 | | Chromosomale Region von Nukleotid 12091000 bis 12200000 von Chromosom 1 mit A an der Position von rs496888 (unterstrichen) |
| | | |
| 2 | | Chromosomale Region von Nukleotid 12091000 bis 12200000 von Chromosom 1 mit G an der Position von rs496888 (unterstrichen) |
| 3 | | Chromosomale Region von Nukleotid 89066000 bis 89088000 von Chromosom 15 mit A an der Position von rs7162960 (unterstrichen) |
| | | |
| 4 | | Chromosomale Region von Nukleotid 89066000 bis 89088000 von Chromosom 15 mit G an der Position von rs7162960 (unterstrichen) |
| 5 | | Chromosomale Region von Nukleotid 41930000-42032000 von Chromosom 2 mit C an der Position von rs2715079 (unterstrichen) |
| | | |
| 6 | | Chromosomale Region von Nukleotid 41930000-42032000 von Chromosom 2 mit T an der Position von rs2715079 (unterstrichen) |
| 7 | | Chromosomale Region von Nukleotid 51508000 bis 51885000 von Chromosom 2 mit C an der Position von rs2374341 (unterstrichen) |
| | | |
| 8 | | Chromosomale Region von Nukleotid 51508000 bis 51885000 von Chromosom 2 mit T an der Position von rs2374341 (unterstrichen) |
| | | |
| 9 | ACGTTGGATGCACCAAATGTACCCTTCACTC | Vorwärtsprimer zur Amplifikation einer bevorzugten chromosomalen Region umfassend rs496888 |
| 10 | ACGTTGGATGCTGCTTGACAGCCTGGTC | Rückwärtsprimer zur Amplifikation einer bevorzugten chromosomalen Region umfassend rs496888 |
| 11 | CACAGCCTTTGCCACAATAC | Sequenz einer bevorzugten Polynukleotidsonde zur Bestimmung des Genotyps an rs496888 |
| 12 | [bio]CAC[L]GCCTTTGCCACAATAC | Polynukleotidsonde umfassend die Sequenz der SEQ ID NO. 11, und umfassend eine Biotinylierung [bio] und eine Photospaltstelle [L] |
| 13 | ACGTTGGATGTAATGATGCCATAGACCACTT | Vorwärtsprimer zur Amplifikation einer bevorzugten chromosomalen Region umfassend rs7162960 |
| 14 | ACGTTGGATGTCAGACTATTGCCCTCTAAGAA | Rückwärtsprimer zur Amplifikation einer bevorzugten chromosomalen Region umfassend rs7162960 |
| 15 | GCAGAGGTACACAGATCTTCAGTCTTAA | Sequenz einer bevorzugten Polynukleotidsonde zur Bestimmung des Genotyps an rs7162960 |
| 16 | [bio]GCAGAGGTACACAGA[L]CTTCAGTCTTAA | Polynukleotidsonde mit der Sequenz der SEQ ID NO. 15, umfassend eine Biotinylierung [bio] und einer Photospaltstelle [L] |
| 17 | ACGTTGGATGGTTTTTTCAGAGGATAGGGG | Vorwärtsprimer zur Amplifikation einer bevorzugten chromosomalen Region umfassend rs2715079 |
| 18 | ACGTTGGATGCTTAACAAGTCCTCAAGTTC | Rückwärtsprimer zur Amplifikation einer bevorzugten chromosomalen Region umfassend rs2715079 |
| 19 | GGGAACATTATAACAACTACACAACATA | Sequenz einer bevorzugten Polynukleotidsonde zur Bestimmung des Genotyps an rs2715079 |
| 20 | [bio]GGGAACATTATAACAACT[L]CACAACATA | Polynukleotidsonde mit der Sequenz der SEQ ID NO. 19, umfassend eine Biotinylierung [bio] und eine Photospaltstelle [L] |
| 21 | ACGTTGGATGCTGGTGTCTTCTTTGTTATGC | Vorwärtsprimer zur Amplifikation einer bevorzugten chromosomalen Region umfassend |
| 22 | ACGTTGGATGGCATGTGATGGCAAAGTCAG | Rückwärtsprimer zur Amplifikation einer bevorzugten chromosomalen Region umfassend rs2374341 |
| 23 | TCTTTGTTATGCTCTTGTCTATCCC | Sequenz einer bevorzugten Polynukleotidsonde zur Bestimmung des Genotyps an rs2374341 |
| 24 | [bio]TCTTTGTTA[L]GCTCTTGTCTATCCC | Polynukleotidsonde mit der Sequenz der SEQ ID NO. 23, umfassend eine Biotinylierung [bio] und eine Photospaltstelle [L] |

## Patentansprüche

1. Verfahren zur Diagnose eines Legasthenierisikos, umfassend die Schritte:
a) Bereitstellung einer Nukleinsäure beinhaltenden Probe von einem zu diagnostizierenden Menschen,
b) Bestimmung des Genotyps der Nukleinsäure der Probe für die chromosomale Region von Nukleotid 12091000 bis 12200000 des Chromosoms 1;
c) Vergleich des in Schritt b) bestimmten Genotyps mit dem Genotyp einer Kontrolle zur Feststellung von Abweichungen des bestimmten Genotyps vom Genotyp der Kontrolle;
d) Zuschreiben einer Präsenz eines Legasthenierisikos in der zu diagnostizierenden Person bei Vorliegen mindestens einer festgestellten Abweichung des bestimmten Genotyps vom Genotyp besagter Kontrolle in der besagten Region, wobei besagte Abweichung SNP rs496888 ist, und wobei das Vorliegen eines A für mindestens ein Allel von rs496888 der Präsenz eines Legasthenierisikos in der zu diagnostizierenden Person zugeschrieben wird.

2. Verfahren nach Anspruch 1, wobei besagte Abweichung in mindestens zwei SNP ist, ausgewählt aus:
(i) rs496888 und rs7162960;
(ii) rs496888 und rs2715079; und/oder
(iii) rs496888 und rs2374341 ist;
wobei das Vorliegen eines C für mindestens ein Allel von rs7162960, das Vorliegen eines C für mindestens ein Allel von rs2715079 oder das Vorliegen eines T für mindestens ein Allel von rs2374341 der Präsenz eines Legasthenierisikos in der zu diagnostizierenden Person zugeschrieben wird.

3. Verfahren nach Anspruch 1 oder 2, wobei das Zuschreiben einer Präsenz eines Legasthenierisikos in der zu diagnostizierenden Person bei homozygotem Vorliegen besagter Abweichung(en) des bestimmten Genotyps vom Genotyp besagter Kontrolle erfolgt.

4. Verfahren gemäß irgendeinem der Ansprüche 1 bis 3, wobei die DNS enthaltende Probe ausgewählt ist aus der Gruppe bestehend aus Gewebeprobe, Körperflüssigkeiten, Blut, Haut, Haar, Nagel und Speichel.

5. Verfahren gemäß irgendeinem der Ansprüche 1 bis 4, wobei Schritt b) die Aufreinigung und/oder Amplifizierung der DNS in besagter Probe umfasst, bevorzugt die Amplifizierung besagter Regionen oder Teilen davon.

## Claims

1. A method for diagnosing a risk for dyslexia, comprising the steps of:
a) providing a sample containing a nucleic acid of a human to be diagnosed;
b) determining the genotype of the nucleic acid of the sample for the chromosomal region of nucleotides 12091000 to 12200000 of chromosome 1;
c) comparing the genotype determined in step b) with the genotype of a control in order to detect deviations of the determined genotype from the genotype of the control;
d) attributing a presence of a risk for dyslexia in the subject to be diagnosed when at least one detected deviation of the determined genotype from the genotype of said control in said region is present, wherein said deviation is SNP rs496888, and wherein the presence of an A for at least one allele of rs496888 is attributed to the presence of a risk for dyslexia in the subject to be diagnosed.

2. The method according to claim 1, wherein said deviation is in at least two SNPs selected from:
(i) rs496888 and rs7162960;
(ii) rs496888 and rs2715079; and/or
(iii) rs496888 and rs2374341;
wherein the presence of a C for at least one allele of rs7162960, the presence of a C for at least one allele of rs2715079 or the presence of a T for at least one allele of rs2374341 is attributed to the risk of dyslexia in the subject to be diagnosed.

3. The method of claim 1 or 2, wherein a presence of a risk for dyslexia in the subject to be diagnosed is attributed if homozygous presence of said deviation(s) of the determined genotype from the genotype of said control is given.

4. The method according to any one of claims 1 to 3, wherein the sample containing DNA is selected from the group consisting of tissue sample, body fluids, blood, skin, hair, nails and saliva.

5. The method according to any one of claims 1 to 4, wherein step b) comprises the purification and/or amplification of DNA in said sample, preferably the amplification of said regions or parts thereof.

## Revendications

1. Procédé de diagnostic d'un risque de dyslexie, comprenant les étapes :
a) de fourniture d'un échantillon contenant un acide nucléique d'un être humain à diagnostiquer,
b) de détermination du génotype de l'acide nucléique de l'échantillon pour la région chromosomique du nucléotide 12091000 à 12200000 du chromosome 1 ;
c) de comparaison du génotype déterminé à l'étape b) avec le génotype d'un témoin pour l'identification d'anomalies du génotype déterminé par rapport au génotype du témoin ;
d) d'attribution d'une présence d'un risque de dyslexie chez la personne à diagnostiquer en présence d'au moins une anomalie identifiée du génotype déterminé par rapport au génotype dudit témoin dans ladite région, dans lequel ladite anomalie est SNP rs496888, et dans lequel l'existence d'un A pour au moins un allèle de rs496888 est attribuée à la présence d'un risque de dyslexie chez la personne à diagnostiquer.

2. Procédé selon la revendication 1, dans lequel ladite anomalie dans au moins deux SNP est choisie parmi :
(i) rs496888 et rs7162960 ;
(ii) rs496888 et rs2715079 ; et/ou
(iii) rs496888 et rs2374341 ;
dans lequel l'existence d'un C pour au moins un allèle de rs7162960, l'existence d'un C pour au moins un allèle de rs2715079 ou l'existence d'un T pour au moins un allèle de rs2374341 est attribuée à la présence d'un risque de dyslexie chez la personne à diagnostiquer.

3. Procédé selon la revendication 1 ou 2, dans lequel l'attribution d'une présence d'un risque de dyslexie chez la personne à diagnostiquer s'effectue en présence homozygote de ladite/desdites anomalie(s) du génotype déterminé par rapport au génotype dudit témoin.

4. Procédé selon l'une quelconque des revendications 1 à 3, dans lequel l'échantillon contenant l'ADN est choisi dans le groupe consistant en échantillon de tissu, fluides corporels, sang, peau, cheveu, ongle et salive.

5. Procédé selon l'une quelconque des revendications 1 à 4, dans lequel l'étape b) comprend la purification et/ou l'amplification de l'ADN dans ledit échantillon, de préférence l'amplification desdites régions ou parties de celles-ci.
